(19) Europäisches Patentamt
European Patent Office
Office européen des brevets

(11) EP 3 564 222 A1

(12) **EUROPEAN PATENT APPLICATION**
published in accordance with Art. 153(4) EPC

(43) Date of publication:
**06.11.2019 Bulletin 2019/45**

(21) Application number: **17889133.9**

(22) Date of filing: **13.12.2017**

(51) Int Cl.:
***C07D 277/82*** *(2006.01)* ***C08F 20/38*** *(2006.01)*
***G02B 1/111*** *(2015.01)* ***G02B 5/30*** *(2006.01)*
***G02F 1/1335*** *(2006.01)* ***G02F 1/13363*** *(2006.01)*

(86) International application number:
**PCT/JP2017/044699**

(87) International publication number:
**WO 2018/123586 (05.07.2018 Gazette 2018/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **27.12.2016 JP 2016254491**
**08.08.2017 JP 2017153659**

(71) Applicant: **Zeon Corporation**
**Tokyo 100-8246 (JP)**

(72) Inventors:
- **SAKAMOTO, Kei**
**Tokyo 100-8246 (JP)**
- **OKUYAMA, Kumi**
**Tokyo 100-8246 (JP)**
- **MIMA, Takanori**
**Tokyo 100-8246 (JP)**

(74) Representative: **Adam, Holger et al**
**Kraus & Weisert**
**Patentanwälte PartGmbB**
**Thomas-Wimmer-Ring 15**
**80539 München (DE)**

(54) **POLYMERIZABLE COMPOUND, POLYMERIZABLE LIQUID CRYSTAL MIXTURE, POLYMER, OPTICAL FILM, OPTICALLY ANISOTROPIC BODY, POLARIZING SHEET, DISPLAY DEVICE, ANTIREFLECTIVE FILM, AND COMPOUND**

(57) Provided is a polymerizable compound that is useful in production of a polymer with which an optical film or the like having good reverse wavelength dispersion at long wavelengths can be produced. The polymerizable compound is indicated by formula (1-1), shown below. [In formula (1-1), $Ar^0$ represents an aromatic hydrocarbon cyclic group having at least $D^0$ as a substituent or an aromatic heterocyclic group having at least $D^0$ as a substituent, and $Ar^1$ represents an aromatic hydrocarbon cyclic group having at least $D^1$ as a substituent or an aromatic heterocyclic group having at least $D^1$ as a substituent, where $D^0$ and $D^1$ each represent, independently of one another, an organic group having a carbon number of 1 to 67 and including at least one aromatic ring selected from the group consisting of an aromatic hydrocarbon ring and an aromatic heterocyclic ring.]

$$P^1-L^1-Y^3\left[B^1-Y^1\right]_p A^1-Z^1-Ar^0-Z^2-Xa-Z^3-Ar^1-Z^4-A^2\left[Y^2-B^2\right]_q Y^4-L^2-P^2$$

(I-1)

EP 3 564 222 A1

**Description**

TECHNICAL FIELD

The present disclosure relates to an optical film and an optically anisotropic body having good reverse wavelength dispersion at long wavelengths, and to a polarizer, a display, and an antireflection film in which the optically anisotropic body is used.

Moreover, the present disclosure relates to a polymer that can be used in production of the optical film and the optically anisotropic body, a polymerizable liquid crystal mixture and a polymerizable compound that can be used in production of the polymer, and a compound that can be used in production of the polymerizable compound.

BACKGROUND

Examples of retardation plates used in various devices such as flat panel displays include quarter-wave plates that convert linearly polarized light to circularly polarized light and half-wave plates that perform 90° conversion of the plane of vibration of linearly polarized light. Such retardation plates can accurately impart a retardation of 1/4λ or 1/2λ of the wavelength of light with respect to specific monochromatic light.

However, conventional retardation plates have a problem that polarized light that passes therethrough and is output therefrom is converted to colored polarized light. Specifically, since a constituent material of the retardation plate has a property of wavelength dispersion with respect to retardation, and a distribution arises in the polarization state of each wavelength for white light, which is a composite wave in which light in the visible region is mixed, it is impossible to achieve accurate adjustment of input light to polarized light with a retardation of 1/4λ or 1/2λ over all wavelength regions.

In order to solve this problem, various retardation plates having a property referred to as "reverse wavelength dispersion" have been studied. These retardation plates are wideband retardation plates that can achieve uniform retardation with respect to light over a wide wavelength region.

On the other hand, enhanced functionality and widespread use of mobile information terminals such as mobile personal computers and mobile phones has been accompanied by demand for thickness-reduction of flat panel displays to as great an extent as possible. Consequently, there has also been demand for thickness-reduction of retardation plates used as components thereof.

In terms of methods of achieving thickness-reduction, a method in which a retardation plate is produced by applying a polymerizable composition containing a low-molecular weight polymerizable compound onto a film substrate to form an optical film has been regarded as the most effective method in recent years. For this reason, there has been much development of polymerizable compounds that are capable of forming optical films that excel in terms of reverse wavelength dispersion, and also polymerizable compositions in which these compounds are used.

In one specific example, a polymerizable compound has been provided that is used in production of an optical film of a polarizer, a retardation plate, or the like for which sedimentation does not readily occur (for example, refer to Patent Literature (PTL) 1).

CITATION LIST

Patent Literature

PTL 1: WO 2014/010325 A1

SUMMARY

(Technical Problem)

In recent years, there has been a need to improve reverse wavelength dispersion with respect to comparatively long-wavelength light. However, it has not been possible to sufficiently improve reverse wavelength dispersion at long wavelengths of optical films and the like obtained using conventional polymerizable compounds such as described in PTL 1.

The present disclosure was completed in view of the circumstances set forth above and has an objective of providing a polymer that is capable of forming an optical film or optically anisotropic body having good reverse wavelength dispersion at long wavelengths.

Another objective of the present disclosure is to provide a polymerizable liquid crystal mixture and a polymerizable compound that can be used in production of the polymer, and also to provide a compound that can be used in production of the polymerizable compound.

Yet another objective of the present disclosure is to provide an optical film and an optically anisotropic body for which reverse wavelength dispersion at long wavelengths is improved to provide excellent reverse wavelength dispersion at long wavelengths, and also to provide a polarizer, a display, and an antireflection film in which the optically anisotropic body is used.

(Solution to Problem)

As a result of diligent research conducted in order to solve the problem set forth above, the inventors discovered that by using a specific polymerizable compound indicated by formula (1-1), shown below, it is possible to obtain a polymer capable of forming an optical film or optically anisotropic body having good reverse wavelength dispersion at long wavelengths, and also discovered that through this polymer, it is possible to produce an optical film or the like for which reverse wavelength dispersion at long wavelengths is improved to provide excellent reverse wavelength dispersion at long wavelengths.

Accordingly, the present disclosure provides the following polymerizable compound, polymerizable liquid crystal mixture, polymer, optical film, optically anisotropic body, polarizer, display, antireflection film, and compound.

[1] A polymerizable compound indicated by formula (1-1), shown below,

$$P^1-L^1-Y^3\left[B^1-Y^1\right]_pA^1-Z^1-Ar^0-Z^2-Xa-Z^3-Ar^1-Z^4-A^2\left[Y^2-B^2\right]_qY^4-L^2-P^2$$

(I − 1)

where, in formula (1-1),

$Ar^0$ represents an aromatic hydrocarbon cyclic group having at least $D^0$ as a substituent or an aromatic heterocyclic group having at least $D^0$ as a substituent,
$Ar^1$ represents an aromatic hydrocarbon cyclic group having at least $D^1$ as a substituent or an aromatic heterocyclic group having at least $D^1$ as a substituent,
$D^0$ and $D^1$ each represent, independently of one another, an organic group having a carbon number of 1 to 67 and including at least one aromatic ring selected from the group consisting of an aromatic hydrocarbon ring and an aromatic heterocyclic ring,
Xa represents an optionally substituted organic group having a carbon number of 1 to 20,
$Z^1$ to $Z^4$ each represent, independently of one another, a single bond, -O-, $-O-CH_2-$, $-CH_2-O-$, -C(=O)-O-, -O-C(=O)-, -C(=O)-S-, -S-C(=O)-, $-NR^{20}C(=O)-$, $-C(=O)-NR^{20}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, $-CH_2-C(=O)-O-$, $-O-C(=O)-CH_2-$, $-CH_2-O-C(=O)-$, $-C(=O)-O-CH_2-$, $-O-CH_2-CH_2-$, $-CH_2-CH_2-O-$, or -C≡C-, where $R^{20}$ represents a hydrogen atom or an alkyl group having a carbon number of 1 to 6,
$A^1$, $A^2$, $B^1$, and $B^2$ each represent, independently of one another, an optionally substituted alicyclic group or an optionally substituted aromatic group,
$Y^1$ to $Y^4$ each represent, independently of one another, a single bond, -O-, -C(=O)-, -C(=O)-O-, -O-C(=O)-, $-NR^{21}-C(=O)-$, $-C(=O)-NR^{21}-$, -O-C(=O)-O-, $-NR^{21}-C(=O)-O-$, $-O-C(=O)-NR^{21}-$, or $-NR^{21}-C(=O)-NR^{22}-$, where $R^{21}$ and $R^{22}$ each represent, independently of one another, a hydrogen atom or an alkyl group having a carbon number of 1 to 6,
$L^1$ and $L^2$ are each, independently of one another, an organic group that is either an alkylene group having a carbon number of 1 to 20 or a group in which at least one methylene group ($-CH_2-$) included in an alkylene group having a carbon number of 1 to 20 is replaced by -O- or -C(=O)-, where hydrogen atoms included in the organic groups of $L^1$ and $L^2$ may each be replaced by an alkyl group having a carbon number of 1 to 5, an alkoxy group having a carbon number of 1 to 5, or a halogen atom, and with a proviso that methylene groups ($-CH_2-$) at both ends of $L^1$ and $L^2$ are not replaced by -O- or -C(=O)-,
one of $P^1$ and $P^2$ represents a hydrogen atom or a polymerizable group and the other of $P^1$ and $P^2$ represents a polymerizable group,
p and q are each, independently of one another, an integer of 0 to 2, and
in a case in which more than one $B^1$, $B^2$, $Y^1$, or $Y^2$ is present, each $B^1$, $B^2$, $Y^1$, or $Y^2$ may be the same or different.

[2] The polymerizable compound according to the foregoing [1], wherein $Ar^0$ and $Ar^1$ are each, independently of

one another, indicated by any one of formulae (II-1) to (II-7), shown below,

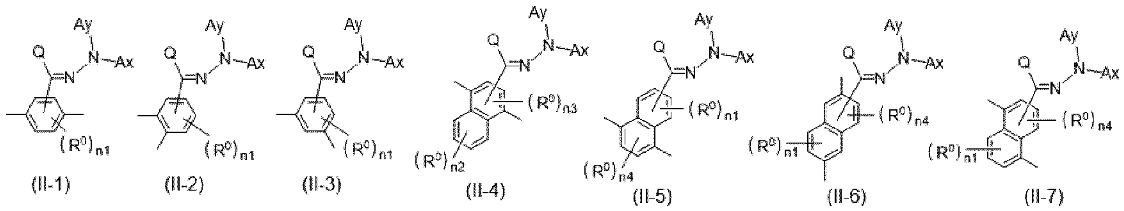

(II-1) (II-2) (II-3) (II-4) (II-5) (II-6) (II-7)

where, in formulae (II-1) to (II-7),

Ax represents an organic group including at least one aromatic ring selected from the group consisting of an aromatic hydrocarbon ring having a carbon number of 6 to 30 and an aromatic heterocyclic ring having a carbon number of 2 to 30, where the aromatic ring of Ax is optionally substituted,
Ay represents a hydrogen atom or an optionally substituted organic group having a carbon number of 1 to 30,
Q represents a hydrogen atom or an alkyl group having a carbon number of 1 to 6,
$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, $-C(=O)-R^a$, $-O-C(=O)-R^a$, $-C(=O)-O-R^a$, or $-SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, n1 is 0 to 3, n2 is 0 to 4, n3 is 0 or 1, and n4 is 0 to 2, and
in a case in which more than one $R^0$ is present, each $R^0$ may be the same or different.

[3] The polymerizable compound according to the foregoing [2], wherein the polymerizable compound is indicated by any one of formulae (III-1) to (III-6), shown below,

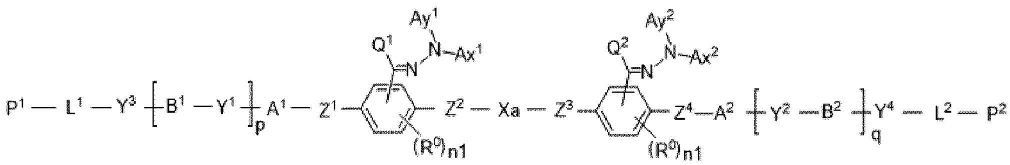

(III-1)

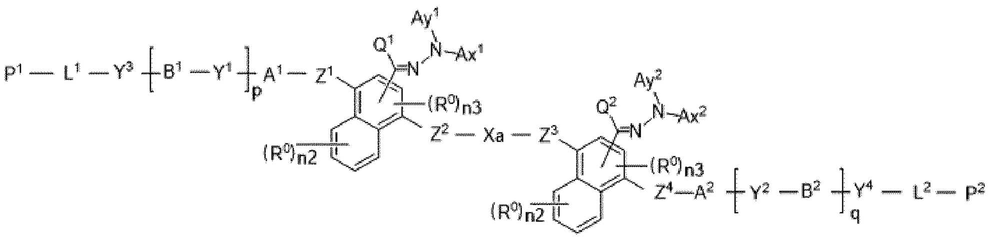

(III-2)

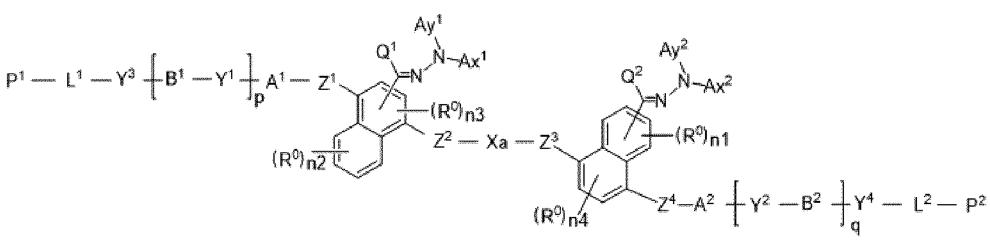

(III-3)

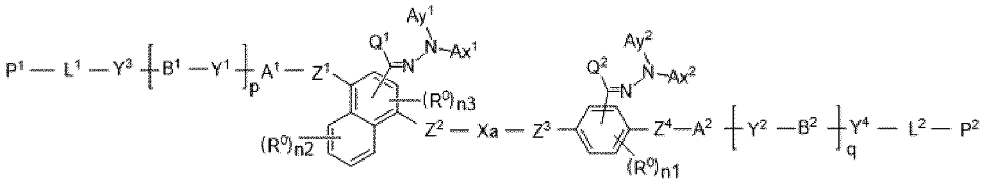

(III-4)

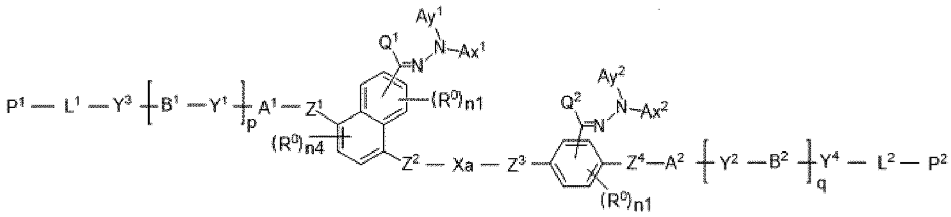

(III-5)

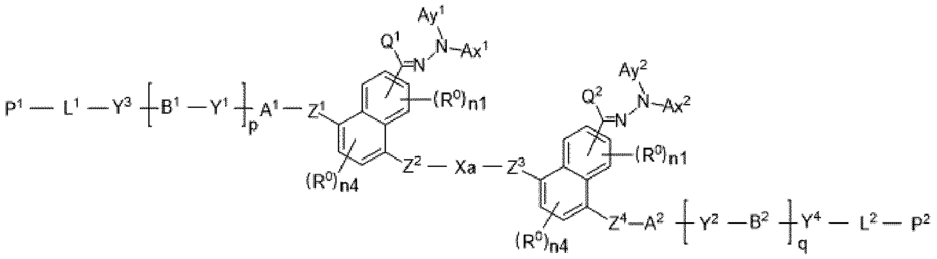

(III-6)

where, in formulae (III-1) to (III-6),

$Z^1$ to $Z^4$, $A^1$, $A^2$, $B^1$, $B^2$, $Y^1$ to $Y^4$, $L^1$, $L^2$, $P^1$, $P^2$, Xa, $R^0$, n1, n2, n3, n4, p, and q have the same meaning as previously described,
$Ax^1$ and $Ax^2$ each represent, independently of one another, an organic group including at least one aromatic ring selected from the group consisting of an aromatic hydrocarbon ring having a carbon number of 6 to 30 and an aromatic heterocyclic ring having a carbon number of 2 to 30, where the aromatic ring of each of $Ax^1$ and $Ax^2$ is optionally substituted,
$Ay^1$ and $Ay^2$ each represent, independently of one another, a hydrogen atom or an optionally substituted organic group having a carbon number of 1 to 30,
$Q^1$ and $Q^2$ each represent, independently of one another, a hydrogen atom or an alkyl group having a carbon number of 1 to 6, and
in a case in which more than one $B^1$, $B^2$, $Y^1$, $Y^2$ , or $R^0$ is present, each $B^1$, $B^2$, $Y^1$, $Y^2$, or $R^0$ may be the same or different.

[4] The polymerizable compound according to the foregoing [3], wherein $Ay^1$ and $Ay^2$ are each, independently of one another, a hydrogen atom, an optionally substituted alkyl group having a carbon number of 1 to 20, an optionally substituted alkenyl group having a carbon number of 2 to 20, an optionally substituted alkynyl group having a carbon number of 2 to 20, an optionally substituted cycloalkyl group having a carbon number of 3 to 12, an optionally substituted aromatic hydrocarbon cyclic group having a carbon number of 6 to 30, or an optionally substituted aromatic heterocyclic group having a carbon number of 2 to 30.
[5] The polymerizable compound according to the foregoing [3] or [4], wherein $Ax^1$ and $Ax^2$ are each, independently of one another, indicated by formula (V), shown below,

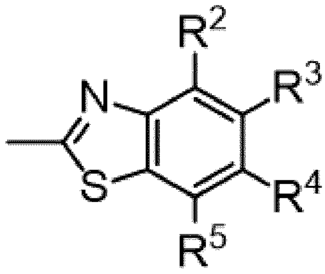

(V)

where, in formula (V), $R^2$ to $R^5$ each represent, independently of one another, a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, $-OCF_3$, $-O-C(=O)-R^b$, or $-C(=O)-O-R^b$,
$R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18, and
$R^2$ to $R^5$ may be the same or different, and one or more of ring constituents C-$R^2$ to C-$R^5$ may be replaced by a nitrogen atom.

[6] The polymerizable compound according to any one of the foregoing [1] to [5], wherein $P^1$ and $P^2$ are each, independently of one another, indicated by formula (IV), shown below,

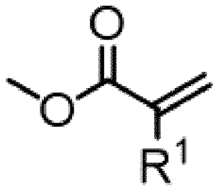

(I V)

where, in formula (IV), $R^1$ represents a hydrogen atom, a methyl group, or a chlorine atom.
[7] The polymerizable compound according to any one of the foregoing [1] to [6], wherein the polymerizable compound indicated by formula (1-1) is indicated by any one of formulae (VI-1) to (VI-3), shown below,

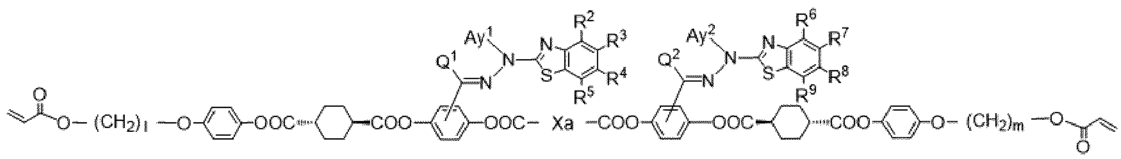

(VI-1)

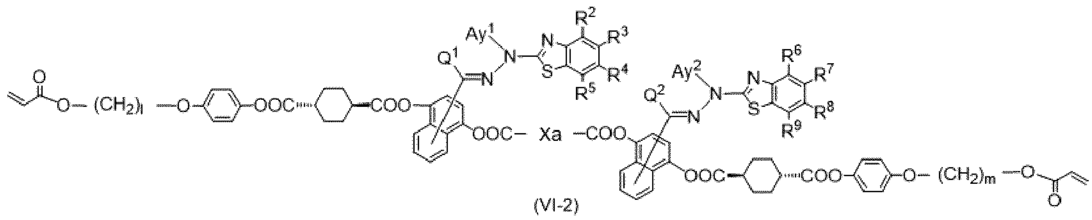

(VI-2)

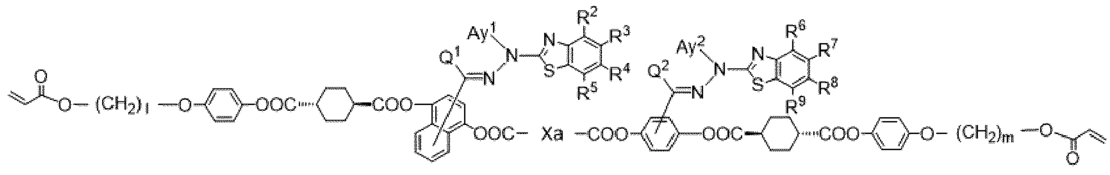

(VI-3)

where, in formulae (VI-1) to (VI-3),

Xa has the same meaning as previously described,
$R^2$ to $R^9$ each represent, independently of one another, a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, $-OCF_3$, $-O-C(=O)-R^b$, or $-C(=O)-O-R^b$,
$R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18,
one or more of ring constituents $C-R^2$ to $C-R^9$ may be replaced by a nitrogen atom,
$Ay^1$ and $Ay^2$ each represent, independently of one another, a hydrogen atom or an optionally substituted organic group having a carbon number of 1 to 30,
$Q^1$ and $Q^2$ each represent, independently of one another, a hydrogen atom or an alkyl group having a carbon number of 1 to 6, and
1 and m each represent, independently of one another, an integer of 1 to 18.

[8] The polymerizable compound according to any one of the foregoing [1] to [7], wherein Xa is represented by any one of formulae (VII-1) to (VII-29), shown below.

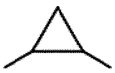
(VII-1)
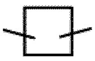
(VII-2)
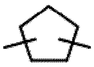
(VII-3)
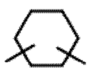
(VII-4)
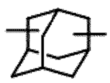
(VII-5)
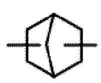
(VII-6)
$-CH_2-$
(VII-7)
$-(CH_2)_2-$
(VII-8)

$-(CH_2)_3-$ $-(CH_2)_4-$ $-(CH_2)_5-$ $-(CH_2)_6-$ $-(CH_2)_7-$ $-(CH_2)_8-$ $-(CH_2)_9-$ (VII-9) (VII-10) (VII-11) (VII-12) (VII-13) (VII-14) (VII-15) $-(CH_2)_{10}-$ $-(CH_2)_{11}-$ $-(CH_2)_{12}-$ $-(CH_2)_{13}-$ $-(CH_2)_{14}-$ $-(CH_2)_{15}-$ (VII-16) (VII-17) (VII-18) (VII-19) (VII-20) (VII-21)

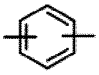
(VII-22)
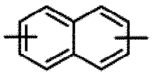
(VII-23)
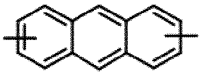
(VII-24)
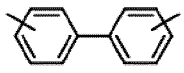
(VII-25)
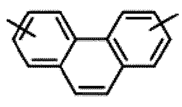
(VII-26)

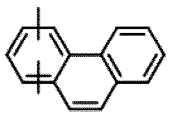
(VII-27)
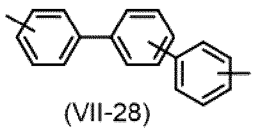
(VII-28)
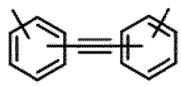
(VII-29)

[9] A polymerizable liquid crystal mixture comprising the polymerizable compound according to any one of the foregoing [1] to [8] as a main component.
[10] The polymerizable liquid crystal mixture according to the foregoing [9], comprising:

the polymerizable compound according to any one of the foregoing [1] to [8]; and
a polymerizable compound having a chemical structure differing from formula (1-1), shown below,

$$P^1-L^1-Y^3\left[B^1-Y^1\right]_pA^1-Z^1-Ar^0-Z^2-Xa-Z^3-Ar^1-Z^4-A^2\left[Y^2-B^2\right]_qY^4-L^2-P^2$$

(I − 1)

where, in formula (1-1),
$Ar^0$ represents an aromatic hydrocarbon cyclic group having at least $D^0$ as a substituent or an aromatic heterocyclic group having at least $D^0$ as a substituent,
$Ar^1$ represents an aromatic hydrocarbon cyclic group having at least $D^1$ as a substituent or an aromatic heterocyclic group having at least $D^1$ as a substituent,
$D^0$ and $D^1$ each represent, independently of one another, an organic group having a carbon number of 1 to 67 and including at least one aromatic ring selected from the group consisting of an aromatic hydrocarbon ring and an aromatic heterocyclic ring,
Xa represents an optionally substituted organic group having a carbon number of 1 to 20,
$Z^1$ to $Z^4$ each represent, independently of one another, a single bond, -O-, $-O-CH_2-$, $-CH_2-O-$, -C(=O)-O-, -O-C(=O)-, -C(=O)-S-, -S-C(=O)-, $-NR^{20}C(=O)-$, $-C(=O)-NR^{20}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, $-CH_2-C(=O)-O-$, $-O-C(=O)-CH_2-$, $-CH_2-O-C(=O)-$, $-C(=O)-O-CH_2-$, $-O-CH_2-CH_2-$, $-CH_2-CH_2-O-$, or -C≡C-, where $R^{20}$ represents a hydrogen atom or an alkyl group having a carbon number of 1 to 6,
$A^1$, $A^2$, $B^1$, and $B^2$ each represent, independently of one another, an optionally substituted alicyclic group or an optionally substituted aromatic group,
$Y^1$ to $Y^4$ each represent, independently of one another, a single bond, -O-, -C(=O)-, -C(=O)-O-, -O-C(=O)-, $-NR^{21}-C(=O)-$, $-C(=O)-NR^{21}-$, -O-C(=O)-O-, $-NR^{21}-C(=O)-O-$, $-O-C(=O)-NR^{21}-$, or $-NR^{21}-C(=O)-NR^{22}-$, where $R^{21}$ and $R^{22}$ each represent, independently of one another, a hydrogen atom or an alkyl group having a carbon number of 1 to 6,
$L^1$ and $L^2$ are each, independently of one another, an organic group that is either an alkylene group having a carbon number of 1 to 20 or a group in which at least one methylene group ($-CH_2-$) included in an alkylene group having a carbon number of 1 to 20 is replaced by -O- or -C(=O)-, where hydrogen atoms included in the organic groups of $L^1$ and $L^2$ may each be replaced by an alkyl group having a carbon number of 1 to 5, an alkoxy group having a carbon number of 1 to 5, or a halogen atom, and with a proviso that methylene groups ($-CH_2-$) at both ends of $L^1$ and $L^2$ are not replaced by -O- or -C(=O)-,
one of $P^1$ and $P^2$ represents a hydrogen atom or a polymerizable group and the other of $P^1$ and $P^2$ represents a polymerizable group,
p and q are each, independently of one another, an integer of 0 to 2, and
in a case in which more than one $B^1$, $B^2$, $Y^1$, or $Y^2$ is present, each $B^1$, $B^2$, $Y^1$, or $Y^2$ may be the same or different, wherein
an area value for the polymerizable compound according to any one of the foregoing [1] to [8] relative to a sum total of area values for the polymerizable compound according to any one of the foregoing [1] to [8] and the polymerizable compound having a chemical structure differing from formula (1-1), as measured by high-performance liquid chromatography (HPLC), is more than 50%.

[11] The polymerizable liquid crystal mixture according to the foregoing [9] or [10], comprising:

the polymerizable compound according to any one of the foregoing [1] to [8]; and
a polymerizable compound indicated by formula (I-2), shown below,

$$P^3-L^3-Y^7\left[B^3-Y^5\right]_{p1}A^3-Z^5-Ar^2-Z^6-A^4\left[Y^6-B^4\right]_{q1}Y^8-L^4-P^4$$

(I − 2)

where, in formula (I-2),
$Ar^2$ represents an aromatic hydrocarbon cyclic group having at least $D^2$ as a substituent or an aromatic heterocyclic group having at least $D^2$ as a substituent,

$D^2$ represents an organic group having a carbon number of 1 to 67 and including at least one aromatic ring selected from the group consisting of an aromatic hydrocarbon ring and an aromatic heterocyclic ring,

$Z^5$ and $Z^6$ each represent, independently of one another, a single bond, -O-, -O-$CH_2$-, -$CH_2$-O-, -C(=O)-O-, -O-C(=O)-, -C(=O)-S-, -S-C(=O)-, -$NR^{20}$-C(=O)-, -C(=O)-$NR^{20}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, -$CH_2$-C(=O)-O-, -O-C(=O)-$CH_2$-, -$CH_2$-O-C(=O)-, -C(=O)-O-$CH_2$-, -O-$CH_2$-$CH_2$-, -$CH_2$-$CH_2$-O-, or -C≡C-, where $R^{20}$ represents a hydrogen atom or an alkyl group having a carbon number of 1 to 6,

$A^3$, $A^4$, $B^3$, and $B^4$ each represent, independently of one another, an optionally substituted alicyclic group or an optionally substituted aromatic group,

$Y^5$ to $Y^8$ each represent, independently of one another, a single bond, -O-, -C(=O)-, -C(=O)-O-, -O-C(=O)-, -$NR^{21}$-C(=O)-, -C(=O)-$NR^{21}$-, -O-C(=O)-O-, -$NR^{21}$-C(=O)-O-, -O-C(=O)-$NR^{21}$-, or -$NR^{21}$-C(=O)-$NR^{22}$-, where $R^{21}$ and $R^{22}$ each represent, independently of one another, a hydrogen atom or an alkyl group having a carbon number of 1 to 6,

$L^3$ and $L^4$ are each, independently of one another, an organic group that is either an alkylene group having a carbon number of 1 to 20 or a group in which at least one methylene group (-$CH_2$-) included in an alkylene group having a carbon number of 1 to 20 is replaced by -O- or -C(=O)-, where hydrogen atoms included in the organic groups of $L^3$ and $L^4$ may each be replaced by an alkyl group having a carbon number of 1 to 5, an alkoxy group having a carbon number of 1 to 5, or a halogen atom, and with a proviso that methylene groups (-$CH_2$-) at both ends of $L^3$ and $L^4$ are not replaced by -O- or -C(=O)-,

one of $P^3$ and $P^4$ represents a hydrogen atom or a polymerizable group and the other of $P^3$ and $P^4$ represents a polymerizable group,

p1 and q1 are each, independently of one another, an integer of 0 to 2, and

in a case in which more than one $B^3$, $B^4$, $Y^5$, or $Y^6$ is present, each $B^3$, $B^4$, $Y^5$, or $Y^6$ may be the same or different, wherein

an area value for the polymerizable compound according to any one of the foregoing [1] to [8] relative to a sum total of area values for the polymerizable compound according to any one of the foregoing [1] to [8] and the polymerizable compound indicated by formula (I-2), as measured by high-performance liquid chromatography (HPLC), is more than 50%.

[12] The polymerizable liquid crystal mixture according to the foregoing [11], wherein $Ar^2$ is indicated by any one of formulae (II-1) to (II-7), shown below,

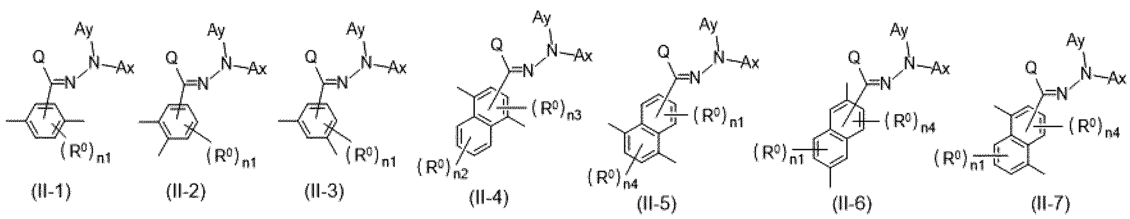

(II-1) (II-2) (II-3) (II-4) (II-5) (II-6) (II-7)

where, in formulae (II-1) to (II-7),

Ax represents an organic group including at least one aromatic ring selected from the group consisting of an aromatic hydrocarbon ring having a carbon number of 6 to 30 and an aromatic heterocyclic ring having a carbon number of 2 to 30, where the aromatic ring of Ax is optionally substituted,

Ay represents a hydrogen atom or an optionally substituted organic group having a carbon number of 1 to 30,

Q represents a hydrogen atom or an alkyl group having a carbon number of 1 to 6,

$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, -C(=O)-$R^a$, -O-C(=O)-$R^a$, -C(=O)-O-$R^a$, or -$SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, n1 is an integer of 0 to 3, n2 is an integer of 0 to 4, n3 is 0 or 1, and n4 is an integer of 0 to 2, and

in a case in which more than one $R^0$ is present, each $R^0$ may be the same or different.

[13] The polymerizable liquid crystal mixture according to the foregoing [11] or [12], wherein $P^3$ and $P^4$ are each,

independently of one another, indicated by formula (IV), shown below,

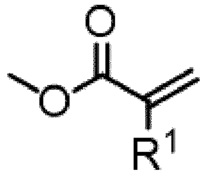

(IV)

where, in formula (IV), $R^1$ represents a hydrogen atom, a methyl group, or a chlorine atom.

[14] A polymer obtained by polymerizing the polymerizable liquid crystal mixture according to any one of the foregoing [9] to [13].

[15] An optical film comprising the polymer according to the foregoing [14] as a constituent material.

[16] An optically anisotropic body comprising a layer having the polymer according to the foregoing [14] as a constituent material.

[17] A polarizer comprising:

the optically anisotropic body according to the foregoing [16]; and
a polarizing film.

[18] A display comprising the polarizer according to the foregoing [17].

[19] An antireflection film comprising the polarizer according to the foregoing [17].

[20] A compound indicated by formula (VIII-1), shown below,

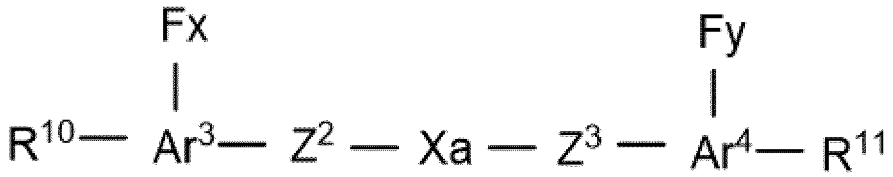

(VIII-1)

where, in formula (VIII-1),

$Ar^3$ and $Ar^4$ each represent, independently of one another, an aromatic hydrocarbon cyclic group or an aromatic heterocyclic group,

Xa represents an optionally substituted organic group having a carbon number of 1 to 20,

$Z^2$ and $Z^3$ each represent, independently of one another, a single bond, -O-, -O-$CH_2$-, -$CH_2$-O-, -C(=O)-O-, -O-C(=O)-, -C(=O)-S-, -S-C(=O)-, -$NR^{20}$-C(=O)-, -C(=O)-$NR^{20}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, -$CH_2$-C(=O)-O-, -O-C(=O)-$CH_2$-, -$CH_2$-O-C(=O)-, -C(=O)-O-$CH_2$-, -O-$CH_2$-$CH_2$-, -$CH_2$-$CH_2$-O-, or -C≡C-, where $R^{20}$ represents a hydrogen atom or an alkyl group having a carbon number of 1 to 6,

Fx and Fy each represent, independently of one another, -C($R^f$)=N-N($R^g$)$R^h$, -C($R^f$)=N-N=C($R^{g1}$)$R^h$, or -CHO, where $R^f$ represents a hydrogen atom or an alkyl group having a carbon number of 1 to 6, $R^g$ and $R^{g1}$ each represent, independently of one another, a hydrogen atom or an optionally substituted organic group having a carbon number of 1 to 30, and $R^h$ represents an organic group including at least one aromatic ring selected from the group consisting of an aromatic hydrocarbon ring having a carbon number of 6 to 30 and an aromatic heterocyclic ring having a carbon number of 2 to 30, and

$R^{10}$ and $R^{11}$ each represent, independently of one another, -$OR^p$, -$CH_2OR^p$, -$CH_2CH_2OR^p$, -C(=O)-$OR^p$, -$CH_2$-C(=O)-$OR^p$, -$CH_2CH_2$-C(=O)-$OR^p$, a hydroxy group, a carboxyl group, -$CH_2$-C(=O)-OH, -$CH_2CH_2$-C(=O)-OH, -$CH_2$OH, -$CH_2CH_2$OH, or an amino group, where $R^p$ represents a protecting group.

[21] The compound according to the foregoing [20], wherein $Ar^3$-Fx and $Ar^4$-Fy are each, independently of one another, indicated by any one of formulae (IX-1) to (IX-14), shown below,

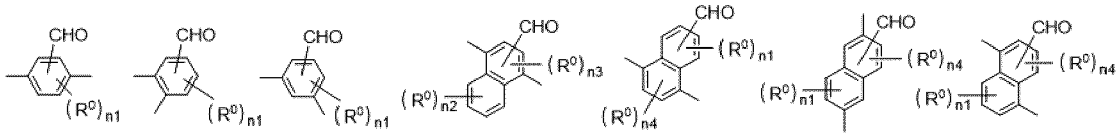

(IX-1) (IX-2) (IX-3) (IX-4) (IX-5) (IX-6) (IX-7)

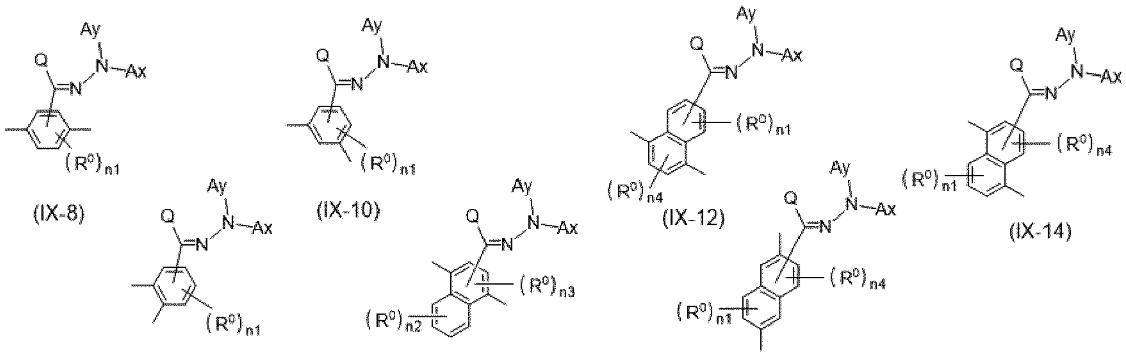

(IX-8) (IX-9) (IX-10) (IX-11) (IX-12) (IX-13) (IX-14)

where, in formulae (IX-1) to (IX-14),

Ax represents an organic group including at least one aromatic ring selected from the group consisting of an aromatic hydrocarbon ring having a carbon number of 6 to 30 and an aromatic heterocyclic ring having a carbon number of 2 to 30, where the aromatic ring of Ax is optionally substituted,
Ay represents a hydrogen atom or an optionally substituted organic group having a carbon number of 1 to 30,
Q represents a hydrogen atom or an alkyl group having a carbon number of 1 to 6,
$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, $-C(=O)-R^a$, $-O-C(=O)-R^a$, $-C(=O)-O-R^a$, or $-SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, n1 is an integer of 0 to 3, n2 is an integer of 0 to 4, n3 is 0 or 1, and n4 is an integer of 0 to 2, and
in a case in which more than one $R^0$ is present, each $R^0$ may be the same or different.

[22] The compound according to the foregoing [21], wherein the compound is indicated by any one of formulae (X-1) to (X-12), shown below,

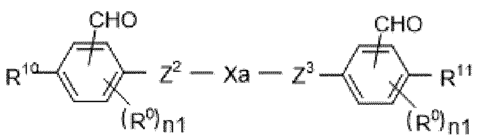 (X-1)

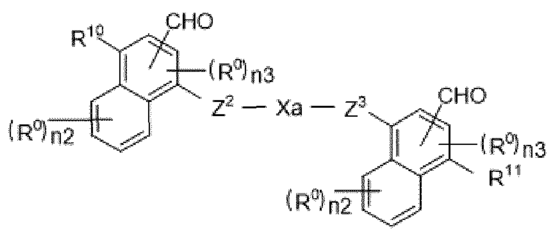 (X-2)

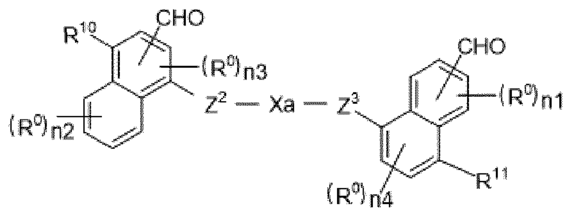

(X-3)

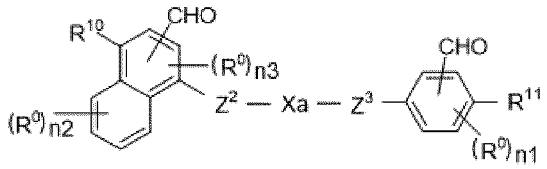

(X-4)

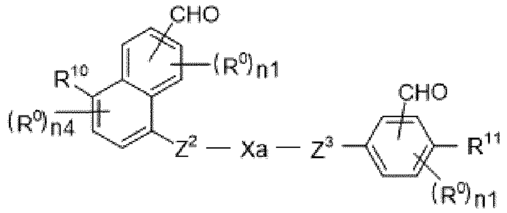

(X-5)

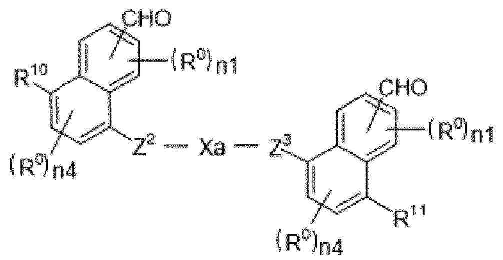

(X-6)

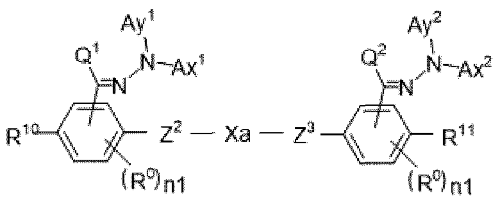

(X-7)

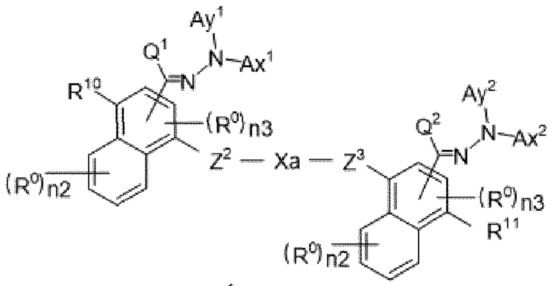

(X-8)

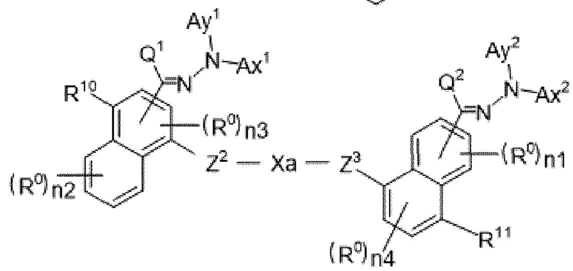

(X-9)

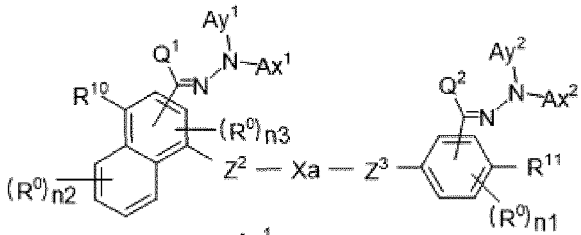

(X-10)

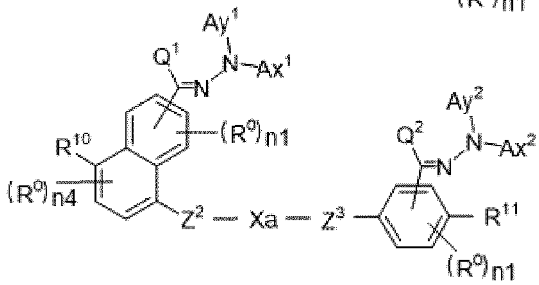

(X-11)

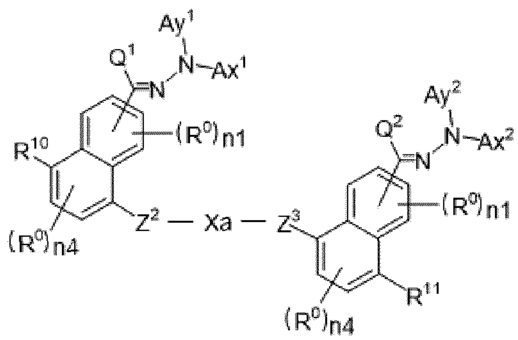

(X-12)

where, in formulae (X-1) to (X-12),

Xa, $Z^2$, $Z^3$, $R^{10}$, $R^{11}$, $R^0$, n1, n2, n3, and n4 have the same meaning as previously described,

$Ax^1$ and $Ax^2$ each represent, independently of one another, an organic group including at least one aromatic ring selected from the group consisting of an aromatic hydrocarbon ring having a carbon number of 6 to 30 and an aromatic heterocyclic ring having a carbon number of 2 to 30, where the aromatic ring of each of $Ax^1$ and $Ax^2$ is optionally substituted,

$Ay^1$ and $Ay^2$ each represent, independently of one another, a hydrogen atom or an optionally substituted organic group having a carbon number of 1 to 30,

$Q^1$ and $Q^2$ each represent, independently of one another, a hydrogen atom or an alkyl group having a carbon number of 1 to 6, and
in a case in which more than one $R^0$ is present, each $R^0$ may be the same or different.

[23] A compound indicated by any one of formulae (XI-1) to (XI-6), shown below,

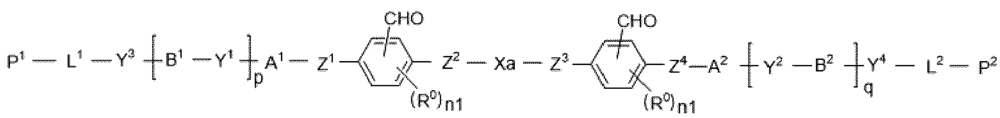
(XI-1)

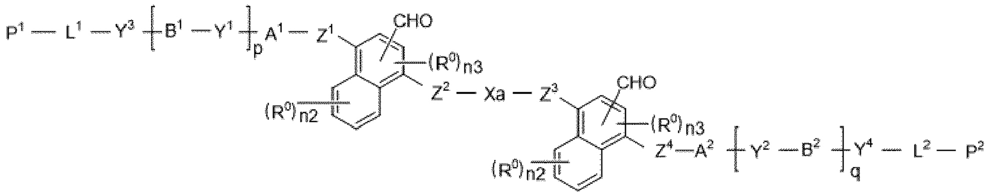
(XI-2)

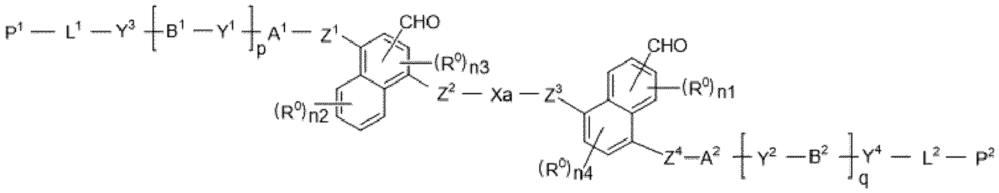
(XI-3)

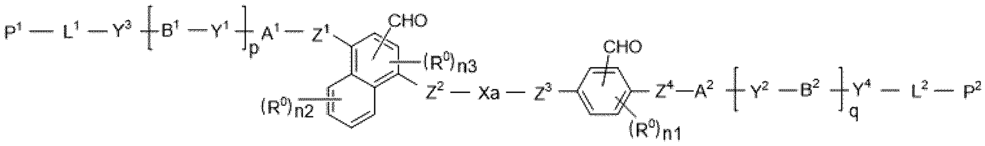
(XI-4)

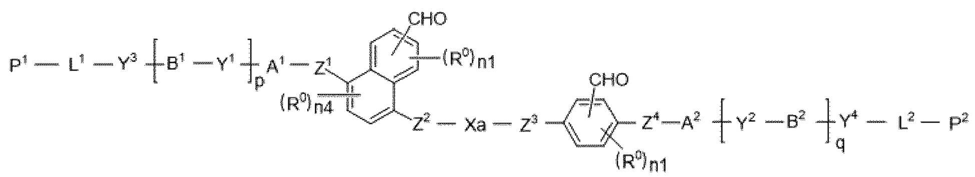
(XI-5)

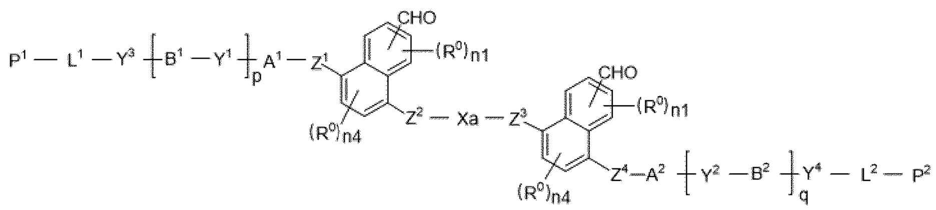
(XI-6)

where, in formulae (XI-1) to (XI-6),

Xa represents an optionally substituted organic group having a carbon number of 1 to 20,
$Z^1$ to $Z^4$ each represent, independently of one another, a single bond, -O-, -O-$CH_2$-, -$CH_2$-O-, -C(=O)-O-, -O-C(=O)-, -C(=O)-S-, -S-C(=O)-, -$NR^{20}$-C(=O)-, -C(=O)-$NR^{20}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, -$CH_2$-C(=O)-O-, -O-C(=O)-$CH_2$-, -$CH_2$-O-C(=O)-, -C(=O)-O-$CH_2$-, -O-$CH_2$-$CH_2$-, -$CH_2$-$CH_2$-O-, or -C≡C-, where $R^{20}$ represents a hydrogen atom or an alkyl group having a carbon number of 1 to 6,

$A^1$, $A^2$, $B^1$, and $B^2$ each represent, independently of one another, an optionally substituted alicyclic group or an optionally substituted aromatic group,

$Y^1$ to $Y^4$ each represent, independently of one another, a single bond, -O-, -C(=O)-, -C(=O)-O-, -O-C(=O)-, $-NR^{21}-C(=O)-$, $-C(=O)-NR^{21}-$, -O-C(=O)-O-, $-NR^{21}-C(=O)-O-$, $-O-C(=O)-NR^{21}-$, or $-NR^{21}-C(=O)-NR^{22}-$, where $R^{21}$ and $R^{22}$ each represent, independently of one another, a hydrogen atom or an alkyl group having a carbon number of 1 to 6,

$L^1$ and $L^2$ are each, independently of one another, an organic group that is either an alkylene group having a carbon number of 1 to 20 or a group in which at least one methylene group ($-CH_2-$) included in an alkylene group having a carbon number of 1 to 20 is replaced by -O- or -C(=O)-, where hydrogen atoms included in the organic groups of $L^1$ and $L^2$ may each be replaced by an alkyl group having a carbon number of 1 to 5, an alkoxy group having a carbon number of 1 to 5, or a halogen atom, and with a proviso that methylene groups ($-CH_2-$) at both ends of $L^1$ and $L^2$ are not replaced by -O- or -C(=O)-,

one of $P^1$ and $P^2$ represents a hydrogen atom or a polymerizable group and the other of $P^1$ and $P^2$ represents a polymerizable group,

p and q are each, independently of one another, an integer of 0 to 2,

$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, $-C(=O)-R^a$, $-O-C(=O)-R^a$, $-C(=O)-O-R^a$, or $-SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, n1 is an integer of 0 to 3, n2 is an integer of 0 to 4, n3 is 0 or 1, and n4 is an integer of 0 to 2, and

in a case in which more than one $R^0$, $B^1$, $B^2$, $Y^1$, or $Y^2$ is present, each $R^0$, $B^1$, $B^2$, $Y^1$, or $Y^2$ may be the same or different.

[24] The compound according to the foregoing [23], wherein the compound is indicated by any one of formulae (XII-1) to (XII-3), shown below,

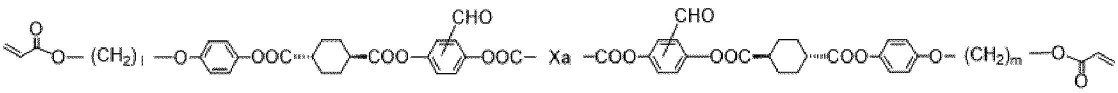

(XII-1)

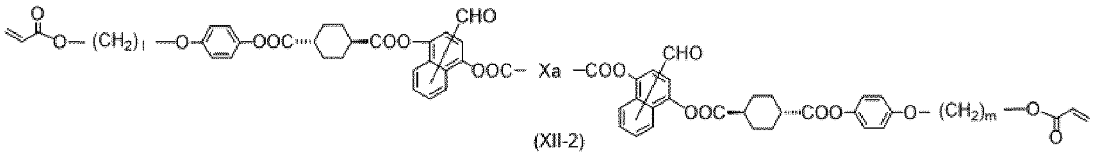

(XII-2)

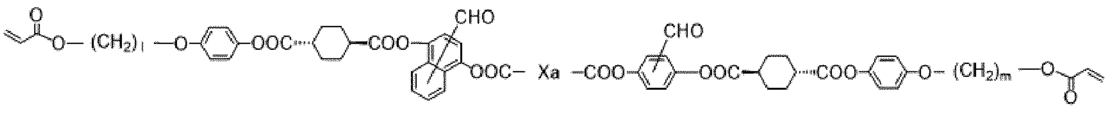

(XII-3)

where, in formulae (XII-1) to (XII-3),

Xa has the same meaning as previously described, and

1 and m each represent, independently of one another, an integer of 1 to 18.

(Advantageous Effect)

The present disclosure provides a polymer capable of forming an optical film or optically anisotropic body having good reverse wavelength dispersion at long wavelengths, and also a polymerizable compound, a polymerizable liquid crystal mixture, and a mixture that are useful in production of the polymer.

Moreover, the present disclosure provides a compound that is useful in production of the polymerizable compound.

Furthermore, the present disclosure provides an optical film and an optically anisotropic body for which reverse

wavelength dispersion at long wavelengths is improved to provide excellent reverse wavelength dispersion at long wavelengths, and also a polarizer, a display, and an antireflection film in which the optically anisotropic body is used.

## DETAILED DESCRIPTION

The following provides a detailed description of the present disclosure. Note that the phrase "optionally substituted" as used in the present disclosure means "unsubstituted or having one or more substituents". Also note that in a case in which an organic group (for example, an alkyl group or an aromatic hydrocarbon cyclic group) included in a general formula has a substituent, the carbon number of the organic group having the substituent is taken to be exclusive of the carbon number of the substituent. For example, in a case in which an aromatic hydrocarbon cyclic group having a carbon number of 6 to 20 has a substituent, the carbon number of the aromatic hydrocarbon cyclic group having a carbon number of 6 to 20 is taken to be exclusive of the carbon number of the substituent. Moreover, the term "alkyl group" as used in the present disclosure refers to chain (linear or branched) saturated hydrocarbon groups and is not inclusive of "cycloalkyl groups", which are cyclic saturated hydrocarbon groups.
A presently disclosed polymerizable compound, a presently disclosed polymerizable liquid crystal mixture, and a presently disclosed mixture can be used in production of a presently disclosed polymer, for example, but are not specifically limited to being using in this manner.
Moreover, the presently disclosed polymer can be used as a constituent material of a presently disclosed optical film or as a constituent material of a layer included in a presently disclosed optically anisotropic body, for example, but is not specifically limited to being used in this manner. Furthermore, the presently disclosed optically anisotropic body can be used in production of a presently disclosed polarizer, for example, but is not specifically limited to being used in this manner. Also, the presently disclosed polarizer can be used in production of a presently disclosed display or a presently disclosed antireflection film, for example, but is not specifically limited to being used in this manner.
Moreover, a presently disclosed compound (intermediate) can be used in production of the presently disclosed polymerizable compound, for example, but is not specifically limited to being used in this manner.

### (1) Polymerizable compound

The presently disclosed polymerizable compound is a compound indicated by the following formula (1-1) (hereinafter, also referred to as polymerizable compound (1-1)) and can advantageously be used in production of a polymer, an optical film, and an optically anisotropic body described further below.

$$P^1 - L^1 - Y^3 \left[ B^1 - Y^1 \right]_p A^1 - Z^1 - Ar^0 - Z^2 - Xa - Z^3 - Ar^1 - Z^4 - A^2 \left[ Y^2 - B^2 \right]_q Y^4 - L^2 - P^2$$

( I − 1 )

By using the polymerizable compound (I-1), it is possible to advantageously produce an optical film or the like having good reverse wavelength dispersion at long wavelengths as described further below.
In formula (I-1), $Ar^0$ is an aromatic hydrocarbon cyclic group having at least $D^0$ as a substituent or an aromatic heterocyclic group having at least $D^0$ s a substituent.
Moreover, $Ar^1$ is an aromatic hydrocarbon cyclic group having at least $D^1$ as a substituent or an aromatic heterocyclic group having at least $D^1$ as a substituent.
$D^0$ and $D^1$ are each, independently of one another, an organic group having a carbon number of 1 to 67 (preferably 2 to 67) and including at least one aromatic ring selected from the group consisting of an aromatic hydrocarbon ring and an aromatic heterocyclic ring. In other words, each of $D^0$ and $D^1$ may be composed of only an aromatic ring or may be composed of an aromatic ring-containing organic group.
Examples of the aromatic hydrocarbon cyclic group of $Ar^0$ and $Ar^1$ include a 1,4-phenylene group, a 1,3-phenylene group, a 1,4-naphthylene group, a 2,6-naphthylene group, a 1,5-naphthylene group, an anthracenyl-9,10-diyl group, an anthracenyl-1,4-diyl group, and an anthracenyl-2,6-diyl group.
Of these aromatic hydrocarbon cyclic groups, a 1,4-phenylene group, a 1,4-naphthylene group, or a 2,6-naphthylene group is preferable, and a 1,4-phenylene group is particularly preferable.
Examples of the aromatic heterocyclic group of $Ar^0$ and $Ar^1$ include a benzothiazole-4,7-diyl group, a 1,2-benzisothiazole-4,7-diyl group, a benzoxazole-4,7-diyl group, an indonyl-4,7-diyl group, a benzimidazole-4,7-diyl group, a benzopyrazole-4,7-diyl group, a 1-benzofuran-4,7-diyl group, a 2-benzofuran-4,7-diyl group, a benzo[1,2-d:4,5-d']dithiazolyl-4,8-diyl group, a benzo[1,2-d:5,4-d']dithiazolyl-4,8-diyl group, a benzothiophenyl-4,7-diyl group, a 1H-isoindole-1,3(2H)-dione-4,7-diyl group, a benzo[1,2-b:5,4-b']dithiophenyl-4,8-diyl group, a benzo[1,2-b:4,5-b']dithiophenyl-4,8-diyl

group, a benzo[1,2-b:5,4-b']difuranyl-4,8-diyl group, a benzo[1,2-b:4,5-b']difuranyl-4,8-diyl group, a benzo[2,1-b:4,5-b']dipyrrole-4,8-diyl group, a benzo[1,2-b:5,4-b']dipyrrole-4,8-diyl group, and a benzo[1,2-d:4,5-d']diimidazole-4,8-diyl group.

Of these aromatic heterocyclic groups, a benzothiazole-4,7-diyl group, a benzoxazole-4,7-diyl group, a 1-benzofuran-4,7-diyl group, a 2-benzofuran-4,7-diyl group, a benzo[1,2-d:4,5-d']dithiazolyl-4,8-diyl group, a benzo[1,2-d:5,4-d']dithiazolyl-4,8-diyl group, a benzothiophenyl-4,7-diyl group, a 1H-isoindole-1,3(2H)-dione-4,7-diyl group, a benzo[1,2-b:5,4-b']dithiophenyl-4,8-diyl group, a benzo[1,2-b:4,5-b']dithiophenyl-4,8-diyl group, a benzo[1,2-b:5,4-b']difuranyl-4,8-diyl group, or a benzo[1,2-b:4,5-b']difuranyl-4,8-diyl group is preferable.

The aromatic hydrocarbon cyclic group and the aromatic heterocyclic group of $Ar^0$ and $Ar^1$ may have a subsequently described substituent $R^0$ in addition to $D^0$ or $D^1$.

The term "aromatic ring" as used in the present description refers to a cyclic structure having aromaticity in the broad sense according to Huckel's law. In other words, "aromatic ring" refers to cyclic conjugated structures including 4n + 2 $\pi$-electrons and cyclic structures that display aromaticity through the contribution of a lone pair of electrons of a heteroatom such as sulfur, oxygen, or nitrogen to the $\pi$-electron system, representative examples of which include thiophenes, furans, and benzothiazoles.

The number of $\pi$-electrons included in each of $Ar^0$ and $Ar^1$ is normally 12 or more, preferably not less than 12 and not more than 36, and more preferably not less than 12 and not more than 30.

Examples of the aromatic hydrocarbon ring of $D^0$ and $D^1$ include a benzene ring, a naphthalene ring, an anthracene ring, a phenanthrene ring, a pyrene ring, and a fluorene ring.

Of these aromatic hydrocarbon rings, a benzene ring, a naphthalene ring, or an anthracene ring is preferable.

Examples of the aromatic heterocyclic ring of $D^0$ and $D^1$ include a 1H-isoindole-1,3(2H)-dione ring, a 1-benzofuran ring, a 2-benzofuran ring, an acridine ring, an isoquinoline ring, an imidazole ring, an indole ring, an oxadiazole ring, an oxazole ring, an oxazolopyrazine ring, an oxazolopyridine ring, an oxazolopyridazyl ring, an oxazolopyrimidine ring, a quinazoline ring, a quinoxaline ring, a quinoline ring, a cinnoline ring, a thiadiazole ring, a thiazole ring, a thiazolopyrazine ring, a thiazolopyridine ring, a thiazolopyridazine ring, a thiazolopyrimidine ring, a thiophene ring, a triazine ring, a triazole ring, a naphthyridine ring, a pyrazine ring, a pyrazole ring, a pyranone ring, a pyran ring, a pyridine ring, a pyridazine ring, a pyrimidine ring, a pyrrole ring, a phenanthridine ring, a phthalazine ring, a furan ring, a benzo[c]thiophene ring, a benzisoxazole ring, a benzisothiazole ring, a benzimidazole ring, a benzoxadiazole ring, a benzoxazole ring, a benzothiadiazole ring, a benzothiazole ring, a benzothiophene ring, a benzotriazine ring, a benzotriazole ring, a benzopyrazole ring, a benzopyranone ring, a dihydropyran ring, a tetrahydropyran ring, a dihydrofuran ring, and a tetrahydrofuran ring.

Of these aromatic heterocyclic rings, a monocyclic aromatic heterocyclic ring such as a furan ring, a pyran ring, a thiophene ring, an oxazole ring, an oxadiazole ring, a thiazole ring, or a thiadiazole ring, or a fused ring aromatic heterocyclic ring such as a benzothiazole ring, a benzoxazole ring, a quinoline ring, a 1-benzofuran ring, a 2-benzofuran ring, a benzothiophene ring, a 1H-isoindole-1,3(2H)-dione ring, a benzo[c]thiophene ring, a thiazolopyridine ring, a thiazolopyrazine ring, a benzisoxazole ring, a benzoxadiazole ring, or a benzothiadiazole ring is preferable.

The organic group constituting $D^0$ or $D^1$ that has a carbon number of 1 to 67 and includes at least one aromatic ring selected from the group consisting of an aromatic hydrocarbon ring and an aromatic heterocyclic ring may be, but is not specifically limited to, an optionally substituted aromatic hydrocarbon cyclic group, an optionally substituted aromatic heterocyclic group, or a group represented by $-C(R^f)=N-N(R^g)R^h$ or $-C(R^f)=N-N=C(R^{g1})R^h$.

In the preceding formulae, $R^f$ represents a hydrogen atom or an alkyl group having a carbon number of 1 to 6 such as a methyl group, an ethyl group, a propyl group, or an isopropyl group.

Moreover, $R^g$ and $R^{g1}$ in the preceding formulae each represent, independently of one another, a hydrogen atom or an optionally substituted organic group having a carbon number of 1 to 30. Examples of the organic group having a carbon number of 1 to 30 and substituents thereof include the same as subsequently listed as specific examples for an organic group of Ay having a carbon number of 1 to 30 and substituents thereof.

Furthermore, $R^h$ in the preceding formulae represents an organic group including at least one aromatic ring selected from the group consisting of an aromatic hydrocarbon ring having a carbon number of 6 to 30 and an aromatic heterocyclic ring having a carbon number of 2 to 30. Specific examples of the organic group including at least one aromatic ring selected from the group consisting of an aromatic hydrocarbon ring having a carbon number of 6 to 30 and an aromatic heterocyclic ring having a carbon number of 2 to 30 include the same as subsequently listed as specific examples for an organic group of Ax including at least one aromatic ring selected from the group consisting of an aromatic hydrocarbon ring having a carbon number of 6 to 30 and an aromatic heterocyclic ring having a carbon number of 2 to 30.

Specific examples of aromatic hydrocarbon cyclic groups that may constitute $D^0$ or $D^1$ include a phenyl group, a naphthyl group, an anthracenyl group, a phenanthrenyl group, a pyrenyl group, and a fluorenyl group.

Of these aromatic hydrocarbon cyclic groups, a phenyl group, a naphthyl group, or an anthracenyl group is preferable.

Examples of aromatic heterocyclic groups that may constitute $D^0$ or $D^1$ include a phthalimide group, a 1-benzofuranyl group, a 2-benzofuranyl group, an acridinyl group, an isoquinolinyl group, an imidazolyl group, an indolinyl group, a furazanyl group, an oxazolyl group, an oxazolopyrazinyl group, an oxazolopyridinyl group, an oxazolopyridazinyl group, an oxazolopyrimidinyl group, a quinazolinyl group, a quinoxalinyl group, a quinolyl group, a cinnolinyl group, a thiadiazolyl group, a thiazolyl group, a thiazolopyrazinyl group, a thiazolopyridyl group, a thiazolopyridazinyl group, a thiazolopyrimidinyl group, a thienyl group, a triazinyl group, a triazolyl group, a naphthyridinyl group, a pyrazinyl group, a pyrazolyl group, a pyranonyl group, a pyranyl group, a pyridyl group, a pyridazinyl group, a pyrimidinyl group, a pyrrolyl group, a phenanthridinyl group, a phthalazinyl group, a furanyl group, a benzo[c]thienyl group, a benzisoxazolyl group, a benzisothiazolyl group, a benzimidazolyl group, a benzoxadiazolyl group, a benzoxazolyl group, a benzothiadiazolyl group, a benzothiazolyl group, a benzothienyl group, a benzotriazinyl group, a benzotriazolyl group, a benzopyrazolyl group, a benzopyranonyl group, a dihydropyranyl group, a tetrahydropyranyl group, a dihydrofuranyl group, and a tetrahydrofuranyl group.

Of these aromatic heterocyclic groups, a monocyclic aromatic heterocyclic group such as a furanyl group, a pyranyl group, a thienyl group, an oxazolyl group, a furazanyl group, a thiazolyl group, or a thiadiazolyl group, or a fused ring aromatic heterocyclic group such as a benzothiazolyl group, a benzoxazolyl group, a quinolyl group, a 1-benzofuranyl group, a 2-benzofuranyl group, a benzothienyl group, a phthalimide group, a benzo[c]thienyl group, a thiazolopyridyl group, a thiazolopyrazinyl group, a benzisoxazolyl group, a benzoxadiazolyl group, or a benzothiadiazolyl group is preferable.

The aromatic hydrocarbon ring and the aromatic heterocyclic ring of $D^0$ and $D^1$ and the aromatic hydrocarbon cyclic group and the aromatic heterocyclic group constituting $D^0$ or $D^1$ may have one or more substituents.

Examples of these substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon number of 1 to 6 such as a trifluoromethyl group; N,N-dialkylamino groups having a carbon number of 2 to 12 such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; $-OCF_3$; $-C(=O)-R^{b1}$; $-O-C(=O)-R^{b1}$; $-C(=O)-O-R^{b1}$; and $-SO_2R^a$.

$R^{b1}$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18.

$R^a$ represents an alkyl group having a carbon number of 1 to 6 such as a methyl group or an ethyl group; or an aromatic hydrocarbon cyclic group having a carbon number of 6 to 20 that is optionally substituted with an alkyl group having a carbon number of 1 to 6 or an alkoxy group having a carbon number of 1 to 6 (for example, a phenyl group, a 4-methylphenyl group, or a 4-methoxyphenyl group).

Of these examples, halogen atoms, a cyano group, a nitro group, alkyl groups having a carbon number of 1 to 6, alkoxy groups having a carbon number of 1 to 6, and haloalkyl groups having a carbon number of 1 to 6 are preferable as substituents of the aromatic hydrocarbon ring and the aromatic heterocyclic ring of $D^0$ and $D^1$ and of the aromatic ring included in the aromatic hydrocarbon cyclic group and the aromatic heterocyclic group constituting $D^0$ or $D^1$.

The aromatic hydrocarbon ring and the aromatic heterocyclic ring of $D^0$ and $D^1$ and the aromatic hydrocarbon cyclic group and the aromatic heterocyclic group constituting $D^0$ or $D^1$ may have a plurality of substituents selected from the substituents described above. In a case in which the aromatic hydrocarbon ring, aromatic heterocyclic ring, aromatic hydrocarbon cyclic group, or aromatic heterocyclic group includes a plurality of substituents, these substituents may be the same or different.

Examples of the alkyl group having a carbon number of 1 to 20 and substituents thereof in the optionally substituted alkyl group having a carbon number of 1 to 20 of $R^{b1}$, the alkenyl group having a carbon number of 2 to 20 and substituents thereof in the optionally substituted alkenyl group having a carbon number of 2 to 20 of $R^{b1}$, the cycloalkyl group having a carbon number of 3 to 12 and substituents thereof in the optionally substituted cycloalkyl group having a carbon number of 3 to 12 of $R^{b1}$, and the aromatic hydrocarbon cyclic group having a carbon number of 5 to 18 and substituents thereof in the optionally substituted aromatic hydrocarbon cyclic group having a carbon number of 5 to 18 of $R^{b1}$ include the same as subsequently listed as specific examples for an alkyl group having a carbon number of 1 to 20 and substituents thereof in an optionally substituted alkyl group having a carbon number of 1 to 20 of $R^b$, an alkenyl group having a carbon number of 2 to 20 and substituents thereof in an optionally substituted alkenyl group having a carbon number of 2 to 20 of $R^b$, a cycloalkyl group having a carbon number of 3 to 12 and substituents thereof in an optionally substituted cycloalkyl group having a carbon number of 3 to 12 of $R^b$, and an aromatic hydrocarbon cyclic group having a carbon number of 5 to 18 and substituents thereof in an optionally substituted aromatic hydrocarbon cyclic group having a carbon number of 5 to 18 of $R^b$.

$Ar^0$ and $Ar^1$ set forth above may, for example, each be, independently of one another, a phenylene group substituted with a group represented by $-C(R^f)=N-N(R^g)R^h$ or $-C(R^f)=N-N=C(R^{g1})R^h$, a benzothiazole-4,7-diyl group substituted with a 1-benzofuran-2-yl group, a benzothiazole-4,7-diyl group substituted with a 5-(2-butyl)-1-benzofuran-2-yl group, a benzothiazole-4,7-diyl group substituted with a 4,6-dimethyl-1-benzofuran-2-yl group, a benzothiazole-4,7-diyl group substituted with a 6-methyl-1-benzofuran-2-yl group, a benzothiazole-4,7-diyl group substituted with a 4,6,7-trimethyl-1-benzofuran-2-yl group, a benzothiazole-4,7-diyl group substituted with a 4,5,6-trimethyl-1-benzofuran-2-yl group, a benzothiazole-4,7-diyl group substituted with a 5-methyl-1-benzofuran-2-yl group, a benzothiazole-4,7-diyl group substituted with a 5-propyl-1-benzofuran-2-yl group, a benzothiazole-4,7-diyl group substituted with a 7-propyl-1-benzofuran-2-yl group, a benzothiazole-4,7-diyl group substituted with a 5-fluoro-1-benzofuran-2-yl group, a benzothiazole-4,7-diyl group substituted with a phenyl group, a benzothiazole-4,7-diyl group substituted with a 4-fluorophenyl group, a benzothiazole-4,7-diyl group substituted with a 4-nitrophenyl group, a benzothiazole-4,7-diyl group substituted with a 4-trifluoromethylphenyl group, a benzothiazole-4,7-diyl group substituted with a 4-cyanophenyl group, a benzothiazole-4,7-diyl group substituted with a 4-methanesulfonyl-phenyl group, a benzothiazole-4,7-diyl group substituted with a thiophene-2-yl group, a benzothiazole-4,7-diyl group substituted with a thiophene-3-yl group, a benzothiazole-4,7-diyl group substituted with a 5-methylthiophene-2-yl group, a benzothiazole-4,7-diyl group substituted with a 5-chlorothiophene-2-yl group, a benzothiazole-4,7-diyl group substituted with a thieno[3,2-b]thiophene-2-yl group, a benzothiazole-4,7-diyl group substituted with a 2-benzothiazolyl group, a benzothiazole-4,7-diyl group substituted with a 4-biphenyl group, a benzothiazole-4,7-diyl group substituted with a 4-propylbiphenyl group, a benzothiazole-4,7-diyl group substituted with a 4-thiazolyl group, a benzothiazole-4,7-diyl group substituted with a 1-phenylethylene-2-yl group, a benzothiazole-4,7-diyl group substituted with a 4-pyridyl group, a benzothiazole-4,7-diyl group substituted with a 2-furyl group, a benzothiazole-4,7-diyl group substituted with a naphtho[1,2-b]furan-2-yl group, a 1H-isoindole-1,3(2H)-dione-4,7-diyl group substituted with a 5-methoxy-2-benzothiazolyl group, a 1H-isoindole-1,3(2H)-dione-4,7-diyl group substituted with a phenyl group, a 1H-isoindole-1,3(2H)-dione-4,7-diyl group substituted with a 4-nitrophenyl group, a 1H-isoindole-1,3(2H)-dione-4,7-diyl group substituted with a 2-thiazolyl group, or the like. $R^f$, $R^g$, $R^{g1}$, and $R^h$ have the same meaning here as previously described.

$Ar^0$ and $Ar^1$ are preferably each, independently of one another, a group indicated by any one of the following formulae (II-1) to (II-7).

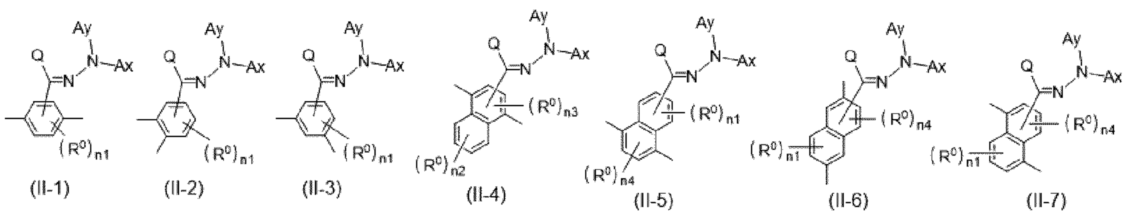

(II-1) (II-2) (II-3) (II-4) (II-5) (II-6) (II-7)

In formulae (II-1) to (II-7): Ax represents an organic group including at least one aromatic ring selected from the group consisting of an aromatic hydrocarbon ring having a carbon number of 6 to 30 and an aromatic heterocyclic ring having a carbon number of 2 to 30, where the aromatic ring of Ax is optionally substituted; Ay represents a hydrogen atom or an optionally substituted organic group having a carbon number of 1 to 30; and Q represents a hydrogen atom or an alkyl group having a carbon number of 1 to 6. Examples of the alkyl group having a carbon number of 1 to 6 of Q include a methyl group, an ethyl group, an n-propyl group, and an isopropyl.

$R^0$ represents a halogen atom; a cyano group; an alkyl group having a carbon number of 1 to 6 such as a methyl group, an ethyl group, a propyl group, an isopropyl group, a butyl group, a sec-butyl group, or a tert-butyl group; an alkenyl group having a carbon number of 2 to 6; a haloalkyl group having a carbon number of 1 to 6; an N,N-dialkylamino group having a carbon number of 2 to 12; an alkoxy group having a carbon number of 1 to 6; a nitro group; $-C(=O)-R^a$; $-O-C(=O)-R^a$; $-C(=O)-O-R^a$; or $-SO_2R^a$, where $R^a$ represents an alkyl group having a carbon number of 1 to 6 such as a methyl group or an ethyl group; or an aromatic hydrocarbon cyclic group having a carbon number of 6 to 20 that is optionally substituted with an alkyl group having a carbon number of 1 to 6 or an alkoxy group having a carbon number of 1 to 6 (for example, a phenyl group, a 4-methylphenyl group, or a 4-methoxyphenyl group). In a case in which a plurality of substituents is present, these substituents may be the same or different. From a viewpoint of improving solubility, a halogen atom, a cyano group, an alkyl group having a carbon number of 1 to 6, a haloalkyl group having a carbon number of 1 to 6, an alkoxy group having a carbon number of 1 to 6, or a nitro group is preferable as $R^0$.

Moreover, n1 is an integer of 0 to 3, n2 is an integer of 0 to 4, n3 is 0 or 1, and n4 is an integer of 0 to 2. It is preferable that n1 = 0, n2 = 0, n3 = 0, and n4 = 0.

$Ar^0$ and $Ar^1$ more preferably have, independently of one another, a structure represented by any one of the following formulae (ii-1) to (ii-21). Note that in the following formulae, $Z^1$ and $Z^2$ are also included for convenience in order to clarify the form of bonding. $Z^1$, $Z^2$, Ax, Ay, Q, $R^0$, n1, n2, n3, and n4 in the formulae have the same meaning as previously described. Of the following formulae, formulae (ii-1), (ii-2), (ii-10), and (ii-12) are particularly preferable.

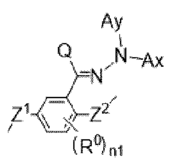
(ii-1)
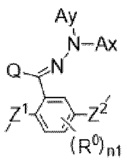
(ii-2)
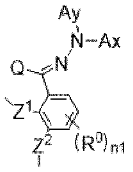
(ii-3)
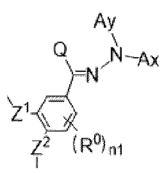
(ii-4)
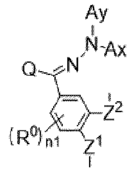
(ii-5)
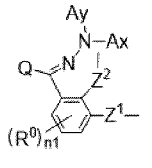
(ii-6)
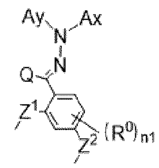
(ii-7)

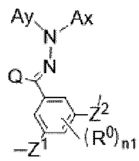
(ii-8)
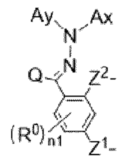
(ii-9)
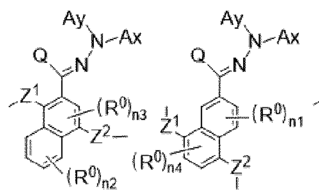
(ii-10) (ii-11)
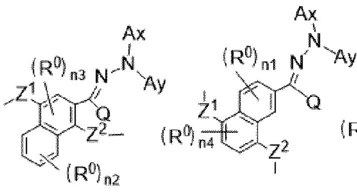
(ii-12) (ii-13)
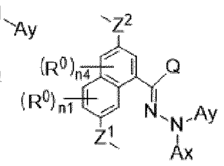
(ii-14)

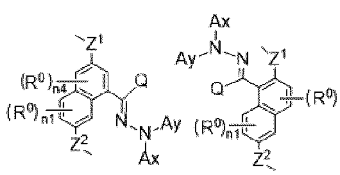
(ii-15) (ii-16)
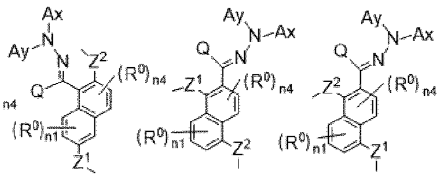
(ii-17) (ii-18) (ii-19)
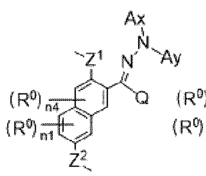
(ii-20)
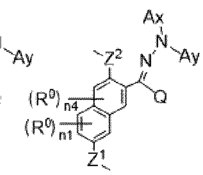
(ii-21)

The organic group of Ax that includes at least one aromatic ring selected from the group consisting of an aromatic hydrocarbon ring having a carbon number of 6 to 30 and an aromatic heterocyclic ring having a carbon number of 2 to 30 may include a plurality of aromatic rings and may include both an aromatic hydrocarbon ring and an aromatic heterocyclic ring. In a case in which the organic group includes a plurality of aromatic hydrocarbon rings or aromatic heterocyclic rings, these rings may be the same or different.

Examples of aromatic hydrocarbon rings that may be included in Ax include a benzene ring, a naphthalene ring, an anthracene ring, a phenanthrene ring, a pyrene ring, and a fluorene ring.

Of these aromatic hydrocarbon rings, a benzene ring, a naphthalene ring, or an anthracene ring is preferable.

Examples of aromatic heterocyclic rings that may be included in Ax include a 1H-isoindole-1,3(2H)-dione ring, a 1-benzofuran ring, a 2-benzofuran ring, an acridine ring, an isoquinoline ring, an imidazole ring, an indole ring, an oxadiazole ring, an oxazole ring, an oxazolopyrazine ring, an oxazolopyridine ring, an oxazolopyridazyl ring, an oxazolopyrimidine ring, a quinazoline ring, a quinoxaline ring, a quinoline ring, a cinnoline ring, a thiadiazole ring, a thiazole ring, a thiazolopyrazine ring, a thiazolopyridine ring, a thiazolopyridazine ring, a thiazolopyrimidine ring, a thiophene ring, a triazine ring, a triazole ring, a naphthyridine ring, a pyrazine ring, a pyrazole ring, a pyranone ring, a pyran ring, a pyridine ring, a pyridazine ring, a pyrimidine ring, a pyrrole ring, a phenanthridine ring, a phthalazine ring, a furan ring, a benzo[c]thiophene ring, a benzisoxazole ring, a benzisothiazole ring, a benzimidazole ring, a benzoxadiazole ring, a benzoxazole ring, a benzothiadiazole ring, a benzothiazole ring, a benzothiophene ring, a benzotriazine ring, a benzotriazole ring, a benzopyrazole ring, a benzopyranone ring, a dihydropyran ring, a tetrahydropyran ring, a dihydrofuran ring, and a tetrahydrofuran ring.

Of these aromatic heterocyclic rings, a monocyclic aromatic heterocyclic ring such as a furan ring, a pyran ring, a thiophene ring, an oxazole ring, an oxadiazole ring, a thiazole ring, or a thiadiazole ring, or a fused ring aromatic heterocyclic ring such as a benzothiazole ring, a benzoxazole ring, a quinoline ring, a 1-benzofuran ring, a 2-benzofuran ring, a benzothiophene ring, a 1H-isoindole-1,3(2H)-dione ring, a benzo[c]thiophene ring, a thiazolopyridine ring, a thiazolopyrazine ring, a benzisoxazole ring, a benzoxadiazole ring, or a benzothiadiazole ring is preferable.

The aromatic ring included in Ax is optionally substituted. Examples of possible substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon number of 1 to 6 such as a trifluoromethyl group; N,N-dialkylamino groups having a carbon number of 2 to 12 such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; $-OCF_3$; $-C(=O)-R^b$; $-O-C(=O)-R^b$; $-C(=O)-O-R^b$; and $-SO_2R^a$. $R^b$ represents an optionally substituted alkyl group having a carbon number of 1 to 20, an optionally substituted alkenyl group having a carbon number of 2 to 20, an optionally substituted cycloalkyl

group having a carbon number of 3 to 12, or an optionally substituted aromatic hydrocarbon cyclic group having a carbon number of 5 to 18. $R^a$ has the same meaning as previously described. Of these examples, halogen atoms, a cyano group, alkyl groups having a carbon number of 1 to 6, and alkoxy groups having a carbon number of 1 to 6 are preferable as substituents of the aromatic ring included in Ax.

Note that Ax may have a plurality of substituents selected from the substituents listed above. In a case in which Ax has a plurality of substituents, these substituents may be the same or different.

Examples of the alkyl group having a carbon number of 1 to 20 in the optionally substituted alkyl group having a carbon number of 1 to 20 of $R^b$ include a methyl group, an ethyl group, an n-propyl group, an isopropyl group, an n-butyl group, an isobutyl group, a 1-methylpentyl group, a 1-ethylpentyl group, a sec-butyl group, a t-butyl group, an n-pentyl group, an isopentyl group, a neopentyl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, and an n-icosyl group. The carbon number of the optionally substituted alkyl group having a carbon number of 1 to 20 is preferably 1 to 12, and more preferably 4 to 10.

Examples of the alkenyl group having a carbon number of 2 to 20 in the optionally substituted alkenyl group having a carbon number of 2 to 20 of $R^b$ include a vinyl group, a propenyl group, an isopropenyl group, a butenyl group, an isobutenyl group, a pentenyl group, a hexenyl group, a heptenyl group, an octenyl group, a nonenyl group, a decenyl group, an undecenyl group, a dodecenyl group, a tridecenyl group, a tetradecenyl group, a pentadecenyl group, a hexadecenyl group, a heptadecenyl group, an octadecenyl group, a nonadecenyl group, and an icosenyl group.

The carbon number of the optionally substituted alkenyl group having a carbon number of 2 to 20 is preferably 2 to 12.

Examples of possible substituents of the alkyl group having a carbon number of 1 to 20 or alkenyl group having a carbon number of 2 to 20 of $R^b$ include halogen atoms such as a fluorine atom and a chlorine atom; a cyano group; N,N-dialkylamino groups having a carbon number of 2 to 12 such as a dimethylamino group; alkoxy groups having a carbon number of 1 to 20 such as a methoxy group, an ethoxy group, an isopropoxy group, and a butoxy group; alkoxy groups having a carbon number of 1 to 12 that are substituted with an alkoxy group having a carbon number of 1 to 12 such as a methoxymethoxy group and a methoxyethoxy group; a nitro group; aromatic hydrocarbon cyclic groups having a carbon number of 6 to 20 such as a phenyl group and a naphthyl group; aromatic heterocyclic groups having a carbon number of 2 to 20 such as a triazolyl group, a pyrrolyl group, a furanyl group, a thiophenyl group, and a benzothiazole-2-ylthio group; cycloalkyl groups having a carbon number of 3 to 8 such as a cyclopropyl group, a cyclopentyl group, and a cyclohexyl group; cycloalkyloxy groups having a carbon number of 3 to 8 such as a cyclopentyloxy group and a cyclohexyloxy group; cyclic ether groups having a carbon number of 2 to 12 such as a tetrahydrofuranyl group, a tetrahydropyranyl group, a dioxolanyl group, and a dioxanyl group; aryloxy groups having a carbon number of 6 to 14 such as a phenoxy group and a naphthoxy group; fluoroalkyl groups having a carbon number of 1 to 12 in which one or more hydrogen atoms are replaced by fluorine atoms such as a trifluoromethyl group, a pentafluoroethyl group, and $-CH_2CF_3$; a benzofuryl group; a benzopyranyl group; a benzodioxolyl group; and a benzodioxanyl group. Of these examples, halogen atoms such as a fluorine atom and a chlorine atom; a cyano group; alkoxy groups having a carbon number of 1 to 20 such as a methoxy group, an ethoxy group, an isopropoxy group, and a butoxy group; a nitro group; aromatic hydrocarbon cyclic groups having a carbon number of 6 to 20 such as a phenyl group and a naphthyl group; aromatic heterocyclic groups having a carbon number of 2 to 20 such as a furanyl group and a thiophenyl group; cycloalkyl groups having a carbon number of 3 to 8 such as a cyclopropyl group, a cyclopentyl group, and a cyclohexyl group; and fluoroalkyl groups having a carbon number of 1 to 12 in which one or more hydrogen atoms are replaced by fluorine atoms such as a trifluoromethyl group, a pentafluoroethyl group, and $-CH_2CF_3$ are preferable as substituents of the alkyl group having a carbon number of 1 to 20 or alkenyl group having a carbon number of 2 to 20 of $R^b$.

The alkyl group having a carbon number of 1 to 20 or alkenyl group having a carbon number of 2 to 20 of $R^b$ may have a plurality of substituents selected from the substituents listed above. In a case in which the alkyl group having a carbon number of 1 to 20 or alkenyl group having a carbon number of 2 to 20 of $R^b$ has a plurality of substituents, these substituents may be the same or different.

Examples of the cycloalkyl group having a carbon number of 3 to 12 in the optionally substituted cycloalkyl group having a carbon number of 3 to 12 of $R^b$ include a cyclopropyl group, a cyclobutyl group, a cyclopentyl group, a cyclohexyl group, and a cyclooctyl group. Of these examples, a cyclopentyl group or a cyclohexyl group is preferable.

Examples of possible substituents of the cycloalkyl group having a carbon number of 3 to 12 of $R^b$ include halogen atoms such as a fluorine atom and a chlorine atom; a cyano group; N,N-dialkylamino groups having a carbon number of 2 to 12 such as a dimethylamino group; alkyl groups having a carbon number of 1 to 6 such as a methyl group, an ethyl group, and a propyl group; alkoxy groups having a carbon number of 1 to 6 such as a methoxy group, an ethoxy group, and an isopropoxy group; a nitro group; and aromatic hydrocarbon cyclic groups having a carbon number of 6 to 20 such as a phenyl group and a naphthyl group. Of these examples, halogen atoms such as a fluorine atom and a chlorine atom; a cyano group; alkyl groups having a carbon number of 1 to 6 such as a methyl group, an

ethyl group, and a propyl group; alkoxy groups having a carbon number of 1 to 6 such as a methoxy group, an ethoxy group, and an isopropoxy group; a nitro group; and aromatic hydrocarbon cyclic groups having a carbon number of 6 to 20 such as a phenyl group and a naphthyl group are preferable as substituents of the cycloalkyl group having a carbon number of 3 to 12 of $R^b$.

The cycloalkyl group having a carbon number of 3 to 12 of $R^b$ may have a plurality of substituents. In a case in which the cycloalkyl group having a carbon number of 3 to 12 of $R^b$ has a plurality of substituents, these substituents may be the same or different.

Examples of the aromatic hydrocarbon cyclic group having a carbon number of 5 to 18 in the optionally substituted aromatic hydrocarbon cyclic group having a carbon number of 5 to 18 of $R^b$ include a phenyl group, a 1-naphthyl group, and a 2-naphthyl group. Of these examples, a phenyl group is preferable.

Examples of possible substituents of the optionally substituted aromatic hydrocarbon cyclic group having a carbon number of 5 to 18 include halogen atoms such as a fluorine atom and a chlorine atom; a cyano group; N,N-dialkylamino groups having a carbon number of 2 to 12 such as a dimethylamino group; alkoxy groups having a carbon number of 1 to 20 such as a methoxy group, an ethoxy group, an isopropoxy group, and a butoxy group; alkoxy groups having a carbon number of 1 to 12 that are substituted with an alkoxy group having a carbon number of 1 to 12 such as a methoxymethoxy group and a methoxyethoxy group; a nitro group; aromatic hydrocarbon cyclic groups having a carbon number of 6 to 20 such as a phenyl group and a naphthyl group; aromatic heterocyclic groups having a carbon number of 2 to 20 such as a triazolyl group, a pyrrolyl group, a furanyl group, and a thiophenyl group; cycloalkyl groups having a carbon number of 3 to 8 such as a cyclopropyl group, a cyclopentyl group, and a cyclohexyl group; cycloalkyloxy groups having a carbon number of 3 to 8 such as a cyclopentyloxy group and a cyclohexyloxy group; cyclic ether groups having a carbon number of 2 to 12 such as a tetrahydrofuranyl group, a tetrahydropyranyl group, a dioxolanyl group, and a dioxanyl group; aryloxy groups having a carbon number of 6 to 14 such as a phenoxy group and a naphthoxy group; fluoroalkyl groups having a carbon number of 1 to 12 in which one or more hydrogen atoms are replaced by fluorine atoms such as a trifluoromethyl group, a pentafluoroethyl group, and $-CH_2CF_3$; $-OCF_3$; a benzofuryl group; a benzopyranyl group; a benzodioxolyl group; and a benzodioxanyl group. Of these examples, one or more substituents selected from halogen atoms such as a fluorine atom and a chlorine atom; a cyano group; alkoxy groups having a carbon number of 1 to 20 such as a methoxy group, an ethoxy group, an isopropoxy group, and a butoxy group; a nitro group; aromatic hydrocarbon cyclic groups having a carbon number of 6 to 20 such as a phenyl group and a naphthyl group; aromatic heterocyclic groups having a carbon number of 2 to 20 such as a furanyl group and a thiophenyl group; cycloalkyl groups having a carbon number of 3 to 8 such as a cyclopropyl group, a cyclopentyl group, and a cyclohexyl group; fluoroalkyl groups having a carbon number of 1 to 12 in which one or more hydrogen atoms are replaced by fluorine atoms such as a trifluoromethyl group, a pentafluoroethyl group, and $-CH_2CF_3$; and $-OCF_3$ are preferable as substituents of the aromatic hydrocarbon cyclic group having a carbon number of 5 to 18.

The aromatic hydrocarbon cyclic group having a carbon number of 5 to 18 may have a plurality of substituents. In a case in which the aromatic hydrocarbon cyclic group having a carbon number of 5 to 18 has a plurality of substituents, these substituents may be the same or different.

The aromatic ring included in Ax may have a plurality of substituents that are the same or different, and two substituents that are adjacent to one another may be bonded to form a ring. The ring that is formed may be a monocycle or a fused polycycle, and may be an unsaturated ring or a saturated ring.

Note that the "carbon number" of the organic group of Ax that includes at least one aromatic ring selected from the group consisting of an aromatic hydrocarbon ring having a carbon number of 6 to 30 and an aromatic heterocyclic ring having a carbon number of 2 to 30 is the carbon number of the aromatic hydrocarbon ring and/or aromatic heterocyclic ring itself and does not include carbon atoms of substituents.

Examples of the organic group of Ax that includes at least one aromatic ring selected from the group consisting of an aromatic hydrocarbon ring having a carbon number of 6 to 30 and an aromatic heterocyclic ring having a carbon number of 2 to 30 include the following groups 1) to 5).

1) A hydrocarbon cyclic group having a carbon number of 6 to 40 and including at least one aromatic hydrocarbon ring having a carbon number of 6 to 30
2) A heterocyclic group having a carbon number of 2 to 40 and including at least one aromatic ring selected from the group consisting of an aromatic hydrocarbon ring having a carbon number of 6 to 30 and an aromatic heterocyclic ring having a carbon number of 2 to 30
3) An alkyl group having a carbon number of 1 to 12 that is substituted with at least one of an aromatic hydrocarbon cyclic group having a carbon number of 6 to 30 and an aromatic heterocyclic group having a carbon number of 2 to 30
4) An alkenyl group having a carbon number of 2 to 12 that is substituted with at least one of an aromatic hydrocarbon cyclic group having a carbon number of 6 to 30 and an aromatic heterocyclic group having a carbon number of 2 to 30
5) An alkynyl group having a carbon number of 2 to 12 that is substituted with at least one of an aromatic hydrocarbon cyclic group having a carbon number of 6 to 30 and an aromatic heterocyclic group having a carbon number of 2 to 30

Specific examples of the aromatic hydrocarbon ring in the "hydrocarbon cyclic group having a carbon number of 6 to 40 and including at least one aromatic hydrocarbon ring having a carbon number of 6 to 30" mentioned above in 1) include the same as listed as specific examples for aromatic hydrocarbon rings that may be included in Ax. Examples of the hydrocarbon cyclic group mentioned above in 1) include an aromatic hydrocarbon cyclic group having a carbon number of 6 to 30 (for example, a phenyl group, a naphthyl group, an anthracenyl group, a phenanthrenyl group, a pyrenyl group, or a fluorenyl group), an indanyl group, a 1,2,3,4-tetrahydronaphthyl group, and a 1,4-dihydronaphthyl group.

Specific examples of the aromatic hydrocarbon ring and the aromatic heterocyclic ring in the "heterocyclic group having a carbon number of 2 to 40 and including at least one aromatic ring selected from the group consisting of an aromatic hydrocarbon ring having a carbon number of 6 to 30 and an aromatic heterocyclic ring having a carbon number of 2 to 30" mentioned above in 2) include the same as listed as specific examples for aromatic hydrocarbon rings and aromatic heterocyclic rings that may be included in Ax. Examples of the heterocyclic group mentioned above in 2) include an aromatic heterocyclic group having a carbon number of 2 to 30 (for example, a phthalimide group, a 1-benzofuranyl group, a 2-benzofuranyl group, an acridinyl group, an isoquinolinyl group, an imidazolyl group, an indolinyl group, a furazanyl group, an oxazolyl group, an oxazolopyrazinyl group, an oxazolopyridinyl group, an oxazolopyridazinyl group, an oxazolopyrimidinyl group, a quinazolinyl group, a quinoxalinyl group, a quinolyl group, a cinnolinyl group, a thiadiazolyl group, a thiazolyl group, a thiazolopyrazinyl group, a thiazolopyridinyl group, a thiazolopyridazinyl group, a thiazolopyrimidinyl group, a thienyl group, a triazinyl group, a triazolyl group, a naphthyridinyl group, a pyrazinyl group, a pyrazolyl group, a pyranonyl group, a pyranyl group, a pyridyl group, a pyridazinyl group, a pyrimidinyl group, a pyrrolyl group, a phenanthridinyl group, a phthalazinyl group, a furanyl group, a benzo[c]thienyl group, a benzisoxazolyl group, a benzisothiazolyl group, a benzimidazolyl group, a benzoxazolyl group, a benzothiadiazolyl group, a benzothiazolyl group, a benzothiophenyl group, a benzotriazinyl group, a benzotriazolyl group, a benzopyrazolyl group, a benzopyranonyl group, a dihydropyranyl group, a tetrahydropyranyl group, a dihydrofuranyl group, or a tetrahydrofuranyl group), a 2,3-dihydroindolyl group, a 9,10-dihydroacridinyl group, and a 1,2,3,4-tetrahydroquinolyl group.

Specific examples of the alkyl group having a carbon number of 1 to 12 in the "alkyl group having a carbon number of 1 to 12 that is substituted with at least one of an aromatic hydrocarbon cyclic group having a carbon number of 6 to 30 and an aromatic heterocyclic group having a carbon number of 2 to 30" mentioned above in 3) include a methyl group, an ethyl group, a propyl group, and an isopropyl group. Specific examples of the aromatic hydrocarbon cyclic group having a carbon number of 6 to 30 and the aromatic heterocyclic group having a carbon number of 2 to 30 mentioned above in 3) include the same as listed as specific examples for the aromatic hydrocarbon cyclic group having a carbon number of 6 to 30 and the aromatic heterocyclic group having a carbon number of 2 to 30 mentioned above in 1) and 2).

Specific examples of the alkenyl group having a carbon number of 2 to 12 in the "alkenyl group having a carbon number of 2 to 12 that is substituted with at least one of an aromatic hydrocarbon cyclic group having a carbon number of 6 to 30 and an aromatic heterocyclic group having a carbon number of 2 to 30" mentioned above in 4) include a vinyl group and an allyl group. Specific examples of the aromatic hydrocarbon cyclic group having a carbon number of 6 to 30 and the aromatic heterocyclic group having a carbon number of 2 to 30 mentioned above in 4) include the same as listed as specific examples for the aromatic hydrocarbon cyclic group having a carbon number of 6 to 30 and the aromatic heterocyclic group having a carbon number of 2 to 30 mentioned above in 1) and 2).

Specific examples of the alkynyl group having a carbon number of 2 to 12 in the "alkynyl group having a carbon number of 2 to 12 that is substituted with at least one of an aromatic hydrocarbon cyclic group having a carbon number of 6 to 30 and an aromatic heterocyclic group having a carbon number of 2 to 30" mentioned above in 5) include an ethynyl group and a propynyl group. Specific examples of the aromatic hydrocarbon cyclic group having a carbon number of 6 to 30 and the aromatic heterocyclic group having a carbon number of 2 to 30 mentioned above in 5) include the same as listed as specific examples for the aromatic hydrocarbon cyclic group having a carbon number of 6 to 30 and the aromatic heterocyclic group having a carbon number of 2 to 30 mentioned above in 1) and 2).

The organic groups listed above in 1) to 5) may have one or a plurality of substituents. In a case in which the organic group has a plurality of substituents, these substituents may be the same or different.

Examples of these substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon number of 1 to 6 such as a trifluoromethyl group; N,N-dialkylamino groups having a carbon number of 2 to 12 such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; $-OCF_3$; $-C(=O)-R^b$; $-C(=O)-O-R^b$; and $-SO_2R^a$. $R^b$ and $R^a$ have the same meaning here as previously described and preferable examples thereof are also the same as previously described.

Of these examples, one or more substituents selected from halogen atoms, a cyano group, alkyl groups having a carbon number of 1 to 6, and alkoxy groups having a carbon number of 1 to 6 are preferable as substituents included

in the organic groups listed above in 1) to 5).

Specific examples that are preferable as the organic group of Ax that includes at least one aromatic ring selected from the group consisting of an aromatic hydrocarbon ring having a carbon number of 6 to 30 and an aromatic heterocyclic ring having a carbon number of 2 to 30 are shown below. However, the following examples are not intended to be limiting. Note that "-" in the following formulae indicates atomic bonding with a N atom that extends from any position in a ring (i.e., a N atom that is bonded to Ax in formula (I)).

1) Specific examples of hydrocarbon cyclic groups having a carbon number of 6 to 40 and including at least one aromatic hydrocarbon ring having a carbon number of 6 to 30 include structures represented by the following formulae (1-1) to (1-21). Aromatic hydrocarbon cyclic groups having a carbon number of 6 to 30 that are represented by formulae (1-9) to (1-21) and the like are preferable.

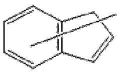
(1-1)
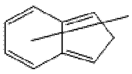
(1-2)
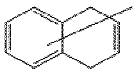
(1-3)
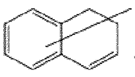
(1-4)
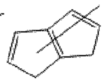
(1-5)
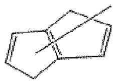
(1-6)
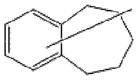
(1-7)
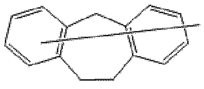
(1-8)

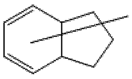
(1-9)
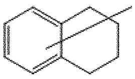
(1-10)
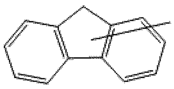
(1-11)
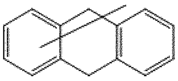
(1-12)
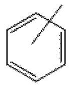
(1-13)
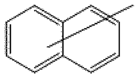
(1-14)
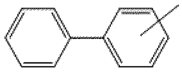
(1-15)

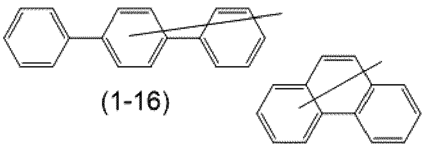
(1-16)
(1-17)
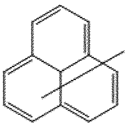
(1-18)
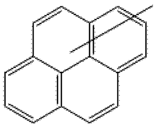
(1-19)
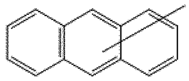
(1-20)
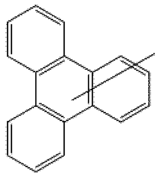
(1-21)

2) Specific examples of heterocyclic groups having a carbon number of 2 to 40 and including at least one aromatic ring selected from the group consisting of an aromatic hydrocarbon ring having a carbon number of 6 to 30 and an aromatic heterocyclic ring having a carbon number of 2 to 30 include structures represented by the following formulae (2-1) to (2-51). Aromatic heterocyclic groups having a carbon number of 2 to 30 that are represented by formulae (2-12) to (2-51) and the like are preferable.

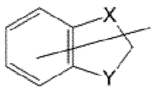
(2-1)
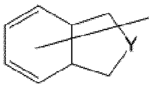
(2-2)
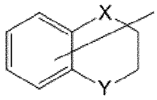
(2-3)
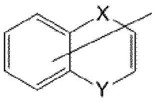
(2-4)
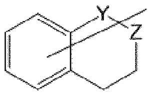
(2-5)
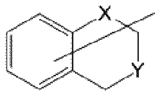
(2-6)

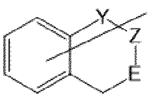
(2-7)
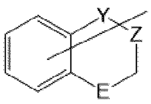
(2-8)
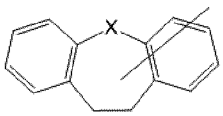
(2-9)
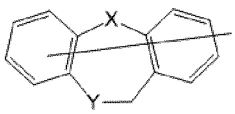
(2-10)
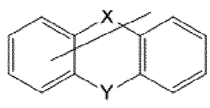
(2-11)

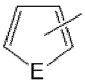
(2-12)
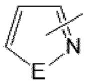
(2-13)
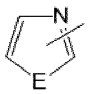
(2-14)
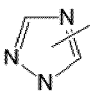
(2-15)
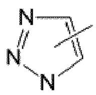
(2-16)
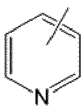
(2-17)
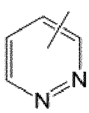
(2-18)
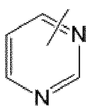
(2-19)

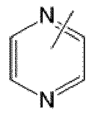
(2-20)
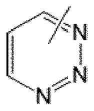
(2-21)
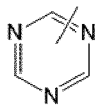
(2-22)
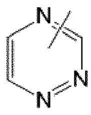
(2-23)
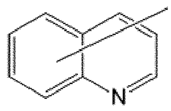
(2-24)
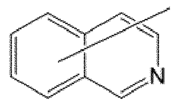
(2-25)
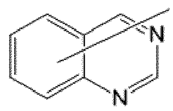
(2-26)

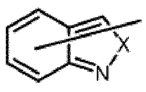
(2-27)
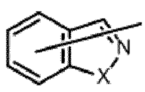
(2-28)
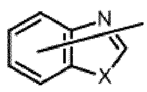
(2-29)
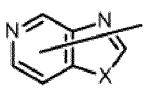
(2-30)
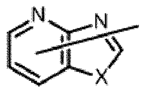
(2-31)
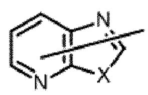
(2-32)
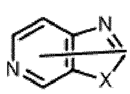
(2-33)

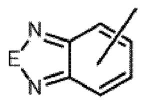
(2-34)
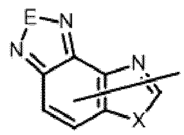
(2-35)
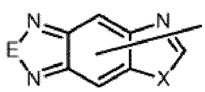
(2-36)
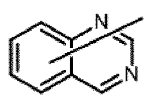
(2-37)
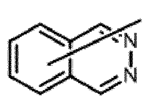
(2-38)
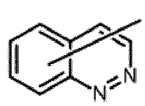
(2-39)

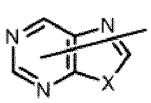
(2-40)
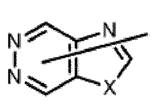
(2-41)
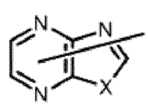
(2-42)
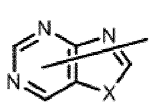
(2-43)
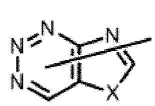
(2-44)
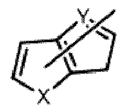
(2-45)
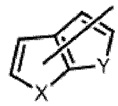
(2-46)

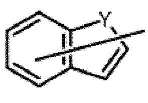
(2-47)
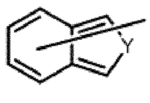
(2-48)
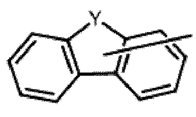
(2-49)
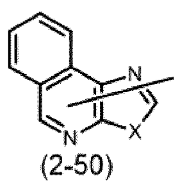
(2-50)
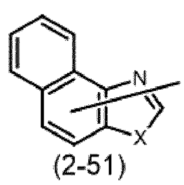
(2-51)

[In each formula: X represents $-CH_2-$, $-NR^c-$, an oxygen atom, a sulfur atom, -SO-, or $-SO_2-$;
Y and Z each represent, independently of one another, $-NR^c-$, an oxygen atom, a sulfur atom, -SO-, or $-SO_2-$; and E represents $-NR^c-$, an oxygen atom, or a sulfur atom.

Moreover, $R^c$ represents a hydrogen atom or an alkyl group having a carbon number of 1 to 6 such as a methyl group, an ethyl group, or a propyl group. (However, in each formula, oxygen atoms, sulfur atoms, -SO-, and $-SO_2-$ are not located adjacently to one another.)]

3) Specific examples of alkyl groups having a carbon number of 1 to 12 that are substituted with at least one of an aromatic hydrocarbon cyclic group having a carbon number of 6 to 30 and an aromatic heterocyclic group having a carbon number of 2 to 30 include structures represented by the following formulae (3-1) to (3-8).

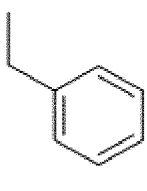
(3-1)
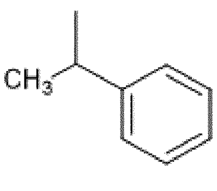
(3-2)
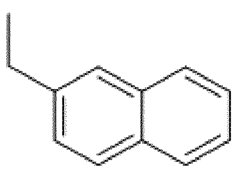
(3-3)
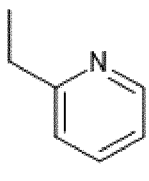
(3-4)
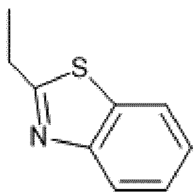
(3-5)

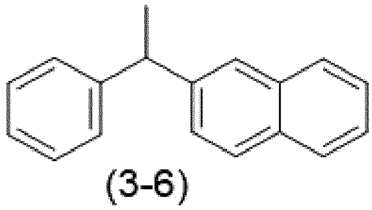
(3-6)

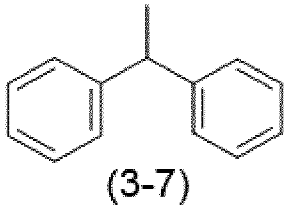
(3-7)

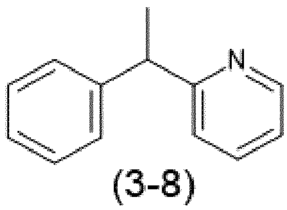
(3-8)

4) Specific examples of alkenyl groups having a carbon number of 2 to 12 that are substituted with at least one of an aromatic hydrocarbon cyclic group having a carbon number of 6 to 30 and an aromatic heterocyclic group having a carbon number of 2 to 30 include structures represented by the following formulae (4-1) to (4-5).

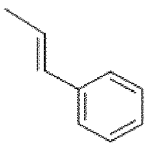
(4-1)

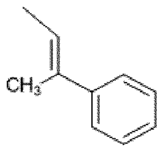
(4-2)

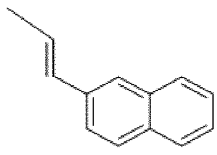
(4-3)

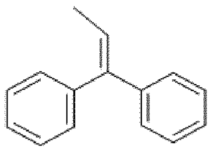
(4-4)

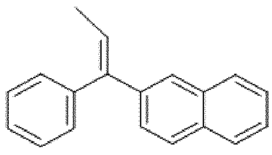
(4-5)

5) Specific examples of alkynyl groups having a carbon number of 1 to 12 that are substituted with at least one selected from the group consisting of an aromatic hydrocarbon ring and an aromatic heterocyclic ring include structures represented by the following formulae (5-1) and (5-2).

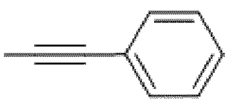
(5-1)

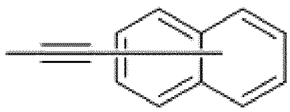
(5-2)

Note that the rings in these preferable specific examples of Ax may have one or a plurality of substituents. In a case in which a ring has a plurality of substituents, these substituents may be the same or different. Examples of these substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon number of 1 to 6 such as a trifluoromethyl group; N,N-dialkylamino groups having a carbon number of 2 to 12 such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; $-OCF_3$; $-C(=O)-R^b$; $-O-C(=O)-R^b$; $-C(=O)-O-R^b$; and $-SO_2R^a$.

$R^b$ and $R^a$ have the same meaning here as previously described and preferable examples thereof are also the same as previously described. Of these examples, halogen atoms, a cyano group, alkyl groups having a carbon number of 1 to 6, and alkoxy groups having a carbon number of 1 to 6 are preferable as substituents of a ring included in Ax.

From among the examples described above, Ax is preferably an aromatic hydrocarbon cyclic group having a carbon number of 6 to 30, an aromatic heterocyclic group having a carbon number of 2 to 30, or a group indicated by the previously shown formula (1-9).

Ax is more preferably an aromatic hydrocarbon cyclic group having a carbon number of 6 to 20 or an aromatic heterocyclic group having a carbon number of 4 to 20, and is even more preferably a group indicated by any one of the previously shown formulae (1-14), (1-20), (2-27) to (2-33), (2-35) to (2-43), (2-50), and (2-51).

Note that the rings may have one or a plurality of substituents as previously described. In a case in which a ring has a plurality of substituents, these substituents may be the same or different. Examples of these substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon number of 1 to 6 such as a trifluoromethyl group and a pentafluoroethyl group; N,N-dialkylamino groups having a carbon number of 2 to 12 such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group

and a naphthyl group; $-C(=O)-R^b$; $-O-C(=O)-R^b$; $-C(=O)-O-R^b$; and $-SO_2R^a$.

$R^b$ and $R^a$ have the same meaning here as previously described and preferable examples thereof are also the same as previously described.

Of these examples, halogen atoms, a cyano group, alkyl groups having a carbon number of 1 to 6, and alkoxy groups having a carbon number of 1 to 6 are preferable as substituents of the rings.

A group represented by the following formula (V) is even more preferable as Ax.

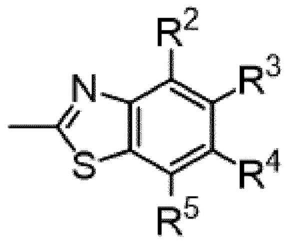

(V)

In formula (V), $R^2$ to $R^5$ each represent, independently of one another, a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, $-OCF_3$, $-O-C(=O)-R^b$, or $-C(=O)-O-R^b$, where $R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18. Of these examples, a case in which $R^2$ to $R^5$ are all hydrogen atoms and a case in which at least one of $R^2$ to $R^5$ is an optionally substituted alkoxy group having a carbon number of 1 to 6 and the rest of $R^2$ to $R^5$ are hydrogen atoms are preferable.

Moreover, $C-R^2$ to $C-R^5$ may be the same or different, and one or more of ring constituents $C-R^2$ to $C-R^5$ may be replaced by a nitrogen atom.

The following shows specific examples of groups resulting from one or more of $C-R^2$ to $C-R^5$ in the group represented by formula (V) being replaced by a nitrogen atom. However, examples of groups resulting from one or more of $C-R^2$ to $C-R^5$ being replaced by a nitrogen atom are not limited to these examples.

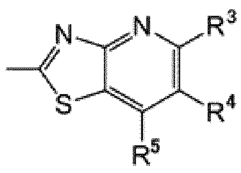
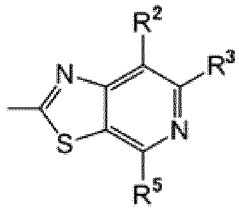
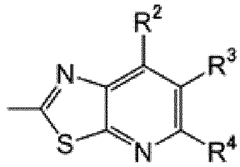
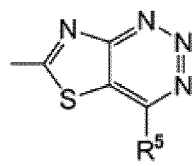
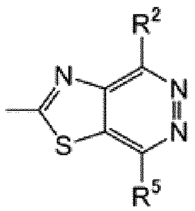
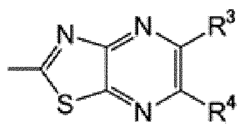
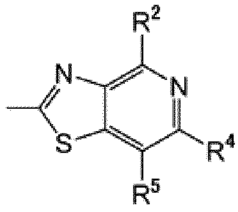
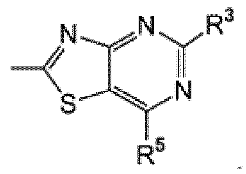

[In each formula, $R^2$ to $R^5$ have the same meaning as previously described and preferable examples thereof are also the same as previously described.]

Examples of the optionally substituted organic group having a carbon number of 1 to 30 of Ay in the groups represented by formulae (II-1) to (II-7) include, but are not specifically limited to, an optionally substituted alkyl group having a carbon number of 1 to 20, an optionally substituted alkenyl group having a carbon number of 2 to 20, an optionally substituted alkynyl group having a carbon number of 2 to 20, an optionally substituted cycloalkyl group having a carbon number of 3 to 12, $-SO_2R^a$, $-O-C(=O)-R^b$, $-C(=O)-O-R^b$, $-C(=O)-R^b$, $-CS-NH-R^b$, $-NH-C(=O)-O-R^b$, $-O-C(=O)-NH-R^b$, an optionally substituted aromatic hydrocarbon cyclic group having a carbon number of 6 to 30, and an optionally substituted aromatic heterocyclic group having a carbon number of 2 to 30.

$R^a$ and $R^b$ have the same meaning here as previously described and preferable examples thereof are also the same as previously described.

Examples of the alkyl group having a carbon number of 1 to 20 in the optionally substituted alkyl group having a carbon number of 1 to 20 of Ay, the alkenyl group having a carbon number of 2 to 20 in the optionally substituted

alkenyl group having a carbon number of 2 to 20 of Ay, and the cycloalkyl group having a carbon number of 3 to 12 in the optionally substituted cycloalkyl group having a carbon number of 3 to 12 of Ay include the same as listed as specific examples for the alkyl group having a carbon number of 1 to 20 in the optionally substituted alkyl group having a carbon number of 1 to 20 of $R^b$, the alkenyl group having a carbon number of 2 to 20 in the optionally substituted alkenyl group having a carbon number of 2 to 20 of $R^b$, and the cycloalkyl group having a carbon number of 3 to 12 in the optionally substituted cycloalkyl group having a carbon number of 3 to 12 of $R^b$. Moreover, the carbon number of the optionally substituted alkyl group having a carbon number of 1 to 20 is preferably 1 to 10, the carbon number of the optionally substituted alkenyl group having a carbon number of 2 to 20 is preferably 2 to 10, and the carbon number of the optionally substituted cycloalkyl group having a carbon number of 3 to 12 is preferably 3 to 10.

Examples of the alkynyl group having a carbon number of 2 to 20 in the optionally substituted alkynyl group having a carbon number of 2 to 20 of Ay include an ethynyl group, a propynyl group, a 2-propynyl group (propargyl group), a butynyl group, a 2-butynyl group, a 3-butynyl group, a pentynyl group, a 2-pentynyl group, a hexynyl group, a 5-hexynyl group, a heptynyl group, an octynyl group, a 2-octynyl group, a nonanyl group, a decanyl group, and a 7-decanyl group.

Examples of possible substituents of the optionally substituted alkyl group having a carbon number of 1 to 20, the optionally substituted alkenyl group having a carbon number of 2 to 20, the optionally substituted cycloalkyl group having a carbon number of 3 to 12, or the alkynyl group having a carbon number of 2 to 20 of Ay include halogen atoms such as a fluorine atom and a chlorine atom; a cyano group; N,N-dialkylamino groups having a carbon number of 2 to 12 such as a dimethylamino group; alkoxy groups having a carbon number of 1 to 20 such as a methoxy group, an ethoxy group, an isopropoxy group, and a butoxy group; alkoxy groups having a carbon number of 1 to 12 that are substituted with an alkoxy group having a carbon number of 1 to 12 such as a methoxymethoxy group and a methoxyethoxy group; a nitro group; aromatic hydrocarbon cyclic groups having a carbon number of 6 to 20 such as a phenyl group and a naphthyl group; aromatic heterocyclic groups having a carbon number of 2 to 20 such as a triazolyl group, a pyrrolyl group, a furanyl group, and a thiophenyl group; cycloalkyl groups having a carbon number of 3 to 8 such as a cyclopropyl group, a cyclopentyl group, and a cyclohexyl group; cycloalkyloxy groups having a carbon number of 3 to 8 such as a cyclopentyloxy group and a cyclohexyloxy group; cyclic ether groups having a carbon number of 2 to 12 such as a tetrahydrofuranyl group, a tetrahydropyranyl group, a dioxolanyl group, and a dioxanyl group; aryloxy groups having a carbon number of 6 to 14 such as a phenoxy group and a naphthoxy group; fluoroalkyl groups having a carbon number of 1 to 12 in which one or more hydrogen atoms are replaced by fluorine atoms such as a trifluoromethyl group, a pentafluoroethyl group, and $-CH_2CF_3$; a benzofuryl group; a benzopyranyl group; a benzodioxolyl group; a benzodioxanyl group; $-O-C(=O)-R^b$; $-C(=O)-R^b$; $-C(=O)-O-R^b$; $-SO_2R^a$; $-SR^b$; alkoxy groups having a carbon number of 1 to 12 that are substituted with $-SR^b$; and a hydroxy group. $R^a$ and $R^b$ have the same meaning here as previously described and preferable examples thereof are also the same as previously described.

The alkyl group having a carbon number of 1 to 20, the alkenyl group having a carbon number of 2 to 20, the cycloalkyl group having a carbon number of 3 to 12, or the alkynyl group having a carbon number of 2 to 20 of Ay may have a plurality of the substituents listed above, and in such a case, these substituents may be the same or different.

Examples of the aromatic hydrocarbon cyclic group having a carbon number of 6 to 30 or aromatic heterocyclic group having a carbon number of 2 to 30 of Ay and substituents thereof include the same as listed for the aromatic hydrocarbon cyclic group or aromatic heterocyclic group of Ax and substituents thereof. The aromatic hydrocarbon cyclic group having a carbon number of 6 to 30 or the aromatic heterocyclic group having a carbon number of 2 to 30 of Ay may have a plurality of substituents selected from those listed above. In a case in which the aromatic hydrocarbon cyclic group or aromatic heterocyclic group of Ay has a plurality of substituents, these substituents may be the same or different. The carbon number of the aromatic hydrocarbon cyclic group of Ay is preferably 6 to 20, more preferably 6 to 18, and even more preferably 6 to 12. Moreover, the carbon number of the aromatic heterocyclic group of Ay is preferably 2 to 20, and more preferably 2 to 18.

Of the examples listed above, Ay is preferably a hydrogen atom, an optionally substituted alkyl group having a carbon number of 1 to 20, an optionally substituted alkenyl group having a carbon number of 2 to 20, an optionally substituted alkynyl group having a carbon number of 2 to 20, an optionally substituted cycloalkyl group having a carbon number of 3 to 12, an optionally substituted aromatic hydrocarbon cyclic group having a carbon number of 6 to 18, or an optionally substituted aromatic heterocyclic group having a carbon number of 2 to 18. Moreover, Ay is more preferably a hydrogen atom, an optionally substituted alkyl group having a carbon number of 1 to 18, an optionally substituted alkenyl group having a carbon number of 2 to 18, an optionally substituted alkynyl group having a carbon number of 2 to 18, an optionally substituted cycloalkyl group having a carbon number of 3 to 10, an optionally substituted aromatic hydrocarbon cyclic group having a carbon number of 6 to 12, or an optionally substituted aromatic heterocyclic group having a carbon number of 2 to 18. Furthermore, Ay is particularly preferably an optionally substituted alkyl group having a carbon number of 1 to 18, and is especially preferably an optionally substituted alkyl group having a carbon number of 2 to 12.

In the previously mentioned formula (1-1), Xa represents an optionally substituted organic group having a

carbon number of 1 to 20. Examples of the organic group having a carbon number of 1 to 20 include an optionally substituted alkylene group having a carbon number of 1 to 18, an optionally substituted alicyclic group having a carbon number of 3 to 18, and an optionally substituted aromatic hydrocarbon cyclic group having a carbon number of 6 to 18.

Examples of possible substituents of Xa include halogen atoms such as a fluorine atom and a chlorine atom; a cyano group; alkyl groups having a carbon number of 1 to 6 such as a methyl group, an ethyl group, a propyl group, an isopropyl group, a butyl group, a sec-butyl group, and a tert-butyl group; alkenyl groups having a carbon number of 2 to 6 such as a vinyl group and an allyl group; haloalkyl groups having a carbon number of 1 to 6 such as a trifluoromethyl group; N,N-dialkylamino groups having a carbon number of 2 to 12 such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; $-OCF_3$; $-C(=O)-R^b$; $-C(=O)-O-R^b$; $-O-C(=O)-R^b$; and $-SO_2R^a$.

$R^a$ represents an alkyl group having a carbon number of 1 to 6 such as a methyl group or an ethyl group; or an aromatic hydrocarbon cyclic group having a carbon number of 6 to 20 that is optionally substituted with an alkyl group having a carbon number of 1 to 6 or an alkoxy group having a carbon number of 1 to 6 (for example, a phenyl group, a 4-methylphenyl group, or a 4-methoxyphenyl group).

$R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18.

In a case in which a plurality of substituents is present, these substituents may be the same or different.

Halogen atoms, a cyano group, alkyl groups having a carbon number of 1 to 6, haloalkyl groups having a carbon number of 1 to 6, alkoxy groups having a carbon number of 1 to 6, and a nitro group are preferable as substituents of Xa from a viewpoint of improving solubility.

In a case in which Xa has a plurality of the substituents described above, these substituents may be the same or different.

Xa is preferably a group represented by any one of the following formulae (VII-1) to (VII-29). Note that the groups represented by the following formulae may have any of the substituents described above.

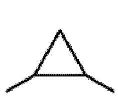
(VII-1)
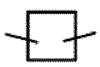
(VII-2)
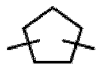
(VII-3)
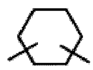
(VII-4)
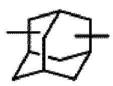
(VII-5)
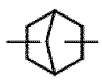
(VII-6)

$-CH_2-$ $-(CH_2)_2-$(VII-7) (VII-8) $-(CH_2)_3-$ $-(CH_2)_4-$ $-(CH_2)_5-$ $-(CH_2)_6-$ $-(CH_2)_7-$ $-(CH_2)_8-$ $-(CH_2)_9-$(VII-9) (VII-10) (VII-11) (VII-12) (VII-13) (VII-14) (VII-15) $-(CH_2)_{10}-$ $-(CH_2)_{11}-$ $-(CH_2)_{12}-$ $-(CH_2)_{13}-$ $-(CH_2)_{14}-$ $-(CH_2)_{15}-$(VII-16) (VII-17) (VII-18) (VII-19) (VII-20) (VII-21)

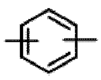
(VII-22)
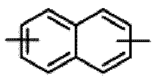
(VII-23)
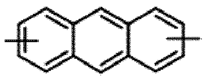
(VII-24)
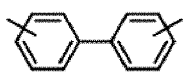
(VII-25)
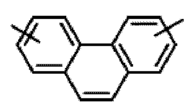
(VII-26)

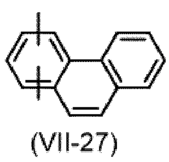
(VII-27)
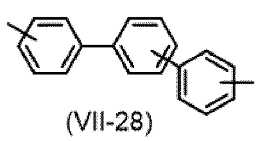
(VII-28)
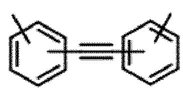
(VII-29)

In the previously mentioned formula (1-1), $Z^1$ to $Z^4$ each represent, independently of one another, a single bond, -O-, $-O-CH_2-$, $-CH_2-O-$, -C(=O)-O-, -O-C(=O)-, -C(=O)-S-, -S-C(=O)-, $-NR^{20}-C(=O)-$, $-C(=O)-NR^{20}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$,$-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, $-CH_2-C(=O)-O-$, $-O-C(=O)-CH_2-$, $-CH_2-O-C(=O)-$, $-C(=O)-O-CH_2-$, $-O-CH_2-CH_2-$, $-CH_2-CH_2-O-$, or -C≡C-. $R^{20}$ represents a hydrogen atom or an alkyl group having a carbon number of 1 to 6.

Of these examples, $Z^1$ and $Z^4$ are preferably each, independently of one another, -C(=O)-O- or -O-C(=O)-.

$Z^2$ and $Z^3$ are preferably each, independently of one another, -C(=O)-O-, -O-C(=O)-, -C(=O)-S-, -S-C(=O)-, $-NR^{20}C(=O)-$, $-C(=O)-NR^{20}-$, $-CF_2-O-$, $-O-CF_2-$, $-CF_2-CF_2-$, -CH=CH-C(=O)-O-, -O-C(=O)-CH=CH-,

$-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, $-CH_2-C(=O)-O-$, $-O-C(=O)-CH_2-$ $-CH_2-O-C(=O)-$, $-C(=O)-O-CH_2-$, or -C≡C-, more preferably -C(=O)-O-, -O-C(=O)-, -C(=O)-S-, -S-C(=O)-, $-NR^{20}C(=O)-$, or $-C(=O)-NR^{20}-$, particularly preferably -C(=O)-O-, -O-C(=O)-, $-NR^{20}C(=O)-$, $-C(=O)-NR^{20}-$, $-CH_2-C(=O)-O-$, $-O-C(=O)-CH_2-$, $-CH_2-O-C(=O)-$, or $-C(=O)-O-CH_2-$, and most preferably -C(=O)-O-, -O-C(=O)-, $-CH_2-O-C(=O)-$, or $-C(=O)-O-CH_2-$.

In the previously mentioned formula (1-1), $L^1$ and $L^2$ are each, independently of one another, an organic group that is either an alkylene group having a carbon number of 1 to 20 or a group in which at least one methylene group ($-CH_2-$) included in an alkylene group having a carbon number of 1 to 20 is replaced by -O- or -C(=O)-, where hydrogen atoms included in the organic groups of $L^1$ and $L^2$ may be replaced by one or more substituents selected from the group consisting of an alkyl group having a carbon number of 1 to 5, an alkoxy group having a carbon number of 1 to 5, and a halogen atom. Note that in the "group in which at least one methylene group ($-CH_2-$) included in an alkylene group having a carbon number of 1 to 20 is replaced by -O- or -C(=O)-", it is preferable that consecutive methylene groups in the alkylene group are not replaced by -O- (i.e., an -O-O- structure is not formed) and that consecutive methylene groups in the alkylene group are not replaced by -C(=O)- (i.e., a -C(=O)-C(=O)- structure is not formed). Moreover, methylene groups ($-CH_2-$) at both ends of $L^1$ and $L^2$ are not replaced by -O- or -C(=O)-.

The organic groups of $L^1$ and $L^2$ are preferably each an alkylene group having a carbon number of 1 to 20 that is optionally substituted with a fluorine atom or a group represented by $-(CH_2)_j-C(=O)-O-(CH_2)_k-$ (j and k in the formula each represent an integer of 2 to 12, and preferably each represent an integer of 2 to 8) that is optionally substituted with a fluorine atom, are more preferably each an alkylene group having a carbon number of 2 to 12 that is optionally substituted with a fluorine atom, are even more preferably each an unsubstituted alkylene group having a carbon number of 2 to 12, and are particularly preferably each a group represented by $-(CH_2)_1-$ (1 in the formula represents an integer of 2 to 12, and preferably represents an integer of 2 to 8).

In the previously mentioned formula (1-1), $A^1$, $A^2$, $B^1$, and $B^2$ each represent, independently of one another, an optionally substituted alicyclic group or an optionally substituted aromatic group, and preferably an optionally substituted alicyclic group having a carbon number of 5 to 20 or an optionally substituted aromatic group having a carbon number of 2 to 20.

Specific examples of the alicyclic group include cycloalkanediyl groups having a carbon number of 5 to 20 such as a cyclopentane-1,3-diyl group, a cyclohexane-1,4-diyl group, a 1,4-cycloheptane-1,4-diyl group, and a cyclooctane-1,5-diyl group; and bicycloalkanediyl groups having a carbon number of 5 to 20 such as a decahydronaphthalene-1,5-diyl group and a decahydronaphthalene-2,6-diyl group. Of these examples, the alicyclic group is preferably an optionally substituted cycloalkanediyl group having a carbon number of 5 to 20, more preferably a cyclohexanediyl group, and particularly preferably a cyclohexane-1,4-diyl group represented by the following formula (a). The alicyclic group may be a trans isomer represented by formula (a1), a cis isomer represented by formula (a2), or a mixture of the trans isomer and the cis isomer, but is more preferably the trans isomer represented by formula (a1).

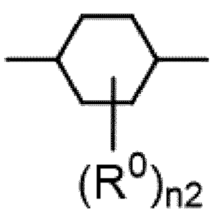
(a)

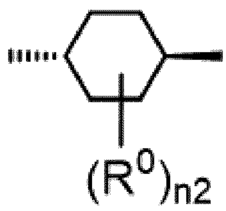
(a1)

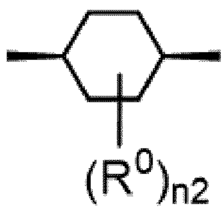
(a2)

(In the formulae, $R^0$ and n2 have the same meaning as previously described and preferable examples thereof are also the same as previously described. Note that in a case in which more than one $R^0$ is included, each $R^0$ may be the same or different.)

Specific examples of the aromatic group include aromatic hydrocarbon cyclic groups having a carbon number of 6 to 20 such as a 1,2-phenylene group, a 1,3-phenylene group, a 1,4-phenylene group, a 1,4-naphthylene group, a 1,5-naphthylene group, a 2,6-naphthylene group, and a 4,4'-biphenylene group; and aromatic heterocyclic groups having a carbon number of 2 to 20 such as a furan-2,5-diyl group, a thiophene-2,5-diyl group, a pyridine-2,5-diyl group, and a pyrazine-2,5-diyl group. Of these examples, the aromatic group is preferably an aromatic hydrocarbon cyclic group having a carbon number of 6 to 20, more preferably a phenylene group, and particularly preferably a 1,4-phenylene group represented by the following formula (b).

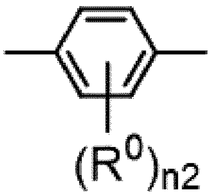

(b)

(In the formula, $R^0$ and n2 have the same meaning as previously described and preferable examples thereof are also the same as previously described. Note that in a case in which more than one $R^0$ is included, each $R^0$ may be the same or different.)

Examples of possible substituents of the alicyclic group and the aromatic group include halogen atoms such as a fluorine atom, a chlorine atom, and a bromine atom; alkyl groups having a carbon number of 1 to 6 such as a methyl group and an ethyl group; alkoxy groups having a carbon number of 1 to 5 such as a methoxy group and an isopropoxy group; a nitro group; and a cyano group. The alicyclic group, alicyclic group having a carbon number of 5 to 20, aromatic group, or aromatic group having a carbon number of 2 to 20 may have one or more substituents selected from the substituents described above. In a case in which the group has a plurality of substituents, these substituents may be the same or different.

In the previously mentioned formula (1-1), $Y^1$ to $Y^4$ each represent, independently of one another, a single bond, -O-, -C(=O)-, -C(=O)-O-, -O-C(=O)-, $-NR^{21}-C(=O)-$, $-C(=O)-NR^{21}-$, -O-C(=O)-O-, $-NR^{21}-C(=O)-O-$, $-O-C(=O)-NR^{21}-$, or $-NR^{21}-C(=O)-NR^{22}-$. $R^{21}$ and $R^{22}$ each represent, independently of one another, a hydrogen atom or an alkyl group having a carbon number of 1 to 6.

Of these examples, $Y^1$ to $Y^4$ are preferably each, independently of one another, -O-, -C(=O)-, -C(=O)-O-, -O-C(=O)-O-, or -O-C(=O)-.

In a case in which more than one $Y^1$ or $Y^2$ is present, each $Y^1$ or $Y^2$ may be the same or different.

In the previously mentioned formula (1-1), one of $P^1$ and $P^2$ represents a hydrogen atom or a polymerizable group and the other of $P^1$ and $P^2$ represents a polymerizable group. $P^1$ and $P^2$ preferably each represent, independently of one another, a polymerizable group.

Examples of the polymerizable group of $P^1$ and $P^2$ include a group represented by $CH_2=CR^1-C(=O)-O-$ ($R^1$ represents a hydrogen atom, a methyl group, or a chlorine atom) such as an acryloyloxy group or a methacryloyloxy group, a vinyl group, a vinyl ether group, a p-stilbene group, an acryloyl group, a methacryloyl group, a carboxyl group, a methylcarbonyl group, a hydroxy group, an amide group, an alkylamino group having a carbon number of 1 to 4, an amino group, an epoxy group, an oxetanyl group, an aldehyde group, an isocyanate group, and a thioisocyanate group. Of these polymerizable groups, a group represented by $CH_2=CR^1-C(=O)-O-$ such as the following formula (IV) is preferable, $CH_2=CH-C(=O)-O-$ (acryloyloxy group) or $CH_2=C(CH_3)-C(=O)-O-$ (methacryloyloxy group) is more preferable, and an acryloyloxy group is even more preferable. In a case in which two $R^1$ groups are present in the polymerizable compound (1-1), each $R^1$ may be the same or different. Moreover, $P^1$ and $P^2$ may be different, but are preferably the same polymerizable group.

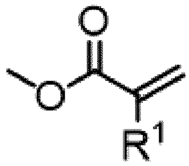

(IV)

[$R^1$ in formula (IV) represents a hydrogen atom, a methyl group, or a chlorine atom.]

In formula (1-1), p and q are each, independently of one another, an integer of 0 to 2, preferably each, independently of one another, 0 or 1, and more preferably 0.

The polymerizable compound (1-1) preferably has a symmetrical structure with Xa as the center (i.e., $Z^2$ and $Z^3$, $Ar^0$ and $Ar^1$, $Z^1$ and $Z^4$, $A^1$ and $A^2$, $Y^1$ and $Y^2$, $B^1$ and $B^2$, p and q, $Y^3$ and $Y^4$, $L^1$ and $L^2$, and $P^1$ and $P^2$ are preferably the same as one another), but is not specifically limited to having such a structure.

The presently disclosed polymerizable compound is preferably a polymerizable compound indicated by any one of the following formulae (III-1) to (III-6), and more preferably a polymerizable compound indicated by any one of the following formulae (VI-1) to (VI-3).

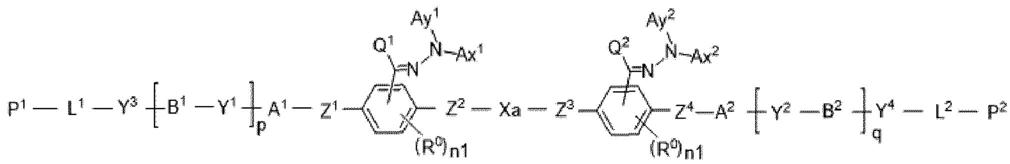

(III-1)

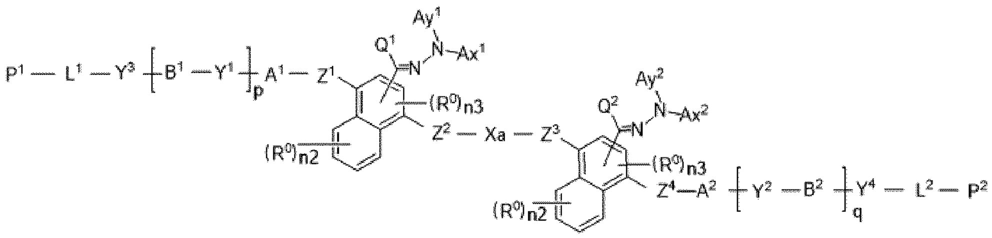

(III-2)

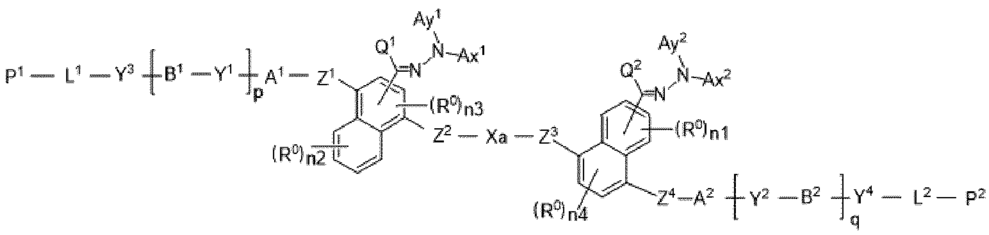

(III-3)

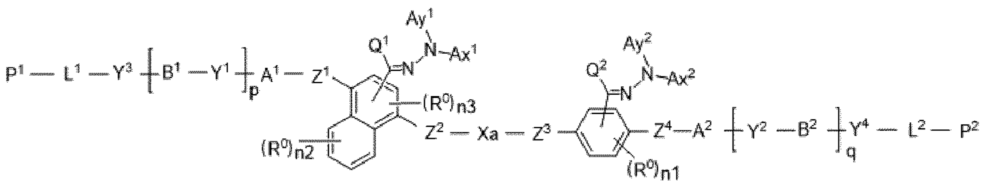

(III-4)

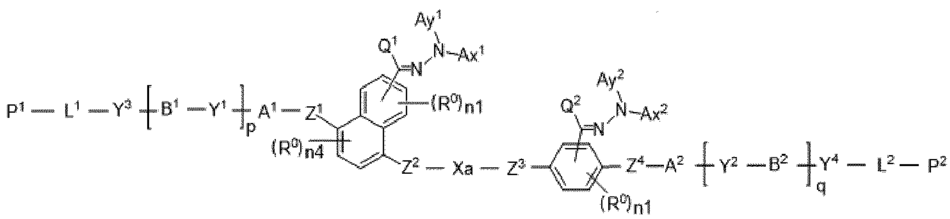

(III-5)

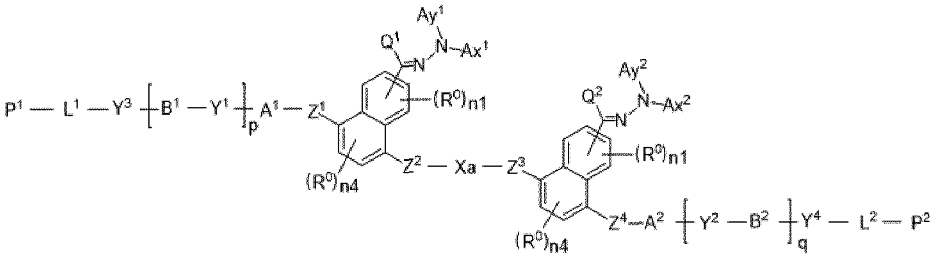

(III-6)

[In formulae (III-1) to (III-6),

$Z^1$ to $Z^4$, $A^1$, $A^2$, $B^1$, $B^2$, $Y^1$ to $Y^4$, $L^1$, $L^2$, $P^1$, $P^2$, Xa, $R^0$, n1, n2, n3, n4, p, and q have the same meaning as previously described and preferable examples thereof are also the same as previously described,
n1, n2, n3, and n4 on the left and right sides of Xa may be the same or different,
$Ax^1$ and $Ax^2$ each represent, independently of one another, an organic group including at least one aromatic ring selected from the group consisting of an aromatic hydrocarbon ring having a carbon number of 6 to 30 and an

aromatic heterocyclic ring having a carbon number of 2 to 30, where the aromatic rings of $Ax^1$ and $Ax^2$ are optionally substituted, $Ay^1$ and $Ay^2$ each represent, independently of one another, a hydrogen atom or an optionally substituted organic group having a carbon number of 1 to 30, $Q^1$ and $Q^2$ each represent, independently of one another, a hydrogen atom or an alkyl group having a carbon number of 1 to 6,
specific examples and preferable examples of $Ax^1$, $Ax^2$, $Ay^1$, $Ay^2$, $Q^1$, and $Q^2$ are the same as for Ax, Ay, and Q, and in a case in which more than one $B^1$, $B^2$, $Y^1$, $Y^2$, or $R^0$ is present, each $B^1$, $B^2$, $Y^1$, $Y^2$, or $R^0$ may be the same or different.]

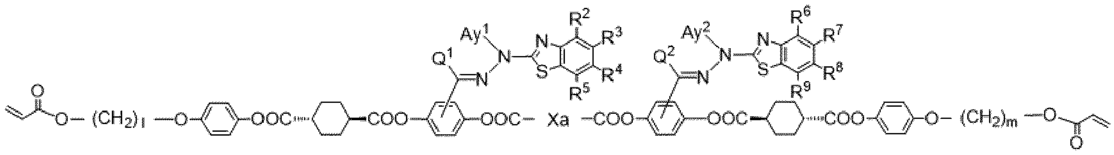

(VI-1)

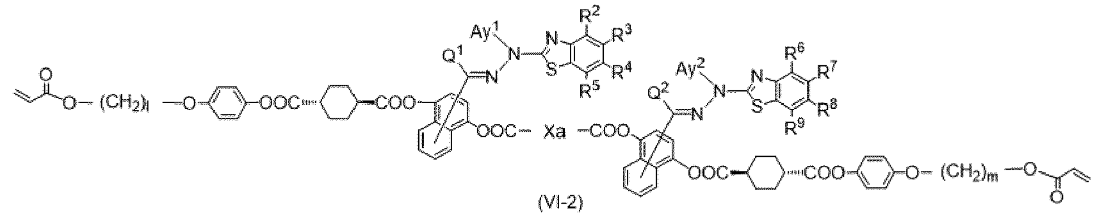

(VI-2)

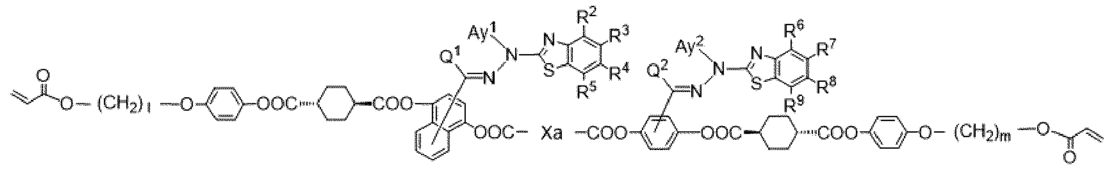

(VI-3)

[In formulae (VI-1) to (VI-3), $R^2$ to $R^9$ each represent, independently of one another, a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, $-OCF_3$, $-O-C(=O)-R^b$, or $-C(=O)-O-R^b$, where $R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18. The plurality of $R^2$ to $R^9$ groups may be the same or different, and one or more of ring constituents C-$R^2$ to C-$R^9$ may be replaced by a nitrogen atom.

Specific examples and preferable examples of $R^2$ to $R^9$ are the same as for $R^2$ to $R^5$ in formula (V).

$Ay^1$, $Ay^2$, $Q^1$, and $Q^2$ have the same meaning as previously described and preferable examples thereof are also the same as previously described. Moreover, 1 and m each represent, independently of one another, an integer of 1 to 18.]

The polymerizable compound (1-1) set forth above can be synthesized through a combination of known synthetic reactions. Specifically, the polymerizable compound (1-1) can be synthesized with reference to methods described in various documents (for example, March's Advanced Organic Chemistry (Wiley); Sandler and Karo, "Syntheses of Organic Compounds Classified by Functional Group", joint translation by Naoki INAMOTO (Hirokawa Publishing Company); and Greene's Protective Groups in Organic Synthesis, Fourth Edition (Wiley)).

(2-1) Polymerizable liquid crystal mixture

The presently disclosed polymerizable liquid crystal mixture is a mixture containing the polymerizable compound (1-1) set forth above as a main component. The mixture and a polymerizable composition containing the mixture can advantageously be used in production of the subsequently described polymer, optical film, and optically anisotropic body. The term "main component" refers to "a component having a highest percentage content in terms of solid content".

In a case in which the polymerizable compound (1-1) does not display liquid crystallinity, the mixture can be caused to display liquid crystallinity by mixing the polymerizable compound (1-1) with a polymerizable compound that does display liquid crystallinity.

The mixing ratio of the polymerizable compound (1-1) set forth above in the polymerizable liquid crystal mixture, in terms of solid content, is preferably more than 50 mass% and less than 100 mass%, more preferably not less than 55 mass% and less than 100 mass%, and particularly preferably not less than 60 mass% and less than 100 mass%.

The mixing ratio of the polymerizable compound (1-1) can be measured by high-performance liquid chromatography (HPLC).

Examples of components other than the polymerizable compound (1-1) set forth above that may be contained in the polymerizable liquid crystal mixture include, but are not specifically limited to, polymerizable compounds having a chemical structure differing from the polymerizable compound (1-1) set forth above such as a by-product produced in preparation of the polymerizable compound (1-1) set forth above, a polymerizable compound indicated by formula (I-2), shown below, which is described further below (hereinafter, also referred to as "polymerizable compound (1-2)"), and copolymerizable monomers such as described further below, and preferable embodiments thereof are also the same.

The presently disclosed polymerizable liquid crystal mixture contains the polymerizable compound (1-1) as a main component as previously described and, in a case in which the presently disclosed polymerizable liquid crystal mixture contains the polymerizable compound (1-1) and a polymerizable compound having a chemical structure differing from the polymerizable compound (1-1), an area value for the polymerizable compound (1-1) relative to a sum total of area values for the polymerizable compound (1-1) and the polymerizable compound having a chemical structure differing from the polymerizable compound (1-1), as measured by high-performance liquid chromatography (HPLC), is preferably more than 50%, more preferably not less than 55% and less than 100 %, and particularly preferably not less than 60% and less than 100 %.

An optical film or the like having good reverse wavelength dispersion at long wavelengths can be produced when the aforementioned area value is more than 50%, and an optical film or the like having even better reverse wavelength dispersion at long wavelengths can be produced when the aforementioned area value is within the more preferable range or the particularly preferable range.

(2-2) Polymerizable compound (I-2)

The polymerizable compound (I-2) is indicated by the following formula (I-2).

$$P^3-L^3-Y^7\left[B^3-Y^5\right]_{p1}A^3-Z^5-Ar^2-Z^6-A^4\left[Y^6-B^4\right]_{q1}Y^8-L^4-P^4$$

(I − 2) .

[In formula (I-2),

$Ar^2$ has the same meaning as $Ar^0$ and $Ar^1$ and preferable examples thereof are also the same,
$Z^5$ and $Z^6$ each, independently of one another, have the same meaning as $Z^1$ to $Z^4$ and preferable examples thereof are also the same,
$A^3$, $A^4$, $B^3$, and $B^4$ each, independently of one another, have the same meaning as $A^1$, $A^2$, $B^1$, and $B^2$ and preferable examples thereof are also the same,
$Y^5$ to $Y^8$ each, independently of one another, have the same meaning as $Y^1$ to $Y^4$ and preferable examples thereof are also the same,
$L^3$ and $L^4$ each, independently of one another, have the same meaning as $L^1$ and $L^2$ and preferable examples thereof are also the same,
$P^3$ and $P^4$ each, independently of one another, have the same meaning as $P^1$ and $P^2$ and preferable examples thereof are also the same,
p1 and q1 each, independently of one another, have the same meaning as p and q and preferable examples thereof are also the same, and
in a case in which more than one $B^3$, $B^4$, $Y^5$, or $Y^6$ is present, each $B^3$, $B^4$, $Y^5$, or $Y^6$ may be the same or different.]

In a case in which the presently disclosed polymerizable liquid crystal mixture contains the polymerizable compound (1-1) and the polymerizable compound (I-2), an area value for the polymerizable compound (1-1) relative to a sum total of area values for the polymerizable compound (1-1) and the polymerizable compound (I-2), as measured by high-performance liquid chromatography (HPLC), is preferably more than 50%, more preferably not less than 55 mass% and less than 100 mass%, and particularly preferably not less than 60 mass% and less than 100 mass%.

An optical film or the like having good reverse wavelength dispersion at long wavelengths can be produced

when the aforementioned area value is more than 50%, and an optical film or the like having even better reverse wavelength dispersion at long wavelengths can be produced when the aforementioned area value is within the more preferable range or the particularly preferable range.

(2-3) Polymerizable composition

The polymerizable composition contains at least the polymerizable compound (1-1) and a polymerization initiator, and preferably contains the polymerizable liquid crystal mixture set forth above (mixture containing polymerizable compound (1-1) as main component), a polymerization initiator, and a solvent.

The polymerizable composition is useful as a production raw material for the presently disclosed polymer, optical film, and optically anisotropic body as described further below. Moreover, the presently disclosed polymerizable composition enables favorable production of an optical film or the like having good reverse wavelength dispersion at long wavelengths.

The polymerization initiator is included from a viewpoint of more efficiently carrying out a polymerization reaction of the polymerizable compound (1-1) contained in the polymerizable composition.

Examples of polymerization initiators that may be used include radical polymerization initiators, anionic polymerization initiators, and cationic polymerization initiators.

Although both thermal radical generators, which are compounds that generate active species that can initiate polymerization of a polymerizable compound upon heating, and photo-radical generators, which are compounds that generate active species that can initiate polymerization of a polymerizable compound upon exposure to exposure light such as visible light rays, ultraviolet rays (i-line, etc.), far ultraviolet rays, an electron beam, or X-rays, can be used as the radical polymerization initiator, use of a photo-radical generator is preferable.

Examples of photo-radical generators that may be used include acetophenone compounds, biimidazole compounds, triazine compounds, O-acyl oxime compounds, onium salt compounds, benzoin compounds, benzophenone compounds, $\alpha$-diketone compounds, polynuclear quinone compounds, xanthone compounds, diazo compounds, and imide sulfonate compounds. These compounds are components that generate active radicals, active acid, or both active radicals and active acid upon photoexposure. One photo-radical generator may be used individually, or two or more photo-radical generators may be used in combination.

Specific examples of acetophenone compounds that may be used include 2-hydroxy-2-methyl-1-phenylpropan-1-one, 2-methyl-1-[4-(methylthio)phenyl]-2-morpholinopropan-1-one, 2-benzyl-2-dimethylamino-1-(4-morpholinophenyl)butan-1-one, 1-hydroxycyclohexyl phenyl ketone, 2,2-dimethoxy-1,2-diphenylethan-1-one, 1,2-octanedione, and 2-benzyl-2-dimethylamino-4'-morpholinobutyrophenone.

Specific examples of biimidazole compounds that may be used include 2,2'-bis(2-chlorophenyl)-4,4',5,5'-tetrakis(4-ethoxycarbonylphenyl)-1,2'-biim idazole, 2,2'-bis(2-bromophenyl)-4,4',5,5'-tetrakis(4-ethoxycarbonylphenyl)-1,2'-biim idazole, 2,2'-bis(2-chlorophenyl)-4,4',5,5'-tetraphenyl-1,2'-biimidazole, 2,2'-bis(2,4-dichlorophenyl)-4,4',5,5'-tetraphenyl-1,2'-biimidazole, 2,2'-bis(2,4,6-trichlorophenyl)-4,4',5,5'-tetraphenyl-1,2'-biimidazole, 2,2'-bis(2-bromophenyl)-4,4',5,5'-tetraphenyl-1,2'-biimidazole, 2,2'-bis(2,4-dibromophenyl)-4,4',5,5'-tetraphenyl-1,2'-biimidazole, and 2,2'-bis(2,4,6-tribromophenyl)-4,4',5,5'-tetraphenyl-1,2'-biimidazole.

In a situation in which a biimidazole compound is used as a photoinitiator (photo-radical generator) in the present disclosure, it is preferable that a hydrogen donor is used in combination therewith in terms that sensitivity can be further enhanced.

The term "hydrogen donor" refers to a compound that can donate a hydrogen atom to a radical generated from the biimidazole compound upon photoexposure. The hydrogen donor is preferably a mercaptan compound, an amine compound, or the like such as defined below.

Specific examples of mercaptan compounds that may be used include 2-mercaptobenzothiazole, 2-mercaptobenzoxazole, 2-mercaptobenzimidazole, 2,5-dimercapto-1,3,4-thiadiazole, and 2-mercapto-2,5-dimethylaminopyridine. Examples of amine compounds that may be used include 4,4'-bis(dimethylamino)benzophenone, 4,4'-bis(diethylamino)benzophenone, 4-diethylaminoacetophenone, 4-dimethylaminopropiophenone, ethyl 4-dimethylaminobenzoate, 4-dimethylaminobenzoic acid, and 4-dimethylaminobenzonitrile.

Specific examples of triazine compounds that may be used include halomethyl group-containing triazine compounds such as 2,4,6-tris(trichloromethyl)-s-triazine, 2-methyl-4,6-bis(trichloromethyl)-s-triazine, 2-[2-(5-methylfuran-2-yl)ethenyl]-4,6-bis(trichloromethyl)-s-triazine, 2-[2-(furan-2-yl)ethenyl]-4,6-bis(trichloromethyl)-s-triazine, 2-[2-(4-diethylamino-2-methylphenyl)ethenyl]-4,6-bis(trichloromethyl)-s-tria zine, 2-[2-(3,4-dimethoxyphenyl)ethenyl]-4,6-bis(trichloromethyl)-s-triazine, 2-(4-methoxyphenyl)-4,6-bis(trichloromethyl)-s-triazine, 2-(4-ethoxystyryl)-4,6-bis(trichloromethyl)-s-triazine, and 2-(4-n-butoxyphenyl)-4,6-bis(trichloromethyl)-s-triazine.

Specific examples of O-acyl oxime compounds that may be used include 1-[4-(phenylthio)phenyl]-heptan-1,2-dione 2-(O-benzoyloxime), 1-[4-(phenylthio)phenyl]-octan-1,2-dione 2-(O-benzoyloxime), 1-[4-(benzoyl)phenyl]-octan-1,2-dione 2-(O-benzoyloxime), 1-[9-ethyl-6-(2-methylbenzoyl)-9H-carbazol-3-yl]-ethanone 1-(O-acetyloxime), 1-[9-ethyl-6-(3-methylbenzoyl)-9H-carbazol-3-yl]ethanone 1-(O-acetyloxime), 1-(9-ethyl-6-benzoyl-9H-carbazol-3-yl)-ethanone 1-(O-acetyloxime), ethanone-1-[9-ethyl-6-(2-methyl-4-tetrahydrofuranylbenzoyl)-9H-carbazol-3-yl]-1-(O-acetyloxime), ethanone-1-[9-ethyl-6-(2-methyl-4-tetrahydropyranylbenzoyl)-9H-carbazol-3-yl]-1-(O-acetyloxime), ethanone-1-[9-ethyl-6-(2-methyl-5-tetrahydrofuranylbenzoyl)-9H-carbazol-3-yl]-1-(O-acetyloxime), ethanone-1-[9-ethyl-6-(2-methyl-5-tetrahydropyranylbenzoyl)-9H-carbazol-3-yl]-1-(O-acetyloxime), ethanone-1-[9-ethyl-6-{2-methyl-4-(2,2-dimethyl-1,3-dioxolanyl)benzoyl}-9 H-carbazol-3-yl]-1-(O-acetyloxime), ethanone-1-[9-ethyl-6-(2-methyl-4-tetrahydrofuranylmethoxybenzoyl)-9H-car bazol-3-yl]-1-(O-acetyloxime), ethanone-1-[9-ethyl-6-(2-methyl-4-tetrahydropyranylmethoxybenzoyl)-9H-ca rbazol-3-yl]-1-(O-acetyloxime), ethanone-1-[9-ethyl-6-(2-methyl-5-tetrahydrofuranylmethoxybenzoyl)-9H-car bazol-3-yl]-1-(O-acetyloxime), ethanone-1-[9-ethyl-6-(2-methylbenzoyl)-9H-carbazol-3-yl]-1-(O-acetyloxim e), ethanone-1-[9-ethyl-6-(2-methyl-5-tetrahydropyranylmethoxybenzoyl)-9H-ca rbazol-3-yl]-1-(O-acetyloxime), and ethanone-1-[9-ethyl-6-{2-methyl-4-(2,2-dimethyl-1,3-dioxolanyl)methoxyben zoyl}-9H-carbazol-3-yl]-1-(O-acetyloxime).

A commercially available product may be used as a photo-radical generator. Specific examples include Irgacure 907 (product name), Irgacure 184 (product name), Irgacure 369 (product name), Irgacure 651 (product name), Irgacure 819 (product name), Irgacure 907 (product name), and Irgacure OXE02 (product name) produced by BASF, and ADEKA ARKLS N1919T (product name) produced by ADEKA Corporation.

Examples of anionic polymerization initiators that may be used include alkyllithium compounds; monolithium salts and monosodium salts of biphenyl, naphthalene, pyrene, and the like; and polyfunctional initiators such as dilithium salts and trilithium salts.

Examples of cationic polymerization initiators that may be used include proton acids such as sulfuric acid, phosphoric acid, perchloric acid, and trifluoromethanesulfonic acid; Lewis acids such as boron trifluoride, aluminum chloride, titanium tetrachloride, and tin tetrachloride; aromatic onium salts; and combinations of an aromatic onium salt and a reducing agent.

One of these polymerization initiators may be used individually, or two or more of these polymerization initiators may be used in combination.

The proportion in which the polymerization initiator is compounded in the polymerizable composition is normally 0.1 parts by mass to 30 parts by mass, and preferably 0.5 parts by mass to 10 parts by mass per 100 parts by mass of polymerizable compound contained in the polymerizable composition.

Moreover, a surfactant is preferably compounded in the polymerizable composition in order to adjust surface tension. Although no specific limitations are placed on the surfactant, a non-ionic surfactant is normally preferable. The non-ionic surfactant may be a commercially available product and may, for example, be a non-ionic surfactant that is an oligomer including a fluorine-containing group, a hydrophilic group, and a lipophilic group. Examples include the SURFLON series (S242, S243, S386, S611, S651, etc.) produced by AGC Seimi Chemical Co., Ltd., the MEGAFACE series (F251, F554, F556, F562, RS-75, RS-76-E, etc.) produced by DIC Corporation, and the Ftergent series (FTX601AD, FTX602A, FTX601ADH2, FTX650A, etc.) produced by Neos Company Limited. One of these surfactants may be used individually, or two or more of these surfactants may be used in combination in a freely selected ratio.

The proportion in which the surfactant is compounded in the polymerizable composition is normally 0.01 parts by mass to 10 parts by mass, and preferably 0.01 parts by mass to 2 parts by mass per 100 parts by mass of polymerizable compound contained in the polymerizable composition.

Besides the polymerizable compound, the polymerization initiator, and the surfactant, the polymerizable composition may further contain other components to the extent that the effects disclosed herein are not affected. Examples of these other components include metals, metal complexes, dyes, pigments, fluorescent materials, phosphorescent materials, leveling agents, thixotropic agents, gelling agents, polysaccharides, ultraviolet absorbers, infrared absorbers, antioxidants, ion exchange resins, and metal oxides such as titanium oxide.

Moreover, other copolymerizable monomers may be used as other components. Specific examples include, but are not specifically limited to, 4'-methoxyphenyl 4-(2-methacryloyloxyethyloxy)benzoate, biphenyl 4-(6-methacryloyloxyhexyloxy)benzoate, 4'-cyanobiphenyl 4-(2-acryloyloxyethyloxy)benzoate, 4'-cyanobiphenyl 4-(2-methacryloyloxyethyloxy)benzoate, 3',4'-difluorophenyl 4-(2-methacryloyloxyethyloxy)benzoate, naphthyl 4-(2-methacryloyloxyethyloxy)benzoate, 4-acryloyloxy-4'-decylbiphenyl, 4-acryloyloxy-4'-cyanobiphenyl, 4-(2-acryloyloxyethyloxy)-4'-cyanobiphenyl, 4-(2-methacryloyloxyethyloxy)-4'-methoxybiphenyl, 4-(2-methacryloyloxyethyloxy)-4'-(4"-fluorobenzyloxy)biphenyl, 4-acryloyloxy-4'-propylcyclohexylphenyl, 4-methacryloyl-4'-butylbicyclohexyl, 4-acryloyl-4'-amyltolan, 4-acryloyl-4'-(3,4-difluorophenyl)bicyclohexyl, 4-amylphenyl 4-(2-acryloyloxyethyl)benzoate, 4-(4'-propylcyclohexyl)phenyl 4-(2-acryloyloxyethyl)benzoate, LC-242 (product name) produced by BASF, trans-1,4-bis[4-[6-(acryloyloxy)hexyloxy]phenyl]cyclohexanedicarboxylate, and other copolymerizable monomers such as compounds disclosed in JP 2007-002208 A, JP 2009-173893 A, JP 2009-274984 A, JP 2010-030979 A, JP 2010-031223 A, JP 2011-006360 A, JP 2010-24438 A, WO 2012/141245 A1, WO 2012/147904 A1, WO 2012/169424 A1, WO 2012/76679 A1, WO 2013/180217 A1, WO 2014/010325 A1, WO 2014/061709 A1, WO 2014/065176 A1, WO 2014/126113 A1, WO 2015/025793 A1, WO 2015/064698 A1, WO 2015/122384 A1, and WO 2015/122385 A1.

The proportion in which these other components are compounded is normally 0.005 parts by mass to 50 parts by mass per 100 parts by mass of polymerizable compound contained in the polymerizable composition.

The polymerizable composition can normally be produced by mixing/dissolving specific amounts of the polymerizable compound, the polymerization initiator, other components compounded as desired, and so forth in an appropriate organic solvent.

Examples of organic solvents that may be used include ketones such as cyclopentanone, cyclohexanone, and methyl ethyl ketone; acetic acid esters such as butyl acetate and amyl acetate; halogenated hydrocarbons such as chloroform, dichloromethane, and dichloroethane; and ethers such as 1,4-dioxane, cyclopentyl methyl ether, tetrahydrofuran, tetrahydropyran, and 1,3-dioxolane.

(3) Polymer

The presently disclosed polymer is obtained through polymerization of the previously described polymerizable compound (1-1), the previously described polymerizable liquid crystal mixture, the previously described mixture, or the previously described polymerizable composition.

Herein, the term "polymerization" is used to refer to a chemical reaction in a broad sense that is inclusive of a normal polymerization reaction and also a crosslinking reaction.

The presently disclosed polymer normally includes the following monomer unit (repeating unit (I-1)') derived from the polymerizable compound (1-1).

The following indicates, as one example, the structure of the repeating unit (I-1)' in a case in which the used polymerizable compound (1-1) has polymerizable groups represented by $CH_2$=$CR^1$-C(=O)-O- as $P^1$ and $P^2$.

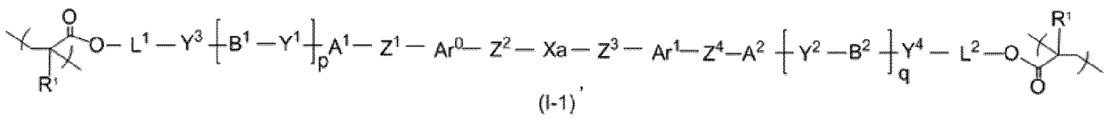

(I-1)'

[In formula (I-1)', Xa, $Ar^0$, $Ar^1$, $Z^1$, $Z^2$, $Z^3$, $Z^4$, $A^1$, $A^2$, $B^1$, $B^2$, $Y^1$, $Y^2$, $Y^3$, $Y^4$, $L^1$, $L^2$, $R^1$, p, and q have the same meaning as previously described.]

The presently disclosed polymer can favorably be used as a constituent material of an optical film or the like as a result of being produced using the previously described polymerizable compound (1-1), the previously described polymerizable liquid crystal mixture, or the previously described mixture.

The presently disclosed polymer may be used in any form depending on the application, such as in the form of a film, a powder, or a layer of aggregated powder, without any specific limitations.

Specifically, a film of the polymer can favorably be used as a constituent material of the subsequently described optical film and optically anisotropic body, a powder of the polymer can be used for a paint, an anti-counterfeiting article, a security article, or the like, and a layer formed from a powder of the polymer can favorably be used as a constituent material of an optically anisotropic body.

The presently disclosed polymer can be suitably produced by ($\alpha$) carrying out a polymerization reaction of the previously described polymerizable compound (1-1), the previously described polymerizable liquid crystal mixture, the previously described mixture, or the previously described polymerizable composition in the presence of an appropriate organic solvent, subsequently isolating the target polymer, dissolving the obtained polymer in an appropriate organic solvent to prepare a solution, applying the solution onto an appropriate substrate to obtain an applied film, drying the applied film, and subsequently performing heating as desired, or ($\beta$) dissolving the previously described polymerizable compound (1-1), the previously described polymerizable liquid crystal mixture, the previously described mixture, or the previously described polymerizable composition in an organic solvent, applying the resultant solution onto a substrate by a commonly known application method, removing the solvent, and then carrying out a polymerization reaction through heating or irradiation with active energy rays. Note that the previously described polymerizable compound (1-1) may be polymerized alone.

The organic solvent used in the polymerization reaction in method ($\alpha$) is not specifically limited so long as it is an inert organic solvent. Examples include aromatic hydrocarbons such as toluene, xylene, and mesitylene; ketones such as cyclohexanone, cyclopentanone, and methyl ethyl ketone; acetic acid esters such as butyl acetate and amyl acetate; halogenated hydrocarbons such as chloroform, dichloromethane, and dichloroethane; and ethers such as cyclopentyl methyl ether, tetrahydrofuran, and tetrahydropyran.

Of these organic solvents, those having a boiling point of 60°C to 250°C are preferable, and those having a boiling point of 60°C to 150°C are more preferable from a viewpoint of having excellent handleability.

Examples of the organic solvent in which the isolated polymer is dissolved in method ($\alpha$) and the organic solvent used in method ($\beta$) include ketone solvents such as acetone, methyl ethyl ketone, methyl isobutyl ketone, cyclopentanone,

and cyclohexanone; ester solvents such as butyl acetate and amyl acetate; halogenated hydrocarbon solvents such as dichloromethane, chloroform, and dichloroethane; ether solvents such as tetrahydrofuran, tetrahydropyran, 1,2-dimethoxyethane, 1,4-dioxane, cyclopentyl methyl ether, and 1,3-dioxolane; and polar aprotic solvents such as N,N-dimethylformamide, N,N-dimethylacetamide, dimethyl sulfoxide, γ-butyrolactone, and N-methylpyrrolidone. Of these organic solvents, those having a boiling point of 60°C to 200°C are preferable in terms of ease of handling. These solvents may be used individually or as a combination of two or more types.

The substrate used in methods (α) and (β) may be made from a commonly known and typically used organic or inorganic material. Examples of organic materials that may be used include polycycloolefin (for example, ZEONEX® and ZEONOR® (ZEONEX and ZEONOR are registered trademarks in Japan, other countries, or both; produced by ZEON Corporation), ARTON® (ARTON is a registered trademark in Japan, other countries, or both; produced by JSR Corporation), and APEL® (APEL is a registered trademark in Japan, other countries, or both; produced by Mitsui Chemicals, Inc.)), polyethylene terephthalate, polycarbonate, polyimide, polyamide, polymethyl methacrylate, polystyrene, polyvinyl chloride, polytetrafluoroethylene, cellulose, cellulose triacetate, and polyethersulfone. Examples of inorganic materials that may be used include silicon, glass, and calcite.

The substrate that is used may be a single-layer substrate or a laminate.

The substrate is preferably a substrate formed from an organic material, and is more preferably a resin film obtained by shaping an organic material into the form of a film.

Examples of substrates that may be used also include substrates that can be used in preparation of the subsequently described optically anisotropic body.

Commonly known methods can be used as the method by which the solution of the polymer is applied onto the substrate in method (α) and the method by which the solution for polymerization reaction is applied onto the substrate in method (β). Specific examples of methods that may be used include curtain coating, extrusion coating, roll coating, spin coating, dip coating, bar coating, spray coating, slide coating, print coating, gravure coating, die coating, and cap coating.

The method of drying or solvent removal in methods (α) and (β) may be natural drying, heated drying, drying under reduced pressure, heated drying under reduced pressure, or the like.

Although no specific limitations are placed on the drying temperature so long as solvent removal is possible, the lower limit for the drying temperature is preferably 50°C or higher, and more preferably 70°C or higher from a viewpoint of stably obtaining a constant temperature.

The upper limit for the drying temperature is preferably 200°C or lower, and more preferably 195°C or lower from a viewpoint of being within a range that does not negatively affect the substrate.

The method by which polymerization of the previously described polymerizable compound (1-1), the previously described polymerizable liquid crystal mixture, the previously described mixture, or the previously described polymerizable composition is carried out may, for example, be thermopolymerization or polymerization through irradiation with active energy rays. Polymerization through irradiation with active energy rays is preferable in terms that the reaction proceeds at room temperature without the need for heating. In particular, irradiation with light such as ultraviolet light is preferable due to the ease of operation.

Although no specific limitations are placed on the temperature at which irradiation with light such as ultraviolet light is performed other than being a temperature at which a liquid crystal phase can be maintained, the lower limit of the temperature is preferably 15°C or higher, and more preferably 20°C or higher from a viewpoint that photopolymerization can proceed in a stable manner.

The upper limit for the temperature at which irradiation with light such as ultraviolet light is performed is preferably 200°C or lower, and more preferably 195°C or lower from a viewpoint of being within a range that does not negatively affect the substrate.

The temperature during photoirradiation is preferably 30°C or lower. The photoirradiation intensity is normally within a range of 1 $W/m^2$ to 10 $kW/m^2$, and preferably within a range of 5 $W/m^2$ to 2 $kW/m^2$.

The polymer obtained as set forth above may be transferred from the substrate for use, may be peeled from the substrate and then used alone, or may be used as a constituent material or the like of an optical film or the like without being peeled from the substrate.

Moreover, polymer that is peeled from the substrate may be used after being pulverized by a known method to obtain a powder.

The number-average molecular weight of the presently disclosed polymer obtained as set forth above is preferably 500 to 500,000, and more preferably 5,000 to 300,000. A number-average molecular weight within any of the ranges set forth above is desirable because this provides high hardness and excellent handleability. The number-average molecular weight of the polymer can be measured by gel permeation chromatography (GPC) using monodisperse polystyrene as a standard sample and tetrahydrofuran as an eluent.

An optical film or the like having good reverse wavelength dispersion at long wavelengths can be obtained through the presently disclosed polymer.

(4) Optical film

The presently disclosed optical film includes a layer that is formed using the presently disclosed polymer and/or polymerizable compound and that has an optical function. The term "optical function" refers to simple transmission, reflection, refraction, birefringence, or the like. The presently disclosed optical film may be an optical film having the presently disclosed polymer as a main constituent material of a layer having an optical function or may be an optical film in which a layer having an optical function contains the presently disclosed polymerizable compound. In an optical film having the presently disclosed polymer as a constituent material, it is preferable that the presently disclosed polymer constitutes more than 50 mass% of a layer having an optical function when all components in the layer are taken to be 100 mass%. Moreover, an optical film containing the presently disclosed polymerizable compound preferably contains 0.01 mass% or more of the presently disclosed polymerizable compound when all components in a layer having an optical function are taken to be 100 mass%.

In terms of the form of the presently disclosed optical film, the optical film may be formed on an alignment substrate that optionally includes an alignment film (i.e., an "alignment substrate/(alignment film)/optical film" form), the optical film may be transferred onto a transparent substrate film or the like differing from an alignment substrate (i.e., a "transparent substrate film/optical film" form), or the optical film may be used as a single layer in a case in which the optical film is self-supporting (i.e., an "optical film" form).

The alignment film and the alignment substrate may the same as a substrate and an alignment film of the subsequently described optically anisotropic body.

The presently disclosed optical film can be produced by methods such as (A) applying a solution containing the presently disclosed polymerizable compound or a solution of the polymerizable liquid crystal mixture or the mixture onto an alignment substrate, drying the resultant applied film, performing heat treatment (liquid crystal alignment), and then carrying out photoirradiation and/or heating (polymerization), (B) applying a solution of a liquid crystal polymer obtained through polymerization of the presently disclosed polymerizable compound, polymerizable liquid crystal mixture, or mixture onto an alignment substrate, and optionally drying the resultant applied film, and (C) applying a solution containing the presently disclosed polymerizable compound and a resin onto an alignment substrate, and then drying the resultant applied film.

The presently disclosed optical film can be used for an optically anisotropic body, an alignment film for a liquid crystal display element, a color filter, a low-pass filter, a light polarizing prism, various light filters, and so forth.

For the presently disclosed optical film, it is preferable that the following wavelength dispersion ratios determined from retardation at wavelengths of 550 nm, 600 nm, 650 nm, 700 nm, 750 nm, and 800 nm measured using a Mueller Matrix Polarimeter AxoScan are close to their ideal values. Specifically, the ideal value of a wavelength dispersion ratio at 600 nm is 1.0909, the ideal value of a wavelength dispersion ratio at 650 nm is 1.1818, the ideal value of a wavelength dispersion ratio at 700 nm is 1.2727, the ideal value of a wavelength dispersion ratio at 750 nm is 1.3636, and the ideal value of a wavelength dispersion ratio at 800 nm is 1.4545. Moreover, the wavelength dispersion ratio at 600 nm is preferably 1.0200 or more, and more preferably 1.0275 or more, and is preferably 1.0909 or less, more preferably 1.0600 or less, and particularly preferably 1.0548 or less, the wavelength dispersion ratio at 650 nm is preferably 1.0400 or more, and more preferably 1.0408 or more, and is preferably 1.1818 or less, more preferably 1.0800 or less, and particularly preferably 1.0797 or less, the wavelength dispersion ratio at 700 nm is preferably 1.0400 or more, and more preferably 1.0495 or more, and is preferably 1.2727 or less, more preferably 1.1100 or less, and particularly preferably 1.1026 or less, the wavelength dispersion ratio at 750 nm is preferably 1.0500 or more, and more preferably 1.0552 or more, and is preferably 1.3636 or less, more preferably 1.1200 or less, and particularly preferably 1.1150 or less, and the wavelength dispersion ratio at 800 nm is preferably 1.0500 or more, and more preferably 1.0593 or more, and is preferably 1.4545 or less, more preferably 1.1300 or less, and particularly preferably 1.1217 or less.

Wavelength dispersion ratio at 600 nm = (Retardation at 600 nm)/(Retardation at 550 nm)

Wavelength dispersion ratio at 650 nm = (Retardation at 650 nm)/(Retardation at 550 nm)

Wavelength dispersion ratio at 700 nm = (Retardation at 700 nm)/(Retardation at 550 nm)

Wavelength dispersion ratio at 750 nm = (Retardation at 750 nm)/(Retardation at 550 nm)

Wavelength dispersion ratio at 800 nm = (Retardation at 800 nm)/(Retardation at 550 nm)

(5) Optically anisotropic body

The presently disclosed optically anisotropic body includes a layer having the presently disclosed polymer as a constituent material.

The presently disclosed optically anisotropic body can be obtained by, for example, forming an alignment film on a substrate and then forming a layer formed from the presently disclosed polymer (liquid crystal layer) on the alignment film. Note that the presently disclosed optically anisotropic body may be a body obtained by forming a layer formed from the presently disclosed polymer (liquid crystal layer) directly on a substrate or may be a body composed only of a layer formed from the presently disclosed polymer (liquid crystal layer).

The layer formed from the polymer may be a layer formed from a film-like polymer or may be an aggregate of a powder-like polymer.

The alignment film is formed on the surface of the substrate in order to regulate in-plane alignment of polymerizable compound in one direction.

The alignment film can be obtained by applying a solution containing a polymer such as a polyimide, polyvinyl alcohol, polyester, polyarylate, polyamide imide, or polyetherimide (composition for alignment film) onto a substrate as a film, drying the film, and then performing rubbing or the like in one direction.

The thickness of the alignment film is preferably 0.001 μm to 5 μm, and more preferably 0.001 μm to 1.0 μm.

The method by which rubbing is performed is not specifically limited and may, for example, be a method in which the alignment film is rubbed in a given direction using a roll around which cloth or felt formed from synthetic fiber (for example, nylon) or natural fiber (for example, cotton) is wound. It is preferable to wash the alignment film with isopropyl alcohol or the like after the rubbing to remove fine powder (foreign matter) formed during the rubbing and to clean the surface of the alignment film.

Besides rubbing methods, a function of regulating in-plane alignment in one direction can be imparted through a method in which the surface of an alignment film is irradiated with polarized ultraviolet rays.

The substrate on which the alignment film is formed may, for example, be a glass substrate, a substrate formed from a synthetic resin film, or the like. Examples of synthetic resins that may be used include thermoplastic resins such as acrylic resin, polycarbonate resin, polyethersulfone resin, polyethylene terephthalate resin, polyimide resin, polymethyl methacrylate resin, polysulfone resin, polyarylate resin, polyethylene resin, polystyrene resin, polyvinyl chloride resin, cellulose diacetate, cellulose triacetate, and alicyclic olefin polymers.

Examples of alicyclic olefin polymers include cycloolefin random multicomponent copolymers described in JP H05-310845 A and the Specification of US 5179171 A, hydrogenated polymers described in JP H05-97978 A and the Specification of US 5202388 A, and thermoplastic dicyclopentadiene ring-opened polymers and hydrogenated products thereof described in JP H11-124429 A (WO 99/20676 A1).

The method by which a liquid crystal layer formed from the presently disclosed polymer is formed on the alignment film may, for example, be the same as any of the methods described in the section pertaining to the presently disclosed polymer (methods (α) and (β)).

Although no specific limitations are placed on the thickness of the obtained liquid crystal layer so long as the desired retardation can be obtained, the thickness of the liquid crystal layer is normally 1 μm to 10 μm and, in the case of a λ/4 retardation plate, is preferably 1.50 μm or more, more preferably 1.80 μm or more, and particularly preferably 1.88 μm or more, and is preferably 3.00 μm or less, more preferably 2.70 μm or less, and particularly preferably 2.60 μm or less.

The presently disclosed optically anisotropic body may be a retardation plate, a viewing angle enhancement plate, or the like, but is not specifically limited to these types of optically anisotropic bodies.

For the presently disclosed optically anisotropic body, it is preferable that the following wavelength dispersion

ratios determined from retardation at wavelengths of 550 nm, 600 nm, 650 nm, 700 nm, 750 nm, and 800 nm measured using a Mueller Matrix Polarimeter AxoScan are close to their ideal values. Specifically, the ideal value of a wavelength dispersion ratio at 600 nm is 1.0909, the ideal value of a wavelength dispersion ratio at 650 nm is 1.1818, the ideal value of a wavelength dispersion ratio at 700 nm is 1.2727, the ideal value of a wavelength dispersion ratio at 750 nm is 1.3636, and the ideal value of a wavelength dispersion ratio at 800 nm is 1.4545. Moreover, the wavelength dispersion ratio at 600 nm is preferably 1.0200 or more, and more preferably 1.0275 or more, and is preferably 1.0909 or less, more preferably 1.0600 or less, and particularly preferably 1.0548 or less, the wavelength dispersion ratio at 650 nm is preferably 1.0400 or more, and more preferably 1.0408 or more, and is preferably 1.1818 or less, more preferably 1.0800 or less, and particularly preferably 1.0797 or less, the wavelength dispersion ratio at 700 nm is preferably 1.0400 or more, and more preferably 1.0495 or more, and is preferably 1.2727 or less, more preferably 1.1100 or less, and particularly preferably 1.1026 or less, the wavelength dispersion ratio at 750 nm is preferably 1.0500 or more, and more preferably 1.0552 or more, and is preferably 1.3636 or less, more preferably 1.1200 or less, and particularly preferably 1.1150 or less, and the wavelength dispersion ratio at 800 nm is preferably 1.0500 or more, and more preferably 1.0593 or more, and is preferably 1.4545 or less, more preferably 1.1300 or less, and particularly preferably 1.1217 or less.

Wavelength dispersion ratio at 600 nm = (Retardation at 600 nm)/(Retardation at 550 nm)

Wavelength dispersion ratio at 650 nm = (Retardation at 650 nm)/(Retardation at 550 nm)

Wavelength dispersion ratio at 700 nm = (Retardation at 700 nm)/(Retardation at 550 nm)

Wavelength dispersion ratio at 750 nm = (Retardation at 750 nm)/(Retardation at 550 nm)

Wavelength dispersion ratio at 800 nm = (Retardation at 800 nm)/(Retardation at 550 nm)

(6) Polarizer, etc.

The presently disclosed polarizer includes the presently disclosed optically anisotropic body and a polarizing film.

A specific example of the presently disclosed polarizer is a polarizer in which the presently disclosed optically anisotropic body is stacked on a polarizing film either directly or with another layer (for example, a glass sheet) in-between.

No specific limitations are placed on the method by which the polarizing film is produced. Examples of methods by which a PVA polarizing film can be produced include a method in which adsorption of iodine ions by a PVA film is carried out and then uniaxial stretching is performed, a method in which uniaxial stretching of a PVA film is performed and then adsorption of iodine ions is carried out, a method in which adsorption of iodine ions to a PVA film and uniaxial stretching are performed simultaneously, a method in which a PVA film is dyed using a dichroic dye and is then uniaxially stretched, a method in which a PVA film is uniaxially stretched and is then dyed using a dichroic dye, and a method in which dyeing of a PVA film using a dichroic dye and uniaxial stretching are performed simultaneously. Examples of methods by which a polyene polarizing film can be produced include commonly known methods such as a method in which a PVA film is uniaxially stretched and is then heated and dehydrated in the presence of a dehydration catalyst and a method in which a polyvinyl chloride film is uniaxially stretched and is then heated and dehydrated in the presence of a dehydrochlorination catalyst.

In the presently disclosed polarizer, the polarizing film and the presently disclosed optically anisotropic body may be in contact via an adhesive layer formed from an adhesive (inclusive of pressure-sensitive adhesives). The

average thickness of the adhesive layer is normally 0.01 μm to 30 μm, and preferably 0.1 μm to 15 μm. The adhesive layer is preferably a layer having a tensile fracture strength of 40 MPa or less according to JIS K7113.

Examples of adhesives that may form the adhesive layer include acrylic adhesives, urethane adhesives, polyester adhesives, polyvinyl alcohol adhesives, polyolefin adhesives, modified polyolefin adhesives, polyvinyl alkyl ether adhesives, rubber adhesives, vinyl chloride-vinyl acetate adhesives, styrene-butadiene-styrene copolymer (SBS copolymer) adhesives and adhesives that are hydrogenated products thereof (SEBS copolymers), ethylene adhesives such as ethylene-vinyl acetate copolymers and ethylene-styrene copolymers, and acrylic acid ester adhesives such as ethylene-methyl methacrylate copolymers, ethylene-methyl acrylate copolymers, ethylene-ethyl methacrylate copolymers, and ethylene-ethyl acrylate copolymers.

The presently disclosed polarizer has good reverse wavelength dispersion at long wavelengths as a result of the presently disclosed optically anisotropic body being used therein.

Moreover, an antireflection film and a display including a panel can suitably be produced by using the presently disclosed polarizer. The panel may, for example, be a liquid crystal panel or an organic electroluminescence panel. The display may, for example, be a flat panel display including a polarizer and a liquid crystal panel or an organic electroluminescence display including a liquid crystal panel and an organic electroluminescence panel.

(7) Compound

The presently disclosed compound is useful as a production intermediate of the previously described polymerizable compound (1-1). One example of the compound is a compound indicated by the following formula (VIII-1). Hereinafter, the compound indicated by formula (VIII-1) is also referred to as "compound (VIII-1)".

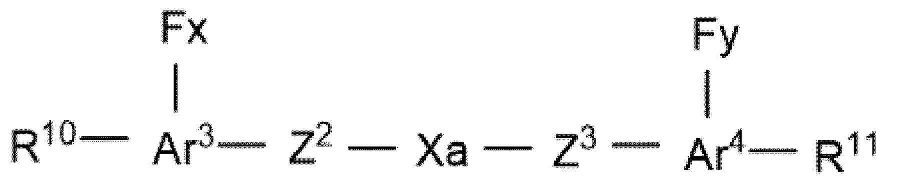

(VIII-1)

In formula (VIII-1), Xa, $Z^2$, and $Z^3$ have the same meaning as previously described and preferable examples thereof are also the same as previously described.

Fx and Fy each represent, independently of one another, $-C(R^f)=N-N(R^g)R^h$, $-C(R^f)=N-N=C(R^{g1})R^h$, or -CHO, where $R^f$ represents a hydrogen atom or an alkyl group having a carbon number of 1 to 6, $R^g$ and $R^{g1}$ each represent, independently of one another, a hydrogen atom or an optionally substituted organic group having a carbon number of 1 to 30, and $R^h$ represents an organic group including at least one aromatic ring selected from the group consisting of an aromatic hydrocarbon ring having a carbon number of 6 to 30 and an aromatic heterocyclic ring having a carbon number of 2 to 30.

In formula (VIII-1), $Ar^3$ and $Ar^4$ each represent, independently of one another, an aromatic hydrocarbon cyclic group or an aromatic heterocyclic group. The aromatic hydrocarbon cyclic group and the aromatic heterocyclic group of $Ar^3$ and $Ar^4$ have the same meaning as the aromatic hydrocarbon cyclic group and the aromatic heterocyclic group of $Ar^0$ and $Ar^1$.

In formula (VIII-1), $Ar^3$-Fx and $Ar^4$-Fy are preferably each, independently of one another, indicated by any one of the following formulae (IX-1) to (IX-14).

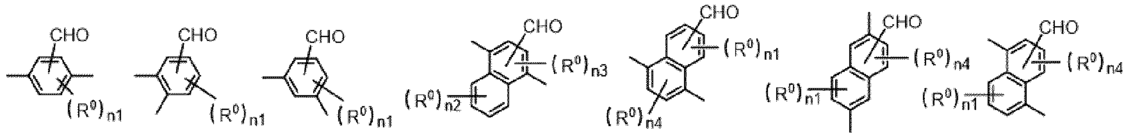

(IX-1) (IX-2) (IX-3) (IX-4) (IX-5) (IX-6) (IX-7)

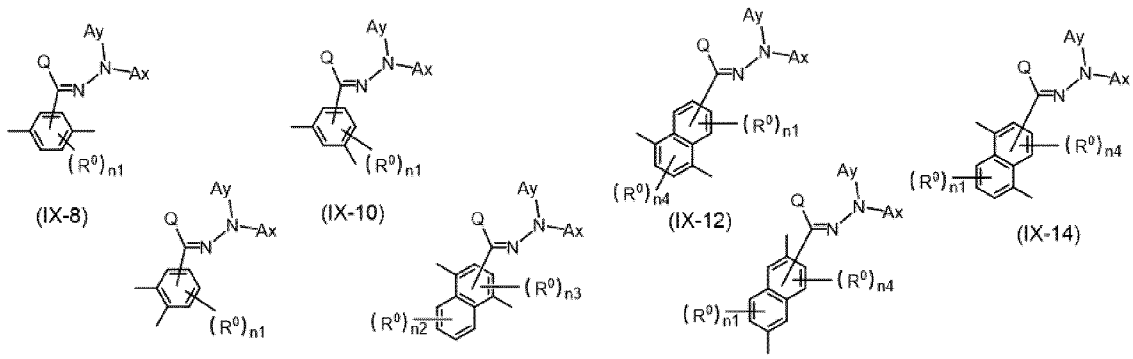

(IX-8) (IX-10) (IX-12) (IX-14)

(IX-9) (IX-11) (IX-13)

In formulae (IX-1) and (IX-14), Ax, Ay, Q, $R^0$, n1, n2, n3, and n4 have the same meaning as previously described.

In a case in which more than one $R^0$ is present, each $R^0$ may be the same or different.

In formula (VIII-1), $R^{10}$ and $R^{11}$ each represent, independently of one another, $-OR^p$, $-CH_2OR^p$, $-CH_2CH_2OR^p$, $-C(=O)-OR^p$, $-CH_2-C(=O)-OR^p$, $-CH_2CH_2-C(=O)-OR^p$, a hydroxy group, a carboxyl group, $-CH_2-C(=O)-OH$, $-CH_2CH_2-C(=O)-OH$, $-CH_2OH$, $-CH_2CH_2OH$, or an amino group.

Of these examples, $R^{10}$ and $R^{11}$ are each, independently of one another, preferably $-OR^p$, $-CH_2OR^p$, $-C(=O)-OR^p$, $-CH_2-C(=O)-OR^p$, a hydroxy group, a carboxyl group, $-CH_2-C(=O)-OH$, $-CH_2OH$, or $-CH_2CH_2OH$, and particularly preferably $-OR^p$, $-CH_2-C(=O)-OR^p$, a hydroxy group, $-CH_2OH$, a carboxyl group, or $-CH_2-C(=O)-OH$.

Here, $R^p$ represents a protecting group.

Examples of the protecting group $R^p$ in formula (VIII-1) include, but are not specifically limited to, protecting groups of a hydroxy group or a carboxyl group such as a tetrahydropyranyl group, a methoxymethyl group, a 2-methoxyethoxymethyl group, a tert-butyldimethylsilyl group, a trimethylsilyl group, and a benzyl group. Of these protecting groups, a tetrahydropyranyl group, a 2-methoxyethoxymethyl group, or a tert-butyldimethylsilyl group is preferable.

The compound represented by formula (VIII-1) can be synthesized through a combination of known synthetic reactions. Specifically, the compound can be synthesized with reference to methods described in various documents (for example, March's Advanced Organic Chemistry (Wiley); Sandler and Karo, "Syntheses of Organic Compounds Classified by Functional Group", joint translation by Naoki INAMOTO (Hirokawa Publishing Company); and Greene's Protective Groups in Organic Synthesis, Fourth Edition (Wiley)).

The compound (VIII-1) is preferably a compound represented by any one of the following formulae (X-1) to (X-12). Hereinafter, a compound indicated by formula (X-1) is also referred to as "compound (X-1)", a compound indicated by formula (X-2) is also referred to as "compound (X-2)", a compound indicated by formula (X-3) is also referred to as "compound (X-3)", a compound indicated by formula (X-4) is also referred to as "compound (X-4)", a compound indicated by formula (X-5) is also referred to as "compound (X-5)", a compound indicated by formula (X-6) is also referred to as "compound (X-6)", a compound indicated by formula (X-7) is also referred to as "compound (X-7)", a compound indicated by formula (X-8) is also referred to as "compound (X-8)", a compound indicated by formula (X-9) is also referred to as "compound (X-9)", a compound indicated by formula (X-10) is also referred to as "compound (X-10)", a compound indicated by formula (X-11) is also referred to as "compound (X-11)", and a compound indicated by formula (X-12) is also referred to as "compound (X-12)",

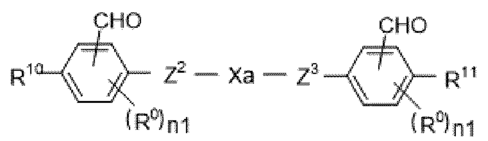

(X-1)

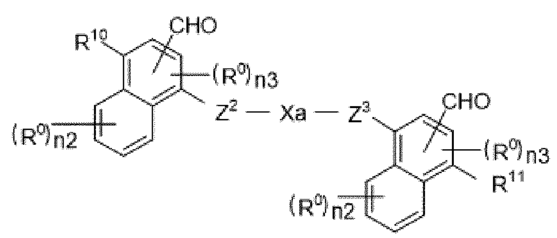

(X-2)

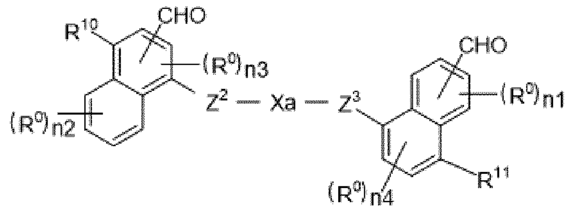
(X-3)

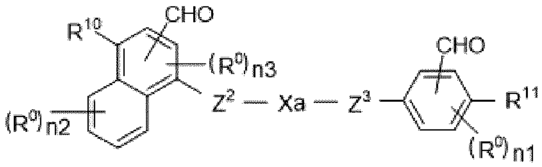
(X-4)

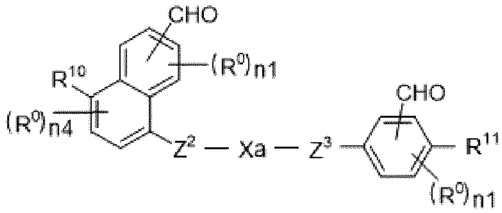
(X-5)

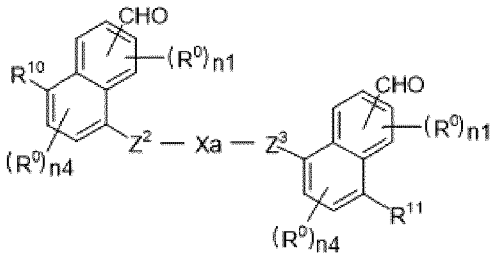
(X-6)

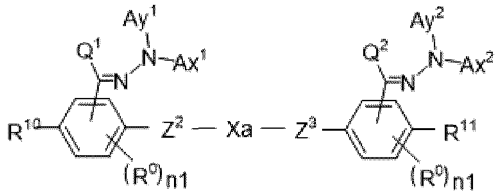
(X-7)

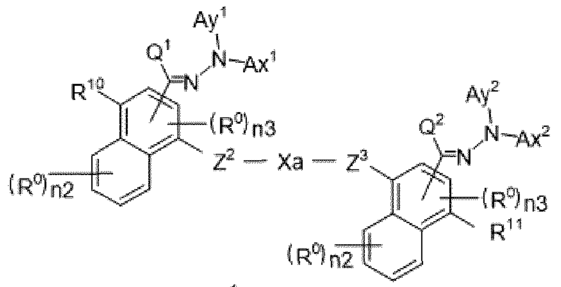
(X-8)

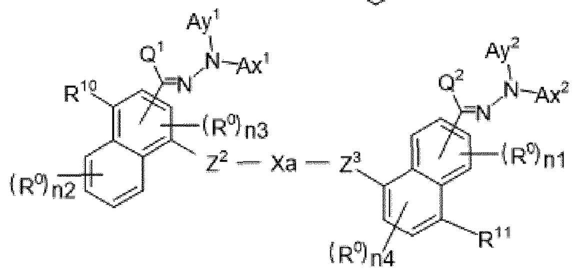
(X-9)

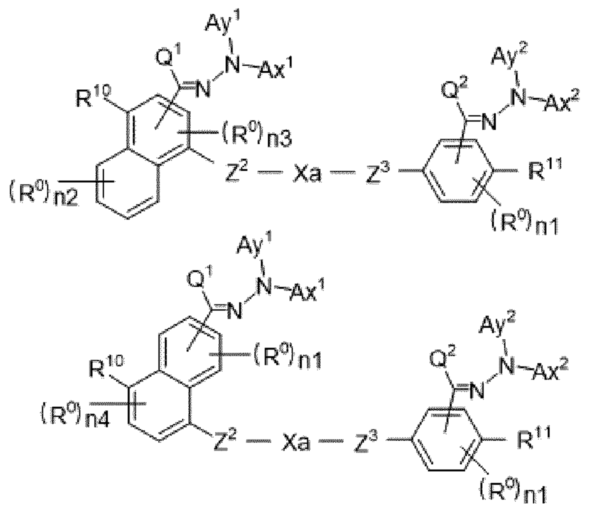
(X-10)

(X-11)

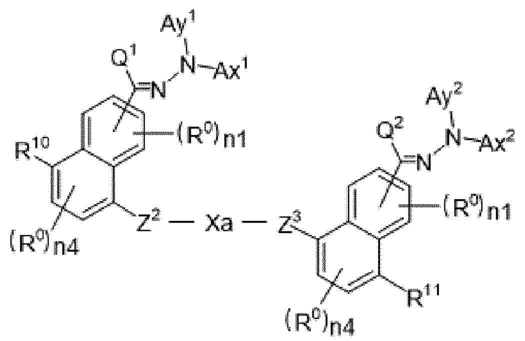
(X-12)

In formulae (X-1) to (X-12), Xa, $Z^2$, $Z^3$, $R^{10}$, $R^{11}$, $R^0$, n1, n2, n3, and n4 have the same meaning as previously described and preferable examples thereof are also the same as previously described.

$Ax^1$ and $Ax^2$ each represent, independently of one another, an organic group including at least one aromatic ring selected from the group consisting of an aromatic hydrocarbon ring having a carbon number of 6 to 30 and an aromatic heterocyclic ring having a carbon number of 2 to 30, where the aromatic rings of $Ax^1$ and $Ax^2$ are optionally substituted.

$Ay^1$ and $Ay^2$ each represent, independently of one another, a hydrogen atom or an optionally substituted organic group having a carbon number of 1 to 30.

$Q^1$ and $Q^2$ each represent, independently of one another, a hydrogen atom or an alkyl group having a carbon

number of 1 to 6.

Note that n1, n2, n3, and n4 on the left and right sides of Xa may be the same or different, but are preferably 0.

In a case in which more than one $R^0$ is present, each $R^0$ may be the same or different.

The compounds represented by formulae (X-1) to (X-12) can be synthesized through a combination of known synthetic reactions. Specifically, the compounds can be synthesized with reference to methods described in various documents (for example, March's Advanced Organic Chemistry (Wiley); Sandler and Karo, "Syntheses of Organic Compounds Classified by Functional Group", joint translation by Naoki INAMOTO (Hirokawa Publishing Company); and Greene's Protective Groups in Organic Synthesis, Fourth Edition (Wiley)).

Another example of the presently disclosed compound is a compound represented by any one of the following formulae (XI-1) to (XI-6). Hereinafter, a compound indicated by formula (XI-1) is also referred to as "compound (XI-1)", a compound indicated by formula (XI-2) is also referred to as "compound (XI-2)", a compound indicated by formula (XI-3) is also referred to as "compound (XI-3)", a compound indicated by formula (XI-4) is also referred to as "compound (XI-4)", a compound indicated by formula (XI-5) is also referred to as "compound (XI-5)", and a compound indicated by formula (XI-6) is also referred to as "compound (XI-6)".

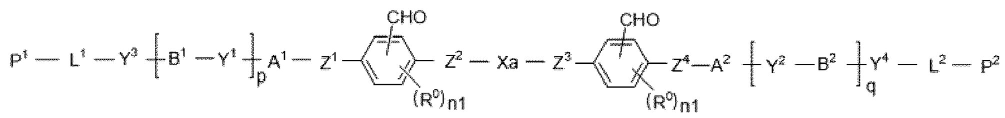
(XI-1)

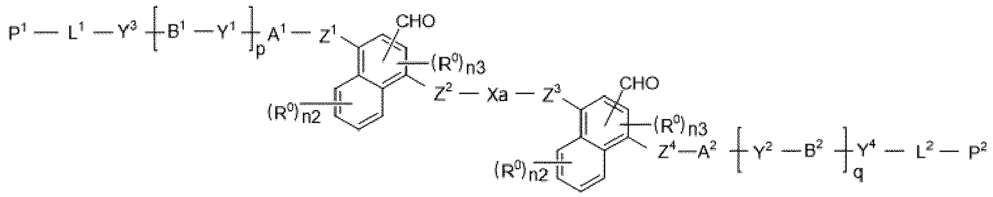
(XI-2)

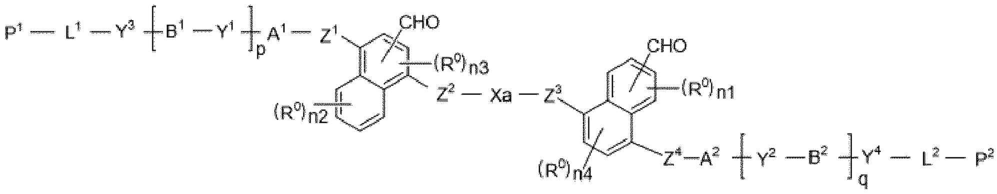
(XI-3)

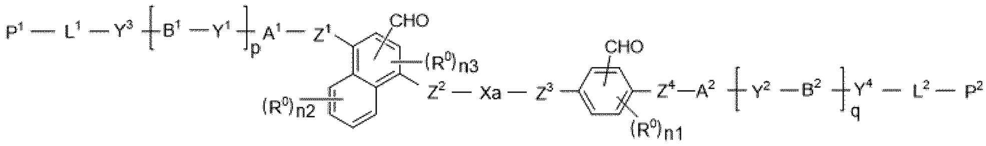
(XI-4)

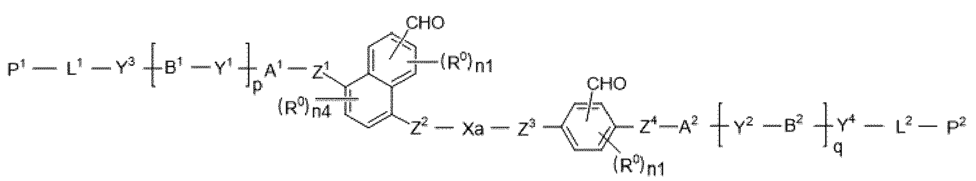
(XI-5)

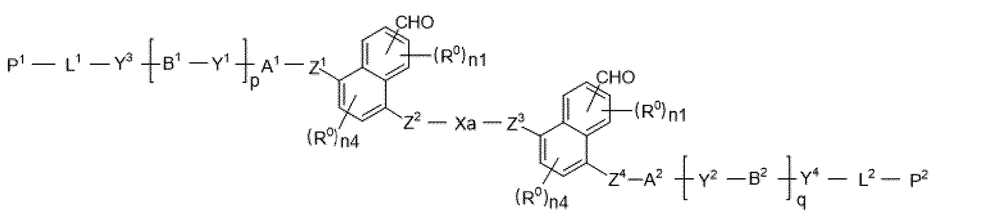
(XI-6)

In formulae (XI-1) to (XI-6), Xa, $Z^1$ to $Z^4$, $A^1$, $A^2$, $B^1$, $B^2$, $Y^1$ to $Y^4$, $L^1$, $L^2$, $P^1$, $P^2$, p, q, $R^0$, n1, n2, n3, and n4 have the same meaning as previously described and preferable examples thereof are also the same as previously described.

In a case in which more than one $R^0$, $B^1$, $B^2$, $Y^1$, or $Y^2$ is present, each $R^0$, $B^1$, $B^2$, $Y^1$, or $Y^2$ may be the same or different.

The compounds represented by formulae (XI-1) to (XI-6) can be synthesized through a combination of known synthetic reactions using the previously described compounds represented by formulae (X-1) to (X-6) as materials. Specifically, the compounds can be synthesized with reference to methods described in various documents (for example, March's Advanced Organic Chemistry (Wiley); Sandler and Karo, "Syntheses of Organic Compounds Classified by Functional Group", joint translation by Naoki INAMOTO (Hirokawa Publishing Company); and Greene's Protective Groups in Organic Synthesis, Fourth Edition (Wiley)).

Among compounds represented by formula (XI-1), a compound represented by the following formula (XII-1) is preferable. Moreover, among compounds represented by formulae (XI-2), (XI-3), and (XI-6), a compound represented by the following formula (XII-2) is preferable. Furthermore, among compounds represented by formulae (XI-4) and (XI-5), a compound represented by the following formula (XII-3) is preferable.

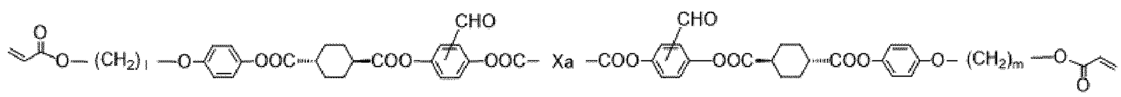

(XII-1)

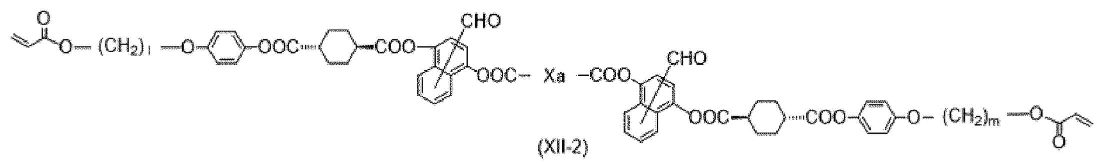

(XII-2)

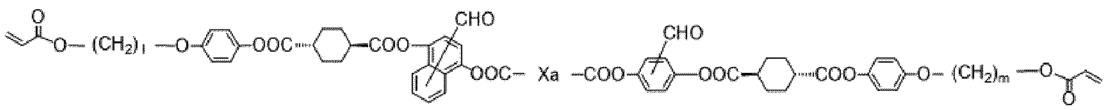

(XII-3)

In formulae (XII-1) to (XII-3), Xa has the same meaning as previously described and preferable examples thereof are also the same as previously described. Moreover, 1 and m each represent, independently of one another, an integer of 1 to 18.

The compounds represented by formulae (XII-1) to (XII-3) can be synthesized through a combination of known synthetic reactions. Specifically, the compounds can be synthesized with reference to methods described in various documents (for example, March's Advanced Organic Chemistry (Wiley); Sandler and Karo, "Syntheses of Organic Compounds Classified by Functional Group", joint translation by Naoki INAMOTO (Hirokawa Publishing Company); and Greene's Protective Groups in Organic Synthesis, Fourth Edition (Wiley)).

The previously described polymerizable compound (1-1) can be obtained from the compounds represented by formulae (XII-1) to (XII-3) by adopting a known synthetic reaction with respect to the CHO parts in formulae (XII-1) to (XII-3).

EXAMPLES

The following provides a more detailed description of the present disclosure through examples. However, the present disclosure is not in any way limited by the following examples.

(Synthesis Example 1: Synthesis of compound 1 (one example of compound represented by formula (VI-1)))

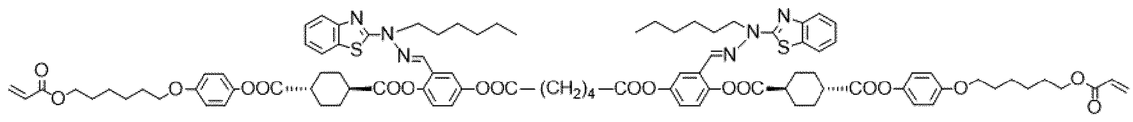

Compound 1

<Step 1: Synthesis of intermediate A>

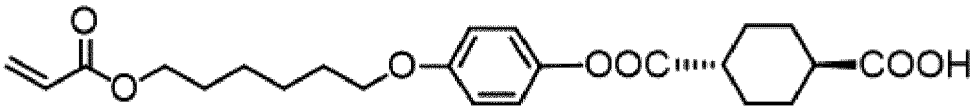

Intermediate A

A three-necked reaction vessel equipped with a thermometer was charged with 17.98 g (104.42 mmol) of trans-1,4-cyclohexanedicarboxylic acid and 180 mL of tetrahydrofuran (THF) in a stream of nitrogen. In addition, 6.58 g (57.43 mmol) of methanesulfonyl chloride was added into the reaction vessel and the reaction vessel was immersed in a water bath to attain a reaction liquid internal temperature of 20°C. Next, 6.34 g (62.65 mmol) of triethylamine was added dropwise over 10 minutes while maintaining the reaction liquid internal temperature at 20°C to 30°C. After completion of the dropwise addition, the entire contents of the reaction vessel were further stirred for 2 hours at 25°C.

Next, 0.64 g (5.22 mmol) of 4-(dimethylamino)pyridine and 13.80 g (52.21 mmol) of 4-(6-acryloyloxy-hex-1-yloxy)phenol (produced by DKSH) were added to the resultant reaction liquid, and the reaction vessel was immersed in a water bath once again to attain a reaction liquid internal temperature of 15°C. Thereafter, 6.34 g (62.65 mmol) of triethylamine was added dropwise over 10 minutes while maintaining the reaction liquid internal temperature at 20°C to 30°C. After completion of the dropwise addition, the entire contents of the reaction vessel were further stirred for 2 hours at 25°C. Once the reaction ended, 1,000 mL of distilled water and 100 mL of saturated saline water were added to the reaction liquid, and two extractions were performed with 400 mL of ethyl acetate. The organic layers were collected and were dried with anhydrous sodium sulfate, and then sodium sulfate was filtered off. Solvent was removed from the filtrate by evaporation in a rotary evaporator and then the resultant residue was purified by silica gel column chromatography (THF:toluene = 1:9 (volume ratio)) to yield 14.11 g of intermediate A in the form of a white solid. The yield was 65 mol%. The structure of the intermediate A was identified by $^1$H-NMR. The $^1$H-NMR spectrum data are shown below.

$^1$H-NMR (500 MHz, DMSO-$d_6$, TMS, $\delta$ ppm): 12.12 (s, 1H), 6.99 (d, 2H, J = 9.0 Hz), 6.92 (d, 2H, J = 9.0 Hz), 6.32 (dd, 1H, J = 1.5 Hz, 17.5 Hz), 6.17 (dd, 1H, J = 10.0 Hz, 17.5 Hz), 5.93 (dd, 1H, J = 1.5 Hz, 10.0 Hz), 4.11 (t, 2H, J = 6.5 Hz), 3.94 (t, 2H, J = 6.5 Hz), 2.48-2.56 (m, 1H), 2.18-2.26 (m, 1H), 2.04-2.10 (m, 2H), 1.93-2.00 (m, 2H), 1.59-1.75 (m, 4H), 1.35-1.52 (m, 8H)

<Step 2: Synthesis of intermediate B (one example of compound represented by formula (X-1))>

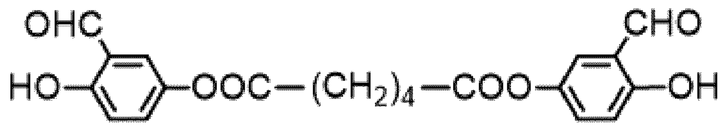

Intermediate B

A three-necked reaction vessel equipped with a thermometer was charged with 10 g (68.4 mmol) of adipic acid, 18.9 g (136.9 mmol) of 2,5-dihydroxybenzaldehyde, 836 mg (6.84 mmol) of N,N-dimethylaminopyridine, and 250 mL of chloroform in a stream of nitrogen. In addition, 20.7 g (164.3 mmol) of N,N'-diisopropylcarbodiimide was added into the reaction vessel. Thereafter, the reaction vessel was stirred for 20 hours at 25°C. Once the reaction ended, 500 mL of distilled water and 100 mL of saturated saline water were added to the resultant reaction liquid, and two extractions were

performed with 300 mL of ethyl acetate. The organic layers were collected and were dried with anhydrous sodium sulfate, and then sodium sulfate was filtered off. Solvent was removed in a rotary evaporator and then the resultant residue was purified by silica gel column chromatography (toluene:ethyl acetate = 90:10 (volume ratio)) to yield 18 g of intermediate B in the form of a pale yellow solid. The yield was 68.1 mol%. The structure of the intermediate B was identified by $^1$H-NMR. The $^1$H-NMR spectrum data are shown below.

Note that the intermediate B is one example of a compound represented by formula (X-1) from among compounds represented by formula (VIII-1).

$^1$H-NMR (500 MHz, DMSO-$d_6$, TMS, $\delta$ ppm): 10.84 (s, 2H), 10.25 (s, 2H), 7.35 (d, 2H, J = 3.0 Hz), 7.29 (dd, 2H, J = 3.0 Hz, 9.0 Hz), 7.02 (d, 2H, J = 9.0 Hz), 2.65-2.60 (m, 4H), 1.75-1.69 (m, 4H)

<Step 3: Synthesis of intermediate C (one example of compound represented by formula (XII-1))>

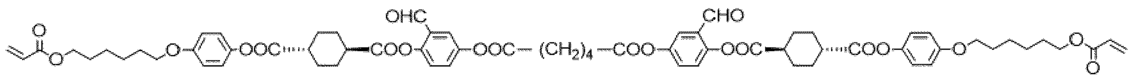

Intermediate C

In a three-necked reaction vessel equipped with a thermometer, 5.0 g (12.9 mmol) of the intermediate B synthesized in step 2, 13.5 g (32.3 mmol) of the intermediate A synthesized in step 1, and 15.9 mg (0.13 mmol) of N,N-dimethylaminopyridine were added to 200 mL of N-methylpyrrolidone in a stream of nitrogen. The reaction vessel was stirred while 7.4 g (38.8 mmol) of 1-ethyl-3-(3-dimethylaminopropyl)carbodiimide hydrochloride was slowly added at 25°C. Thereafter, the reaction vessel was stirred for 15 hours at 25°C to carry out a reaction. Once the reaction ended, 1 L of distilled water and 100 mL of saturated saline water were added to the resultant reaction liquid, and two extractions were performed with 250 mL of chloroform. The resultant organic layers were dried with anhydrous sodium sulfate, and then sodium sulfate was filtered off. A rotary evaporator was used to remove 250 mL of solvent and then the resultant organic layer was slowly added dropwise to 2 L of methanol. Precipitated solid was filtered and collected. The obtained solid was vacuum dried to yield 9.2 g of intermediate C in the form of a white solid. The yield was 60.8 mol%. The structure of the intermediate C was identified by $^1$H-NMR. The $^1$H-NMR spectrum data are shown below.

Note that the intermediate C is one example of a compound represented by formula (XII-1) from among compounds represented by formula (XI-1).

$^1$H-NMR (500 MHz, $CDCl_3$, TMS, $\delta$ ppm): 10.07 (s, 2H), 7.63 (d, 2H, J = 3.0 Hz), 7.38 (dd, 2H, J = 3.0 Hz, 9.0 Hz), 7.20 (d, 2H, J = 9.0 Hz), 7.00 (d, 4H, J = 9.0 Hz), 6.88 (d, 4H, J = 9.0 Hz), 6.40 (dd, 2H, J = 1.5 Hz, 17.5 Hz), 6.12 (dd, 2H, J = 10.5 Hz, 17.5 Hz), 5.82 (dd, 2H, J = 1.5 Hz, 10.5 Hz), 4.17 (t, 4H, J = 6.5 Hz), 3.94 (t, 4H, J = 6.5 Hz), 2.74-2.55 (m, 8H), 2.39-2.27 (m, 8H), 1.94-1.85 (m, 4H), 1.83-1.63 (m, 14H), 1.54-1.41 (m, 8H)

<Step 4: Synthesis of intermediate D>

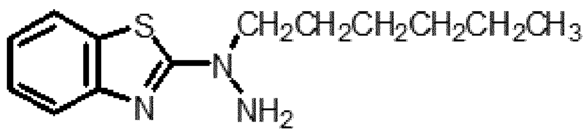

Intermediate D

In a four-necked reaction vessel equipped with a thermometer, 2.00 g (12.1 mmol) of 2-hydrazinobenzothiazole was dissolved in 20 mL of dimethylformamide in a stream of nitrogen. In addition, 8.36 g (60.5 mmol) of potassium carbonate and 3.08 g (14.5 mmol) of 1-iodohexane were added to the solution and were stirred therewith for 7 hours at 50°C. Once the reaction ended, the reaction liquid was cooled to 20°C and was added into 200 mL of water. An extraction was performed with 300 mL of ethyl acetate. The ethyl acetate layer was dried with anhydrous sodium sulfate. Sodium sulfate was filtered off and then ethyl acetate was evaporated under reduced pressure in a rotary evaporator to yield a yellow solid. The yellow solid was purified by silica gel column chromatography (hexane:ethyl acetate = 75:25 (volume ratio)) to yield 2.10 g of intermediate E in the form of a white solid. The yield was 69.6 mol%. The structure of the intermediate D was identified by $^1$H-NMR. The $^1$H-NMR spectrum data are shown below.

$^1$H-NMR (500 MHz, $CDCl_3$, TMS, δ ppm): 7.60 (dd, 1H, J = 1.0, 8.0 Hz), 7.53 (dd, 1H, J = 1.0, 8.0 Hz), 7.27 (ddd, 1H, J = 1.0, 8.0, 8.0 Hz), 7.06 (ddd, 1H, J = 1.0, 8.0, 8.0 Hz), 4.22 (s, 2H), 3.74 (t, 2H, J = 7.5 Hz), 1.69-1.76 (m, 2H), 1.29-1.42 (m, 6H), 0.89 (t, 3H, J = 7.0 Hz)

<Step 5: Synthesis of compound 1 (one example of compound represented by formula (VI-1))>

In a four-necked reaction vessel equipped with a thermometer, 3.0 g (2.56 mmol) of the intermediate C synthesized in step 3, 1.66 g (6.66 mmol) of the intermediate D synthesized in step 4, and 119 mg (0.51 mmol) of (±)-10-camphorsulfonic acid were added to a mixed solution of 100 mL of tetrahydrofuran and 10 mL of ethanol in a stream of nitrogen. The solution was then stirred for 2 hours at 50°C. Once the reaction ended, the reaction liquid was cooled and was added into 500 mL of 10 mass% sodium bicarbonate water. Two extractions were then performed with 300 mL of ethyl acetate. The organic layers were collected and were dried with anhydrous sodium sulfate, and then sodium sulfate was filtered off. Solvent was removed in a rotary evaporator and then the resultant residue was purified by silica gel column chromatography (toluene:ethyl acetate = 85:15 (volume ratio)) to yield 3.22 g of compound 1 in the form of a pale yellow solid. The yield was 75.6 mol%. The structure of the target (compound 1) was identified by $^1$H-NMR. The $^1$H-NMR spectrum data are shown below.

$^1$H-NMR (500 MHz, $CDCl_3$, TMS, δ ppm): 7.76-7.75 (m, 2H), 7.64 (d, 2H, J = 8.0 Hz), 7.53 (dd, 2H, J = 0.5 Hz, 8.0 Hz), 7.48 (s, 2H), 7.31 (dd, 1H, J = 1.0 Hz, 8.0 Hz), 7.30 (dd, 1H, J = 1.0 Hz, 8.0 Hz), 7.13 (m, 6H), 6.98 (d, 4H, J = 9.0 Hz), 6.88 (d, 4H, J = 9.0 Hz), 6.40 (dd, 2H, J = 1.5 Hz, 17.5 Hz), 6.13 (dd, 2H, J = 10.5 Hz, 17.5 Hz), 5.82 (dd, 2H, J = 1.5 Hz, 10.5 Hz), 4.18 (t, 4H, J = 6.5 Hz), 4.14 (t, 4H, J = 7.5 Hz), 3.95 (t, 4H, J = 6.5 Hz), 2.78-2.55 (m, 8H), 2.38-2.25 (m, 8H), 2.02-1.95 (m, 4H), 1.85-1.30 (m, 40H), 0.95 (t, 6H, J = 7.0 Hz)

(Synthesis Example 2: Synthesis of compound 2 (another example of compound represented by formula (VI-1)))

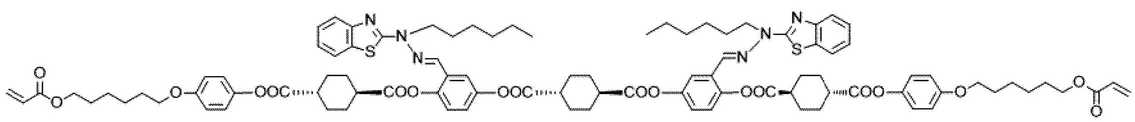

Compound 2

<Step 1: Synthesis of intermediate E (another example of compound represented by formula (X-1))>

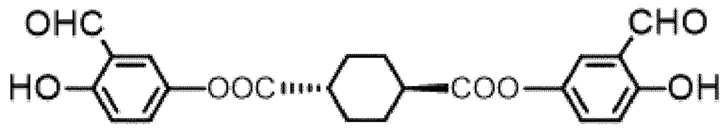

Intermediate E

In a three-necked reaction vessel equipped with a thermometer, 10 g (58.1 mmol) of trans-1,4-cyclohexanedicarboxylic acid, 16.0 g (116 mmol) of 2,5-dihydroxybenzaldehyde, and 710 mg (5.8 mmol) of N,N-dimethylaminopyridine were added to 350 mL of chloroform in a stream of nitrogen. In addition, 17.6 g (139 mmol) of N,N'-diisopropylcarbodiimide was slowly added dropwise at 15°C under vigorous stirring. Thereafter, the reaction vessel was stirred for 6 hours at 25°C to carry out a reaction. Once the reaction ended, a precipitate was filtered and removed using a Kiriyama funnel in which Celite had been spread. The resultant reaction liquid was washed with 300 mL of 0.1 N hydrochloric acid aqueous solution. In addition, 200 mL of saturated saline water was added to the organic layer to perform washing. The resultant organic layer was dried with anhydrous sodium sulfate, and then sodium sulfate was filtered off. Solvent was removed in a rotary evaporator and then the resultant residue was purified by silica gel column chromatography (toluene:ethyl acetate = 90:10 (volume ratio)) to yield 18 g of intermediate E in the form of a pale yellow solid. The yield was 75.1 mol%. The structure of the intermediate E was identified by $^1$H-NMR. The $^1$H-NMR spectrum data are shown below.

Note that the intermediate E is another example of a compound represented by formula (X-1) from among compounds represented by formula (VIII-1).

$^1$H-NMR (500 MHz, DMSO-$d_6$, TMS, δ ppm): 10.78 (s, 2H), 10.26 (s, 2H), 7.34 (d, 2H, J = 3.0 Hz), 7.29 (dd,

2H, J = 3.0 Hz, 9.0 Hz), 7.03 (d, 2H, J = 9.0 Hz), 2.65-2.58 (m, 2H), 2.18-2.12 (m, 4H), 1.62-1.52 (m, 4H)

<Step 2: Synthesis of compound 2 (another example of compound represented by formula (VI-1))>

In a four-necked reaction vessel equipped with a thermometer, 3.0 g (7.27 mmol) of the intermediate E synthesized in step 1, 7.6 g (18.19 mmol) of intermediate A synthesized in the same way as in step 1 of Synthesis Example 1, and 444 mg (3.64 mmol) of N,N-dimethylaminopyridine were added to 200 mL of chloroform in a stream of nitrogen. Dropwise addition of 2.75 g (21.8 mmol) of N,N'-diisopropylcarbodiimide to the solution was performed slowly at 25°C. The solution was then stirred for 6 hours at 25°C. Once the reaction ended, the reaction liquid was cooled in an ice bath, and then 4.35 g (17.4 mmol) of intermediate D synthesized in the same way as in step 4 of Synthesis Example 1 and 14.4 mL (14.4 mmol) of 1 N hydrochloric acid aqueous solution were added thereto. This solution was allowed to react for 3 hours at 40°C. Once the reaction ended, the solution was added into 500 mL of 3 mass% sodium bicarbonate water, and two extractions were performed with 500 mL of ethyl acetate. The organic layers were collected and were dried with anhydrous sodium sulfate, and then sodium sulfate was filtered off. Solvent was removed in a rotary evaporator and then the resultant residue was purified by silica gel column chromatography (toluene:ethyl acetate = 90:10 (volume ratio)) to yield 5.5 g of compound 2 in the form of a pale yellow solid. The yield was 45.1 mol%. The structure of the target (compound 2) was identified by $^1$H-NMR. The $^1$H-NMR spectrum data are shown below.

$^1$H-NMR (500 MHz, $CDCl_3$, TMS, $\delta$ ppm): 7.76 (dd, 2H, J = 1.0 Hz, 2.0 Hz), 7.70-7.67 (m, 6H), 7.35 (dd, 1H, J = 1.0 Hz, 8.0 Hz), 7.34 (dd, 1H, J = 1.0 Hz, 8.0 Hz), 7.18-7.12 (m, 6H), 6.98 (d, 4H, J = 9.0 Hz), 6.88 (d, 4H, J = 9.0 Hz), 6.40 (dd, 2H, J = 1.5 Hz, 17.5 Hz), 6.13 (dd, 2H, J = 10.5 Hz, 17.5 Hz), 5.82 (dd, 2H, J = 1.5 Hz, 10.5 Hz), 4.30 (t, 4H, J = 7.5 Hz), 4.18 (t, 4H, J = 6.5 Hz), 3.95 (t, 4H, J = 6.5 Hz), 2.75-2.56 (m, 6H), 2.44-2.27 (m, 12H), 1.91-1.31 (m, 44H), 0.91 (t, 6H, J = 7.0 Hz)

(Synthesis Example 3: Synthesis of compound 3 (another example of compound represented by formula (VI-1)))

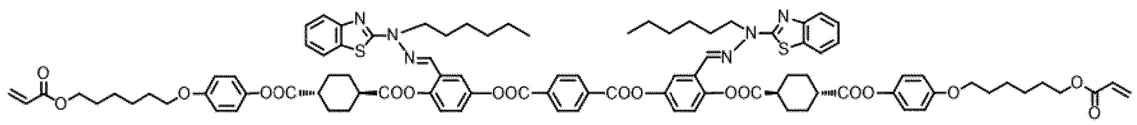

Compound 3

<Step 1: Synthesis of intermediate F (another example of compound represented by formula (X-1))>

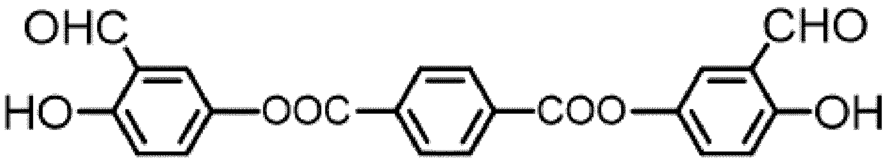

Intermediate F

In a three-necked reaction vessel equipped with a thermometer, 10 g (60.2 mmol) of terephthalic acid, 16.6 g (120 mmol) of 2,5-dihydroxybenzaldehyde, and 735 mg (6.0 mmol) of N,N-dimethylaminopyridine were added to 300 mL of chloroform in a stream of nitrogen. In addition, 18.2 g (144.5 mmol) of N,N'-diisopropylcarbodiimide was slowly added dropwise at 15°C under vigorous stirring. Thereafter, the reaction vessel was stirred for 12 hours at 25°C to carry out a reaction. Once the reaction ended, a precipitate was filtered and removed using a Kiriyama funnel in which Celite had been spread. The resultant reaction liquid was washed with 100 mL of 0.1 N hydrochloric acid aqueous solution. In addition, 100 mL of saturated saline water was added to the organic layer to perform washing. The resultant organic layer was dried with anhydrous sodium sulfate, and then sodium sulfate was filtered off. Solvent was removed in a rotary evaporator and then the resultant residue was purified by silica gel column chromatography (toluene:ethyl acetate = 85:15 (volume ratio)) to yield 12.3 g of intermediate F in the form of a pale yellow solid. The yield was 50.3 mol%. The structure of the intermediate F was identified by $^1$H-NMR. The $^1$H-NMR spectrum data are shown below.

Note that the intermediate F is another example of a compound represented by formula (X-1) from among compounds represented by formula (VIII-1).

$^1$H-NMR (500 MHz, DMSO-$d_6$, TMS, $\delta$ ppm): 10.88 (s, 2H), 10.30 (s, 2H), 8.31 (s, 4H), 7.58 (d, 2H, J = 3.0 Hz), 7.52 (dd, 2H, J = 3.0 Hz, 9.0 Hz), 7.10 (d, 2H, J = 9.0 Hz)

<Step 2: Synthesis of compound 3 (another example of compound represented by formula (VI-1))>

In a four-necked reaction vessel equipped with a thermometer, 4.0 g (9.84 mmol) of the intermediate F synthesized in step 1, 10.3 g (24.6 mmol) of intermediate A synthesized in the same way as in step 1 of Synthesis Example 1, and 601 mg (4.92 mmol) of N,N-dimethylaminopyridine were added to 400 mL of chloroform in a stream of nitrogen. Dropwise addition of 3.73 g (29.5 mmol) of N,N'-diisopropylcarbodiimide to the solution was performed slowly at 25°C. The solution was then stirred for 12 hours at 25°C. Once the reaction ended, the reaction liquid was cooled in an ice bath, and then 5.89 g (23.6 mmol) of intermediate D synthesized in the same way as in step 4 of Synthesis Example 1 and 19.5 mL (19.5 mmol) of 1 N hydrochloric acid aqueous solution were added thereto. This solution was allowed to react for 3 hours at 40°C. Once the reaction ended, the reaction liquid was added into 500 mL of 3 mass% sodium bicarbonate water to perform washing. The organic layer was collected and was dried with anhydrous sodium sulfate, and then sodium sulfate was filtered off. Solvent was removed in a rotary evaporator and then the resultant residue was purified by silica gel column chromatography (toluene:ethyl acetate = 85:15 (volume ratio)) to yield 3.8 g of compound 3 in the form of a pale yellow solid. The yield was 23.1 mol%. The structure of the target (compound 3) was identified by $^1$H-NMR. The $^1$H-NMR spectrum data are shown below.

$^1$H-NMR (500 MHz, $CDCl_3$, TMS, $\delta$ ppm): 8.43 (s, 4H), 7.95 (d, 2H, J = 3.0 Hz), 7.73 (s, 2H), 7.68-7.64 (m, 4H), 7.34 (dd, 1H, J = 1.0 Hz, 8.0 Hz), 7.33 (dd, 1H, J = 1.0 Hz, 8.0 Hz), 7.29 (dd, 2H, J = 3.0 Hz, 8.0 Hz), 7.21 (d, 2H, J = 8.0 Hz), 7.16 (dd, 1H, J = 1.0 Hz, 8.0 Hz), 7.14 (dd, 1H, J = 1.0 Hz, 8.0 Hz), 6.99 (d, 4H, J = 9.0 Hz), 6.89 (d, 4H, J = 9.0 Hz), 6.41 (dd, 2H, J = 1.5 Hz, 17.5 Hz), 6.13 (dd, 2H, J = 10.5 Hz, 17.5 Hz), 5.82 (dd, 2H, J = 1.5 Hz, 10.5 Hz), 4.32 (t, 4H, J = 7.5 Hz), 4.18 (t, 4H, J = 6.5 Hz), 3.95 (t, 4H, J = 6.5 Hz), 2.77-2.58 (m, 4H), 2.40-2.29 (m, 8H), 1.83-1.32 (m, 40H), 0.91 (t, 6H, J = 7.0 Hz)

(Synthesis Example 4: Synthesis of compound 4 (another example of compound represented by formula (VI-1)))

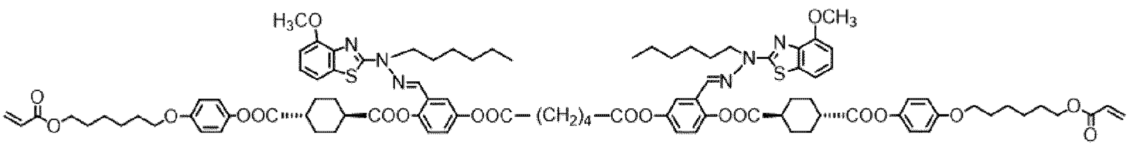

Compound 4

<Step 1: Synthesis of intermediate G>

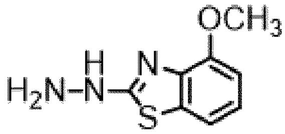

Intermediate G

In a four-necked reaction vessel equipped with a thermometer, 4.00 g (22.2 mmol) of 2-amino-4-methoxybenzothiazole was dissolved in 40 mL of ethylene glycol and 15 mL of water in a stream of nitrogen. Next, 11.1 g (222 mmol) of hydrazine monohydrate and 2.8 mL (33.3 mmol) of 12 N hydrochloric acid were added to the solution and were stirred therewith for 15 hours at 120°C. Once the reaction ended, the reaction liquid was cooled to 20°C and was added into 200 mL of 10% sodium bicarbonate water. An extraction was performed with 800 mL of ethyl acetate. The ethyl acetate layer was dried with anhydrous sodium sulfate. Sodium sulfate was filtered off and then ethyl acetate was evaporated under reduced pressure in a rotary evaporator to yield a yellow solid. The yellow solid was recrystallized from ethyl

acetate to yield 2.3 g of intermediate G. The yield was 53.1 mol%. The structure of the intermediate G was identified by $^1$H-NMR. The $^1$H-NMR spectrum data are shown below.

$^1$H-NMR (500 MHz, DMSO-$d_6$, TMS, $\delta$ ppm): 8.93 (s, 1H), 7.27 (dd, 1H, J = 1.0, 8.0 Hz), 6.94 (dd, 1H, J = 8.0, 8.0 Hz), 6.82 (dd, 1H, J = 1.0 Hz, 8.0 Hz), 5.00 (s, 2H), 3.82 (s, 3H)

<Step 2: Synthesis of intermediate H>

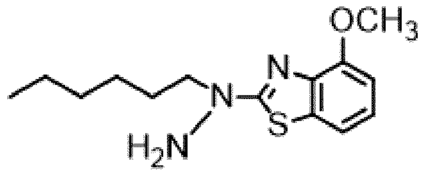

Intermediate H

In a four-necked reaction vessel equipped with a thermometer, 2.00 g (10.2 mmol) of the intermediate G synthesized in step 1 was dissolved in 20 mL of dimethylformamide in a stream of nitrogen. In addition, 6.68 g (20.4 mmol) of cesium carbonate and 2.0 g (12.2 mmol) of 1-bromohexane were added to the solution and were stirred therewith for 6 hours at 50°C. Once the reaction ended, the reaction liquid was cooled to 20°C and was added into 200 mL of water. An extraction was performed with 300 mL of ethyl acetate. The ethyl acetate layer was dried with anhydrous sodium sulfate. Sodium sulfate was filtered off and then ethyl acetate was evaporated under reduced pressure in a rotary evaporator to yield a yellow solid. The yellow solid was purified by silica gel column chromatography (hexane:THF = 80:20 (volume ratio)) to yield 2.0 g of intermediate H in the form of a white solid. The yield was 70.2 mol%. The structure of the intermediate H was identified by $^1$H-NMR. The $^1$H-NMR spectrum data are shown below.

$^1$H-NMR (500 MHz, $CDCl_3$, TMS, $\delta$ ppm): 7.22 (dd, 1H, J = 1.0 Hz, 8.0 Hz), 7.04 (dd, 1H, J = 8.0 Hz, 8.0 Hz), 6.81 (dd, 1H, J = 1.0 Hz, 8.0 Hz), 4.26 (s, 2H), 3.98 (s, 3H), 3.73 (t, 2H, J = 7.5 Hz), 1.75-1.69 (m, 2H), 1.41-1.27 (m, 6H), 0.89 (t, 3H, J = 7.0 Hz)

<Step 3: Synthesis of compound 4 (another example of compound represented by formula (VI-1))>

In a four-necked reaction vessel equipped with a thermometer, 2.0 g (1.70 mmol) of the intermediate C synthesized in step 3 of Synthesis Example 1 and 1.19 g (4.26 mmol) of intermediate H synthesized in step 2 were added to a mixed solvent of 50 mL of tetrahydrofuran (THF) and 5 mL of ethanol in a stream of nitrogen. In addition, 79 mg (0.34 mmol) of (±)-10-camphorsulfonic acid was added to the solution and was stirred therewith for 4 hours at 50°C. Once the reaction ended, the reaction liquid was added into 300 mL of 3 mass% sodium bicarbonate water, and two extractions were performed with 200 mL of ethyl acetate. The organic layers were combined and were washed with 300 mL of saturated saline water. Thereafter, drying was performed with anhydrous sodium sulfate, and then sodium sulfate was filtered off. Solvent was removed in a rotary evaporator and then the resultant residue was purified by silica gel column chromatography (chloroform:THF = 90:10 (volume ratio)) to yield 2.05 g of compound 4 in the form of a pale yellow solid. The yield was 70.5 mol%. The structure of the target (compound 4) was identified by $^1$H-NMR. The $^1$H-NMR spectrum data are shown below.

$^1$H-NMR (500 MHz, $CDCl_3$, TMS, $\delta$ ppm): 7.76 (dd, 2H, J = 0.5 Hz, 2.5 Hz), 7.60 (s, 2H), 7.24 (dd, 2H, J = 1.0 Hz, 7.5 Hz), 7.11-7.07 (m, 6H), 6.98 (d, 4H, J = 9.0 Hz), 6.88 (d, 4H, J = 9.0 Hz), 6.83 (dd, 2H, J = 1.0 Hz, 8.5 Hz), 6.40 (dd, 2H, J = 1.5 Hz, 17.5 Hz), 6.13 (dd, 2H, J = 10.5 Hz, 17.5 Hz), 5.82 (dd, 2H, J = 1.5 Hz, 10.5 Hz), 4.31 (t, 4H, J = 7.5 Hz), 4.18 (t, 4H, J = 6.5 Hz), 4.01 (s, 6H), 3.95 (t, 4H, J = 6.5 Hz), 2.74-2.55 (m, 8H), 2.32-2.31 (m, 8H), 2.00-1.94 (m, 4H), 1.83-1.29 (m, 40H), 0.89 (t, 6H, J = 7.0 Hz)

(Synthesis Example 5: Synthesis of compound 5 (another example of compound represented by formula (VI-1)))

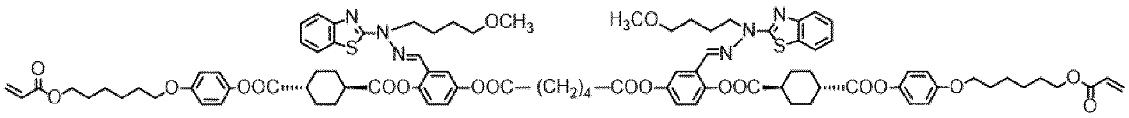

Compound 5

<Step 1: Synthesis of intermediate I>

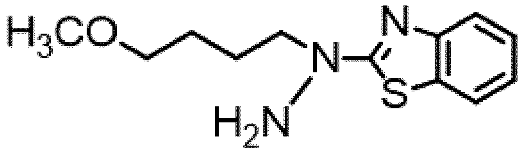

Intermediate I

In a four-necked reaction vessel equipped with a thermometer, 3.00 g (18.16 mmol) of 2-hydrazinobenzothiazole was dissolved in 50 mL of dimethylformamide in a stream of nitrogen. In addition, 11.83 g (36.32 mmol) of cesium carbonate and 3.64 g (21.79 mmol) of 1-bromo-4-methoxybutane were added to the solution and were stirred therewith for 6 hours at room temperature. Once the reaction ended, the reaction liquid was cooled to 20°C and was added into 300 mL of water. An extraction was performed with 300 mL of ethyl acetate. The ethyl acetate layer was dried with anhydrous sodium sulfate. Sodium sulfate was filtered off and then ethyl acetate was evaporated under reduced pressure in a rotary evaporator to yield a yellow solid. The yellow solid was purified by silica gel column chromatography (hexane:THF = 70:30 (volume ratio)) to yield 2.8 g of intermediate I in the form of a white solid. The yield was 61.3 mol%. The structure of the intermediate I was identified by $^1$H-NMR. The $^1$H-NMR spectrum data are shown below.

$^1$H-NMR (500 MHz, $CDCl_3$, TMS, $\delta$ ppm): 7.60 (dd, 1H, J = 1.0 Hz, 7.5 Hz), 7.52 (dd, 1H, J = 1.0 Hz, 7.5 Hz), 7.30-7.25 (m, 1H), 7.06 (ddd, 1H, J = 1.0 Hz, 7.5 Hz, 7.5 Hz), 4.27 (s, 2H), 3.80 (t, 2H, J = 7.5 Hz), 3.43 (t, 2H, J = 6.5 Hz), 3.34 (s, 3H), 1.87-1.81 (m, 2H), 1.70-1.65 (m, 2H)

<Step 2: Synthesis of compound 5 (another example of compound represented by formula (VI-1))>

In a four-necked reaction vessel equipped with a thermometer, 2.0 g (1.70 mmol) of the intermediate C synthesized in step 3 of Synthesis Example 1 and 1.07 g (4.26 mmol) of the intermediate I synthesized in step 1 were added to a mixed solvent of 50 mL of tetrahydrofuran (THF) and 5 mL of ethanol in a stream of nitrogen. In addition, 79 mg (0.34 mmol) of ($\pm$)-10-camphorsulfonic acid was added to the solution and was stirred therewith for 6 hours at 50°C. Once the reaction ended, the reaction liquid was added into 300 mL of 3 mass% sodium bicarbonate water, and two extractions were performed with 200 mL of ethyl acetate. The organic layers were combined and were washed with 300 mL of saturated saline water. Thereafter, drying was performed with anhydrous sodium sulfate, and then sodium sulfate was filtered off. Solvent was removed in a rotary evaporator and then the resultant residue was purified by silica gel column chromatography (chloroform:THF = 90:10 (volume ratio)) to yield 1.89 g of compound 5 in the form of a pale yellow solid. The yield was 67.2 mol%. The structure of the target (compound 5) was identified by $^1$H-NMR. The $^1$H-NMR spectrum data are shown below.

$^1$H-NMR (500 MHz, $CDCl_3$, TMS, $\delta$ ppm): 7.75 (dd, 2H, J = 1.0 Hz, 2.5 Hz), 7.63 (d, 2H, J = 8.0 Hz), 7.54 (dd, 2H, J = 1.0 Hz, 8.0 Hz), 7.51 (s, 2H), 7.30 (ddd, 2H, J = 1.5 Hz, 7.5 Hz, 7.5 Hz), 7.11-7.06 (m, 6H), 6.98 (d, 4H, J = 9.0 Hz), 6.88 (d, 4H, J = 9.0 Hz), 6.41 (dd, 2H, J = 1.5 Hz, 17.5 Hz), 6.13 (dd, 2H, J = 10.5 Hz, 17.5 Hz), 5.82 (dd, 2H, J = 1.5 Hz, 10.5 Hz), 4.20-4.16 (m, 8H), 3.95 (t, 4H, J = 6.5 Hz), 3.41 (t, 4H, J = 6.5 Hz), 3.32 (s, 6H), 2.75-2.57 (m, 8H), 2.32-2.31 (m, 8H), 2.05-1.98 (m, 4H), 1.81-1.42 (m, 32H)

(Synthesis Example 6: Synthesis of compound 1 (one example of compound represented by formula (VI-1)))

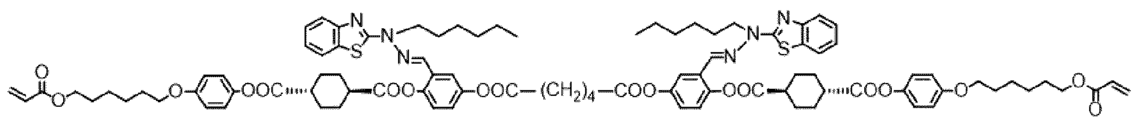

Compound 1

<Step 1: Synthesis of intermediate J>

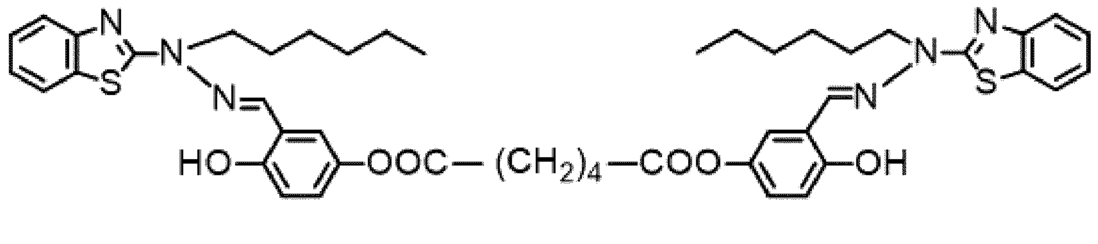

Intermediate J

In a four-necked reaction vessel equipped with a thermometer, 2.0 g (5.18 mmol) of the intermediate B synthesized in step 2 of Synthesis Example 1, 3.23 g (12.94 mmol) of the intermediate D synthesized in step 4 of Synthesis Example 1, and 240 mg (10.4 mmol) of (±)-10-camphorsulfonic acid were added to 50 mL of methanol in a stream of nitrogen. The solution was stirred for 2 hours at 40°C. Once the reaction ended, the reaction liquid was added into 300 mL of 3 mass% sodium bicarbonate water, and an extraction was performed with 500 mL of ethyl acetate. The organic layer was collected and was dried with anhydrous sodium sulfate, and then sodium sulfate was filtered off. Solvent was evaporated in a rotary evaporator and then the resultant residue was purified by silica gel column chromatography (hexane:THF = 50:50 (volume ratio)) to yield 2.3 g of intermediate J in the form of a pale yellow solid. The yield was 52.3 mol%. The structure of the intermediate J was identified by $^1$H-NMR.

$^1$H-NMR (500 MHz, $CDCl_3$, TMS, δ ppm): 10.16 (s, 2H), 7.77 (s, 2H), 7.67 (m, 4H), 7.36 (ddd, 2H, J = 1.0 Hz, 8.0 Hz, 8.0 Hz), 7.17 (ddd, 2H, J = 1.0 Hz, 8.0 Hz, 8.0 Hz), 7.05-7.00 (m, 6H), 4.35 (t, 4H, J = 7.5 Hz), 2.68-2.64 (m, 4H), 1.94-1.80 (m, 4H), 1.79-1.72 (m, 4H), 1.48-1.26 (m, 12H), 0.90 (t, 6H, J = 7.0 Hz)

<Step 2: Synthesis of compound 1 (one example of compound represented by formula (VI-1))>

In a three-necked reaction vessel equipped with a thermometer, 2.0 g (2.36 mmol) of the intermediate J synthesized in step 1, 2.46 g (5.89 mmol) of the intermediate A synthesized in step 1 of Synthesis Example 1, and 72 mg (0.59 mmol) of N,N-dimethylaminopyridine were added to 100 mL of N-methylpyrrolidone in a stream of nitrogen. In addition, 1.35 g (7.07 mmol) of 1-ethyl-3-(3-dimethylaminopropyl)carbodiimide hydrochloride was slowly added under stirring at 25°C. Thereafter, the reaction vessel was stirred for 15 hours at 25°C to carry out a reaction. Once the reaction ended, 500 mL of distilled water and 100 mL of saturated saline water were added to the resultant reaction liquid, and two extractions were performed with 200 mL of ethyl acetate. The resultant organic layers were dried with anhydrous sodium sulfate, and then sodium sulfate was filtered off. Solvent was removed in a rotary evaporator and then the resultant residue was purified by silica gel column chromatography (toluene:ethyl acetate = 85:15 (volume ratio)) to yield 2.5 g of compound 1 in the form of a pale yellow solid. The yield was 64.2 mol%. The structure of the target (compound 1) was identified by $^1$H-NMR. The $^1$H-NMR spectrum data are shown below.

$^1$H-NMR (500 MHz, $CDCl_3$, TMS, δ ppm): 7.76-7.75 (m, 2H), 7.64 (d, 2H, J = 8.0 Hz), 7.53 (dd, 2H, J = 0.5 Hz, 8.0 Hz), 7.48 (s, 2H), 7.31 (dd, 1H, J = 1.0 Hz, 8.0 Hz), 7.30 (dd, 1H, J = 1.0 Hz, 8.0 Hz), 7.13 (m, 6H), 6.98 (d, 4H, J = 9.0 Hz), 6.88 (d, 4H, J = 9.0 Hz), 6.40 (dd, 2H, J = 1.5 Hz, 17.5 Hz), 6.13 (dd, 2H, J = 10.5 Hz, 17.5 Hz), 5.82 (dd, 2H, J = 1.5 Hz, 10.5 Hz), 4.18 (t, 4H, J = 6.5 Hz), 4.14 (t, 4H, J = 7.5 Hz), 3.95 (t, 4H, J = 6.5 Hz), 2.78-2.55 (m, 8H), 2.38-2.25 (m, 8H), 2.02-1.95 (m, 4H), 1.85-1.30 (m, 40H), 0.95 (t, 6H, J = 7.0 Hz)

(Comparative Synthesis Example 1: Synthesis of compound X (one example of polymerizable compound represented by formula (I-2)))

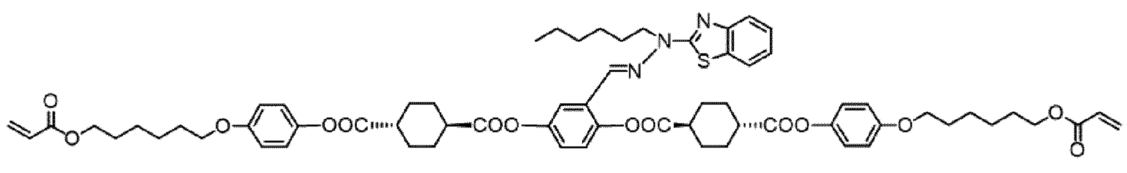

Compound X

<Step 1: Synthesis of intermediate X1>

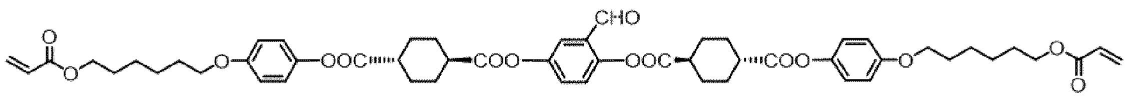

Intermediate X1

A three-necked reaction vessel equipped with a thermometer was charged with 4.00 g (9.56 mmol) of intermediate A synthesized in the same way as in step 1 of Synthesis Example 1 and 60 mL of THF in a stream of nitrogen, and a homogeneous solution was obtained. In addition, 1.12 g (9.78 mmol) of methanesulfonyl chloride was added into the reaction vessel and the reaction vessel was immersed in a water bath to attain a reaction liquid internal temperature of 20°C. Next, 1.01 g (9.99 mmol) of triethylamine was added dropwise over 5 minutes while maintaining the reaction liquid internal temperature at 20°C to 30°C. After completion of the dropwise addition, the entire contents of the reaction vessel were further stirred for 2 hours at 25°C. Next, 0.11 g (0.87 mmol) of 4-(dimethylamino)pyridine and 0.60 g (4.35 mmol) of 2,5-dihydroxybenzaldehyde were added to the resultant reaction liquid, and the reaction vessel was immersed in a water bath once again to attain a reaction liquid internal temperature of 15°C. Thereafter, 1.10 g (10.87 mmol) of triethylamine was added dropwise over 5 minutes while maintaining the reaction liquid internal temperature at 20°C to 30°C. After completion of the dropwise addition, the entire contents of the reaction vessel were further stirred for 2 hours at 25°C. Once the reaction ended, 400 mL of distilled water and 50 mL of saturated saline water were added to the reaction liquid, and two extractions were performed with 750 mL of ethyl acetate. The organic layers were collected and were dried with anhydrous sodium sulfate, and then sodium sulfate was filtered off. Solvent was removed from the filtrate by evaporation in a rotary evaporator and then the resultant residue was dissolved in 100 mL of THF. Crystals were caused to precipitate by adding 500 mL of methanol to the solution and were then collected by filtration. The obtained crystals were washed with methanol and were then vacuum dried to yield 2.51 g of intermediate XI in the form of a white solid. The yield was 62 mol%. The structure of the intermediate XI was identified by $^1$H-NMR. The $^1$H-NMR spectrum data are shown below.

$^1$H-NMR (500 MHz, DMSO-$d_6$, TMS, $\delta$ ppm): 10.02 (s, 1H), 7.67 (d, 1H, J = 3.0 Hz), 7.55 (dd, 1H, J = 3.0 Hz, 8.5 Hz), 7.38 (d, 1H, J = 8.5 Hz), 6.99-7.04 (m, 4H), 6.91-6.96 (m, 4H), 6.32 (dd, 2H, J = 1.5 Hz, 17.5 Hz), 6.17 (dd, 2H, J = 10.0 Hz, 17.5 Hz), 5.93 (dd, 2H, J = 1.5 Hz, 10.0 Hz), 4.11 (t, 4H, J = 6.5 Hz), 3.95 (t, 4H, J = 6.5 Hz), 2.56-2.81 (m, 4H), 2.10-2.26 (m, 8H), 1.50-1.76 (m, 16H), 1.33-1.49 (m, 8H)

<Step 2: Synthesis of compound X (one example of polymerizable compound represented by formula (I-2))>

In a four-necked reaction vessel equipped with a thermometer, 697 mg (2.37 mmol) of intermediate D synthesized in the same way as in step 4 of Synthesis Example 1 and 2.00 g (2.13 mmol) of the intermediate XI synthesized in step 1 were dissolved in a mixed solvent of 3 mL of ethanol and 20 mL of tetrahydrofuran in a stream of nitrogen. Next, 55.1 mg (0.24 mmol) of (±)-10-camphorsulfonic acid was added to the solution and was stirred therewith for 5 hours at 40°C. Once the reaction ended, the reaction liquid was added into 150 mL of water, and an extraction was performed with 300 mL of ethyl acetate. The ethyl acetate layer was dried with anhydrous sodium sulfate. Sodium sulfate was filtered off and then ethyl acetate was evaporated under reduced pressure in a rotary evaporator to yield a white solid. The white solid was purified by silica gel column chromatography (toluene:ethyl acetate = 90:10 (volume ratio)) to yield 2.24 g of compound X in the form of a white solid. The yield was 86.4 mol%. The structure of the target (compound X) was identified by $^1$H-NMR. The $^1$H-NMR spectrum data are shown below.

H-NMR (400 MHz, $CDCl_3$, TMS, $\delta$ ppm): 7.75 (d, 1H, J = 2.5 Hz), 7.67-7.70 (m, 3H), 7.34 (ddd, 1H, J = 1.0 Hz, 7.0 Hz, 7.5 Hz), 7.17 (ddd, 1H, J = 1.0 Hz, 7.5 Hz, 7.5 Hz), 7.12 (d, 1H, J = 9.0 Hz), 7.10 (dd, 1H, J = 2.5 Hz, 9.0 Hz), 6.99 (d, 2H, J = 9.0 Hz), 6.98 (d, 2H, J = 9.0 Hz), 6.88 (d, 4H, J = 9.0 Hz), 6.40 (dd, 2H, J = 1.5 Hz, 17.0 Hz), 6.13 (dd, 2H, J = 10.5 Hz, 17.5 Hz), 5.82 (dd, 2H, J = 1.5 Hz, 10.5 Hz), 4.30 (t, 2H, J = 8.0 Hz), 4.18 (t, 4H, J = 6.5 Hz), 3.95 (t,

4H, J = 6.5 Hz), 2.58-2.70 (m, 4H), 2.31-2.35 (m, 8H), 1.66-1.82 (m, 18H), 1.31-1.54 (m, 14H), 0.90 (t, 3H, J = 7.0 Hz)

<Measurement of phase transition temperature>

For each of the compounds 1 to 5 and the compound X, 5 mg of the compound was weighed out and, in a solid state, was sandwiched between two glass substrates each provided with a polyimide alignment film that had been subjected to rubbing (produced by E.H.C. Co., Ltd.; product name: Alignment Treatment Glass Substrate). The substrates were placed on a hot plate and were heated from 50°C to 200°C before being allowed to cool back to 50°C. A polarizing optical microscope (ECLIPSE LV100POL produced by Nikon Corporation) was used to observe change in structure during heating and cooling.

The measured phase transition temperatures are shown below in Table 1.

In Table 1, "C" represents crystal, "N" represents nematic, and "I" represents isotropic. Moreover, "crystal" indicates that the test compound is in a solid phase, "nematic" indicates that the test compound is in a nematic liquid crystal phase, and "isotropic" indicates that the test compound is in an isotropic liquid phase.

Table 1

| Compound number | Phase transition temperature |
|---|---|
| Compound 1 | $C \underset{\leq 50^\circ C}{\overset{152^\circ C}{\rightleftarrows}} N \underset{152^\circ C}{\overset{155^\circ C}{\rightleftarrows}} I$ |
| Compound 2 | $C \underset{\leq 50^\circ C}{\overset{183^\circ C}{\rightleftarrows}} N \xrightarrow{\geq 200^\circ C} I$ |
| Compound 3 | $C \underset{\leq 50^\circ C}{\overset{190^\circ C}{\rightleftarrows}} N \xrightarrow{\geq 200^\circ C} I$ |
| Compound 4 | $C \underset{142^\circ C}{\overset{191^\circ C}{\rightleftarrows}} N \underset{185^\circ C}{\overset{192^\circ C}{\rightleftarrows}} I$ |
| Compound 5 | $C \xrightarrow{167^\circ C} I$; $I \xrightarrow{150^\circ C} N \xrightarrow{\leq 50^\circ C} C$ |
| Compound X | $C \underset{\leq 50^\circ C}{\overset{96^\circ C}{\rightleftarrows}} N \xrightarrow{\geq 200^\circ C} I$ |

(Examples 1 to 5)

<Preparation of polymerizable liquid crystal compositions>

For each of the compounds 1 to 5 obtained in Synthesis Examples 1 to 5, a solution was obtained by dissolving 1.0 g of the compound, 43 mg of a photoinitiator ADEKA ARKLS N1919T (produced by ADEKA Corporation), and 300 mg of a mixed solvent of cyclopentanone and 1,3-dioxolane (mixing ratio (mass ratio): cyclopentanone/1,3-dioxolane = 4/6) containing 1 mass% of a surfactant MEGAFACE F-562 (produced by DIC Corporation) in a separately prepared mixed solvent of 2.05 g of 1,3-dioxolane and 1.37 g of cyclopentanone. The resultant solution was filtered using a disposable filter having a pore diameter of 0.45 μm. In this manner, polymerizable compositions 1 to 5 were obtained.

(Example 6)

A solution was obtained by dissolving 0.8 g of the compound 4 obtained in Synthesis Example 4, 0.2 g of the compound X obtained in Comparative Synthesis Example 1, 43 mg of a photoinitiator ADEKA ARKLS N1919T (produced by ADEKA Corporation), and 300 mg of a mixed solvent of cyclopentanone and 1,3-dioxolane (mixing ratio (mass ratio): cyclopentanone/1,3-dioxolane = 4/6) containing 1 mass% of a surfactant MEGAFACE F-562 (produced by DIC Corporation) in a mixed solvent of 2.05 g of 1,3-dioxolane and 1.37 g of cyclopentanone. The resultant solution was filtered using a disposable filter having a pore diameter of 0.45 μm to obtain a polymerizable composition 6.

(Example 7)

A solution was obtained by dissolving 0.55 g of the compound 4 obtained in Synthesis Example 4, 0.45 g of the compound X obtained in Comparative Synthesis Example 1, 43 mg of a photoinitiator ADEKA ARKLS N1919T (produced by ADEKA Corporation), and 300 mg of a mixed solvent of cyclopentanone and 1,3-dioxolane (mixing ratio (mass ratio): cyclopentanone/1,3-dioxolane = 4/6) containing 1 mass% of a surfactant MEGAFACE F-562 (produced by DIC Corporation) in a mixed solvent of 2.05 g of 1,3-dioxolane and 1.37 g of cyclopentanone. The resultant solution was filtered using a disposable filter having a pore diameter of 0.45 μm to obtain a polymerizable composition 7.

(Example 8)

A solution was obtained by dissolving 0.70 g of the compound 2 obtained in Synthesis Example 2, 0.30 g of the compound X obtained in Comparative Synthesis Example 1, 43 mg of a photoinitiator ADEKA ARKLS N1919T (produced by ADEKA Corporation), and 300 mg of a mixed solvent of cyclopentanone and 1,3-dioxolane (mixing ratio (mass ratio): cyclopentanone/1,3-dioxolane = 4/6) containing 1 mass% of a surfactant MEGAFACE F-562 (produced by DIC Corporation) in a mixed solvent of 2.05 g of 1,3-dioxolane and 1.37 g of cyclopentanone. The resultant solution was filtered using a disposable filter having a pore diameter of 0.45 μm to obtain a polymerizable composition 8.

(Comparative Example 1)

<Preparation of polymerizable liquid crystal composition>

A solution was obtained by dissolving 1.0 g of the compound X obtained in Comparative Synthesis Example 1, 43 mg of a photoinitiator ADEKA ARKLS N1919T (produced by ADEKA Corporation), and 300 mg of a mixed solvent of cyclopentanone and 1,3-dioxolane (mixing ratio (mass ratio): cyclopentanone/1,3-dioxolane = 4/6) containing 1 mass% of a surfactant MEGAFACE F-562 (produced by DIC Corporation) in a separately prepared mixed solvent of 2.05 g of 1,3-dioxolane and 1.37 g of cyclopentanone. The resultant solution was filtered using a disposable filter having a pore diameter of 0.45 μm to obtain a polymerizable composition 1r.

<Evaluation of optical properties>

(i) Formation of liquid crystal layer by polymerizable composition

Each of the polymerizable compositions 1 to 8 and 1r obtained as described above was applied onto a transparent glass substrate provided with a polyimide alignment film that had been subjected to rubbing (product name: Alignment Treatment Glass Substrate; produced by E.H.C. Co., Ltd.) using a #6 wire bar so as to obtain an applied film. The resultant applied film was dried for 1 minute at a temperature indicated below in Tables 2 and 3, and was then subjected to alignment treatment for 1 minute at a temperature indicated in Tables 2 and 3 so as to form a liquid crystal layer.

(ii) Formation of optically anisotropic body

Irradiation with 2000 mJ/$cm^2$ of ultraviolet rays was subsequently performed from a side of the application surface of the liquid crystal layer prepared in (i) at a temperature indicated in Tables 2 and 3 so as to cause polymerization and thereby obtain an optically anisotropic body attached to a transparent glass substrate as a sample for wavelength dispersion measurement. The film thickness of the optically anisotropic body was measured by using a needle to form a scratch in the optically anisotropic body of the transparent glass substrate-attached optically anisotropic body, and then measuring a step at the scratch using a surface profiler Dektak 150 (produced by ULVAC, Inc.). The results are shown in Tables 2 and 3.

(iii) Measurement of retardation

A Mueller Matrix Polarimeter AxoScan (produced by Axometrics, Inc.) was used to measure retardation at wavelengths from 400 nm to 800 nm for each sample obtained in (ii). The retardation at a wavelength of 550 nm is shown in Tables 2 and 3.

(iv) Evaluation of wavelength dispersion

Wavelength dispersion was evaluated based on wavelength dispersion ratios calculated as follows using the measured retardation. The results are shown in Tables 2 and 3.

Wavelength dispersion ratio at 600 nm = (Retardation at 600 nm)/(Retardation at 550 nm)

Wavelength dispersion ratio at 650 nm = (Retardation at 650 nm)/(Retardation at 550 nm)

Wavelength dispersion ratio at 700 nm = (Retardation at 700 nm)/(Retardation at 550 nm)

Wavelength dispersion ratio at 750 nm = (Retardation at 750 nm)/(Retardation at 550 nm)

Wavelength dispersion ratio at 800 nm = (Retardation at 800 nm)/(Retardation at 550 nm)

Table 2

| | Polymerizable composition | Polymerizable compound | Drying temperature (°C) | Alignment treatment temperature (°C) | Temperature during photoexposure (°C) | Film thickness (μm) | Retardation at 550 nm (nm) | Wavelength dispersion ratio | | | | |
|---|---|---|---|---|---|---|---|---|---|---|---|---|
| | | Used compound | | | | | | 600 nm | 650 nm | 700 nm | 750 nm | 800 nm |
| Example 1 | 1 | Compound 1 | 160 | 23 | 23 | 2.15 | 130.124 | 1.0496 | 1.0750 | 1.0910 | 1.1007 | 1.1072 |
| Example 2 | 2 | Compound 2 | 185 | 23 | 23 | 1.93 | 108.537 | 1.0502 | 1.0797 | 1.1026 | 1.1150 | 1.1217 |
| Example 3 | 3 | Compound 3 | 195 | 23 | 23 | 1.88 | 143.271 | 1.0275 | 1.0408 | 1.0495 | 1.0552 | 1.0593 |
| Example 4 | 4 | Compound 4 | 195 | 150 | 150 | 2.59 | 79.211 | 1.0548 | 1.0795 | 1.1018 | 1.1150 | 1.1190 |
| Example 5 | 5 | Compound 5 | 175 | 23 | 23 | 2.08 | 113.650 | 1.0325 | 10.535 | 1.0782 | 1.0922 | 1.0980 |
| Comparative Example 1 | 1r | Compound X | 110 | 23 | 23 | 1.92 | 141.876 | 1.0242 | 1.0368 | 1.0429 | 1.0481 | 1.0509 |
| Ideal value | | | | | | | | 1.0909 | 1.1818 | 1.2727 | 1.3636 | 1.4545 |

Table 3

| | Polymenzable composition | Polymerizable compounds %) (proportion: mass%) | | Drying temperature (°C) | Alignment treatment temperature (°C) | Temperature during photo exposure (°C) | Film thickness (μm) | Retardation at 550 nm (nm) | Wavelength dispersion ratio | | | | |
|---|---|---|---|---|---|---|---|---|---|---|---|---|---|
| | | | | | | | | | 600 nm | 650 nm | 700 nm | 750 nm | 800 nm |
| Example 6 | 6 | Compound X (20) | Compound 4(80) | 195 | 150 | 150 | 2.60 | 85.733 | 1.0494 | 1.0699 | 1.0849 | 1.1011 | 1.1125 |
| Example 7 | 7 | Compound X (45) | Compound 4(55) | 195 | 140 | 140 | 2.58 | 93.886 | 1.0438 | 1.0599 | 1.0702 | 1.0810 | 1.0903 |
| Example 8 | 8 | Compound X (30) | Compound 2(70) | 185 | 23 | 23 | 1.95 | 109.523 | 1.0445 | 1.0677 | 1.0820 | 1.0945 | 1.1042 |
| Comparative Example 1 | 1r | Compound X (100) | - | 110 | 23 | 23 | 1.92 | 141.876 | 1.0242 | 1.0368 | 1.0429 | 1.0481 | 1.0509 |
| Ideal value | | | | | | | | | 1.0909 | 1.1818 | 1.2727 | 1.3636 | 1.4545 |

It can be seen from Table 2 that improvement was observed in Examples 1 to 5 (i.e., for optically anisotropic bodies formed using the polymerizable compositions 1 to 5 containing the compounds 1 to 5) in terms that deviation from the ideal values for wavelength dispersion ratios at long wavelengths (600 nm to 800 nm) decreased. In particular, reverse wavelength dispersion at long wavelengths noticeably improved at wavelengths from 700 nm to 800 nm.

Moreover, it can be seen from Table 3 that for optically anisotropic bodies formed using the polymerizable compositions 6 to 8 (compositions in which the compound X was mixed with the compound 2 or the compound 4), improvement was observed in the same manner as for the optically anisotropic bodies formed using the polymerizable compositions 1 to 5.

INDUSTRIAL APPLICABILITY

The present disclosure provides a polymer capable of forming an optical film or optically anisotropic body having good reverse wavelength dispersion at long wavelengths, and also a polymerizable compound and a polymerizable liquid crystal mixture that are useful in production of the polymer.

Moreover, the present disclosure provides a compound that is useful in production of the polymerizable compound.

Furthermore, the present disclosure provides an optical film and an optically anisotropic body for which reverse wavelength dispersion at long wavelengths is improved to provide excellent reverse wavelength dispersion at long wavelengths, and also a polarizer, a display, and an antireflection film in which the optically anisotropic body is used.

## Claims

**1.** A polymerizable compound indicated by formula (1-1), shown below,

$$P^1-L^1-Y^3\left[B^1-Y^1\right]_p A^1-Z^1-Ar^0-Z^2-Xa-Z^3-Ar^1-Z^4-A^2\left[Y^2-B^2\right]_q Y^4-L^2-P^2$$

(I − 1)

where, in formula (1-1),

$Ar^0$ represents an aromatic hydrocarbon cyclic group having at least $D^0$ as a substituent or an aromatic heterocyclic group having at least $D^0$ as a substituent,
$Ar^1$ represents an aromatic hydrocarbon cyclic group having at least $D^1$ as a substituent or an aromatic heterocyclic group having at least $D^1$ as a substituent,
$D^0$ and $D^1$ each represent, independently of one another, an organic group having a carbon number of 1 to 67 and including at least one aromatic ring selected from the group consisting of an aromatic hydrocarbon ring and an aromatic heterocyclic ring,
Xa represents an optionally substituted organic group having a carbon number of 1 to 20,
$Z^1$ to $Z^4$ each represent, independently of one another, a single bond, -O-, $-O-CH_2-$, $-CH_2-O-$, -C(=O)-O-, -O-C(=O)-, -C(=O)-S-, -S-C(=O)-, $-NR^{20}C(=O)-$, $-C(=O)-NR^{20}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, $-CH_2-C(=O)-O-$, $-O-C(=O)-CH_2-$, $-CH_2-O-C(=O)-$, $-C(=O)-O-CH_2-$, $-O-CH_2-CH_2-$, $-CH_2-CH_2-O-$, or -C≡C-, where $R^{20}$ represents a hydrogen atom or an alkyl group having a carbon number of 1 to 6,
$A^1$, $A^2$, $B^1$, and $B^2$ each represent, independently of one another, an optionally substituted alicyclic group or an optionally substituted aromatic group,
$Y^1$ to $Y^4$ each represent, independently of one another, a single bond, -O-, -C(=O)-, -C(=O)-O-, -O-C(=O)-, $-NR^{21}-C(=O)-$, $-C(=O)-NR^{21}-$, -O-C(=O)-O-, $-NR^{21}-C(=O)-O-$, $-O-C(=O)-NR^{21}-$, or $-NR^{21}-C(=O)-NR^{22}-$, where $R^{21}$ and $R^{22}$ each represent, independently of one another, a hydrogen atom or an alkyl group having a carbon number of 1 to 6,
$L^1$ and $L^2$ are each, independently of one another, an organic group that is either an alkylene group having a carbon number of 1 to 20 or a group in which at least one methylene group ($-CH_2-$) included in an alkylene group having a carbon number of 1 to 20 is replaced by -O- or -C(=O)-, where hydrogen atoms included in the organic groups of $L^1$ and $L^2$ may each be replaced by an alkyl group having a carbon number of 1 to 5, an alkoxy group having a carbon number of 1 to 5, or a halogen atom, and with a proviso that methylene groups

(-$CH_2$-) at both ends of $L^1$ and $L^2$ are not replaced by -O- or -C(=O)-, one of $P^1$ and $P^2$ represents a hydrogen atom or a polymerizable group and the other of $P^1$ and $P^2$ represents a polymerizable group, p and q are each, independently of one another, an integer of 0 to 2, and in a case in which more than one $B^1$, $B^2$, $Y^1$, or $Y^2$ is present, each $B^1$, $B^2$, $Y^1$, or $Y^2$ may be the same or different.

**2.** The polymerizable compound according to claim 1, wherein $Ar^0$ and $Ar^1$ are each, independently of one another, indicated by any one of formulae (II-1) to (II-7), shown below,

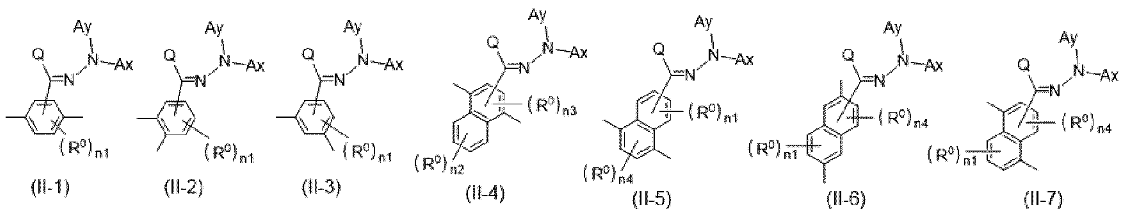

(II-1) (II-2) (II-3) (II-4) (II-5) (II-6) (II-7)

where, in formulae (II-1) to (II-7),

Ax represents an organic group including at least one aromatic ring selected from the group consisting of an aromatic hydrocarbon ring having a carbon number of 6 to 30 and an aromatic heterocyclic ring having a carbon number of 2 to 30, where the aromatic ring of Ax is optionally substituted,
Ay represents a hydrogen atom or an optionally substituted organic group having a carbon number of 1 to 30,
Q represents a hydrogen atom or an alkyl group having a carbon number of 1 to 6,
$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, -C(=O)-$R^a$, -O-C(=O)-$R^a$, -C(=O)-O-$R^a$, or -$SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, n1 is 0 to 3, n2 is 0 to 4, n3 is 0 or 1, and n4 is 0 to 2, and
in a case in which more than one $R^0$ is present, each $R^0$ may be the same or different.

**3.** The polymerizable compound according to claim 2, wherein the polymerizable compound is indicated by any one of formulae (III-1) to (III-6), shown below,

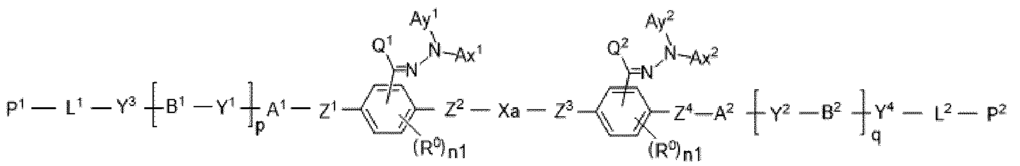

(III-1)

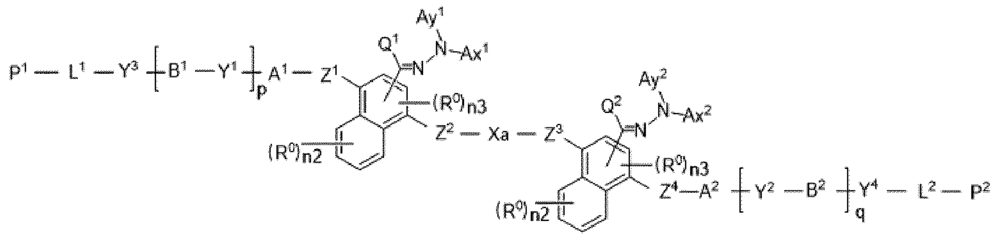

(III-2)

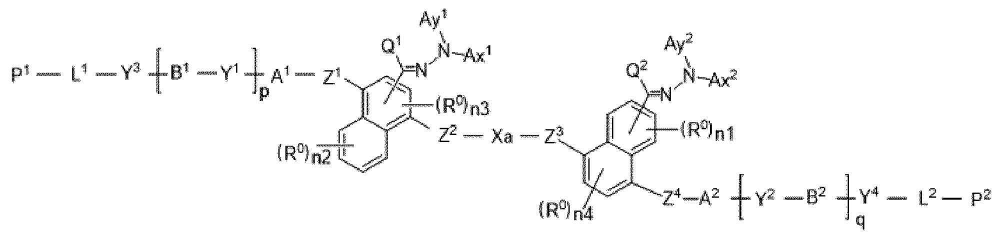

(III-3)

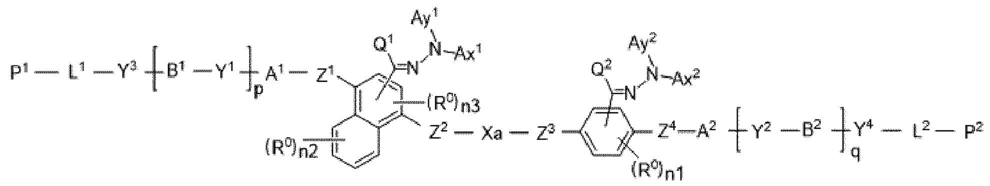

(III-4)

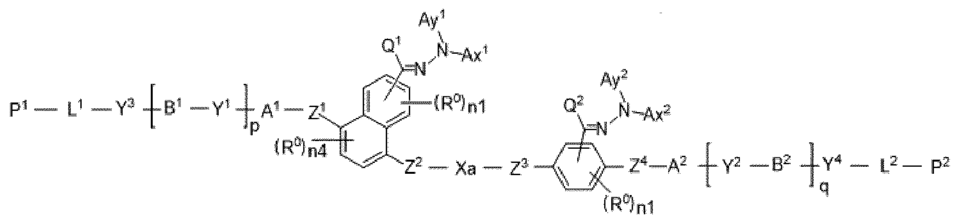

(III-5)

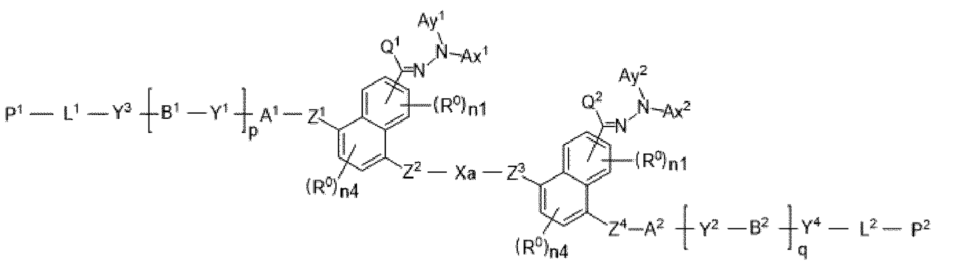

(III-6)

where, in formulae (III-1) to (III-6),

$Z^1$ to $Z^4$, $A^1$, $A^2$, $B^1$, $B^2$, $Y^1$ to $Y^4$, $L^1$, $L^2$, $P^1$, $P^2$, Xa, $R^0$, n1, n2, n3, n4, p, and q have the same meaning as previously described,
$Ax^1$ and $Ax^2$ each represent, independently of one another, an organic group including at least one aromatic ring selected from the group consisting of an aromatic hydrocarbon ring having a carbon number of 6 to 30 and an aromatic heterocyclic ring having a carbon number of 2 to 30, where the aromatic ring of each of $Ax^1$ and $Ax^2$ is optionally substituted,
$Ay^1$ and $Ay^2$ each represent, independently of one another, a hydrogen atom or an optionally substituted organic group having a carbon number of 1 to 30,
$Q^1$ and $Q^2$ each represent, independently of one another, a hydrogen atom or an alkyl group having a carbon number of 1 to 6, and
in a case in which more than one $B^1$, $B^2$, $Y^1$, $Y^2$, or $R^0$ is present, each $B^1$, $B^2$, $Y^1$, $Y^2$, or $R^0$ may be the same or different.

4. The polymerizable compound according to claim 3, wherein $Ay^1$ and $Ay^2$ are each, independently of one another, a hydrogen atom, an optionally substituted alkyl group having a carbon number of 1 to 20, an optionally substituted alkenyl group having a carbon number of 2 to 20, an optionally substituted alkynyl group having a carbon number of 2 to 20, an optionally substituted cycloalkyl group having a carbon number of 3 to 12, an optionally substituted aromatic hydrocarbon cyclic group having a carbon number of 6 to 30, or an optionally substituted aromatic heterocyclic group having a carbon number of 2 to 30.

5. The polymerizable compound according to claim 3 or 4, wherein $Ax^1$ and $Ax^2$ are each, independently of one another,

indicated by formula (V), shown below,

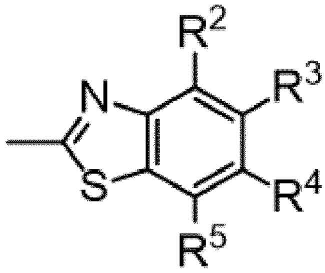

(V)

where, in formula (V), $R^2$ to $R^5$ each represent, independently of one another, a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, $-OCF_3$, $-O-C(=O)-R^b$, or $-C(=O)-O-R^b$,
$R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18, and
$R^2$ to $R^5$ may be the same or different, and one or more of ring constituents $C-R^2$ to $C-R^5$ may be replaced by a nitrogen atom.

**6.** The polymerizable compound according to any one of claims 1 to 5, wherein $P^1$ and $P^2$ are each, independently of one another, indicated by formula (IV), shown below,

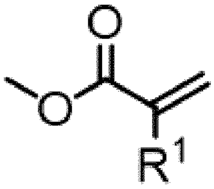

(IV)

where, in formula (IV), $R^1$ represents a hydrogen atom, a methyl group, or a chlorine atom.

**7.** The polymerizable compound according to any one of claims 1 to 6, wherein the polymerizable compound indicated by formula (1-1) is indicated by any one of formulae (VI-1) to (VI-3), shown below,

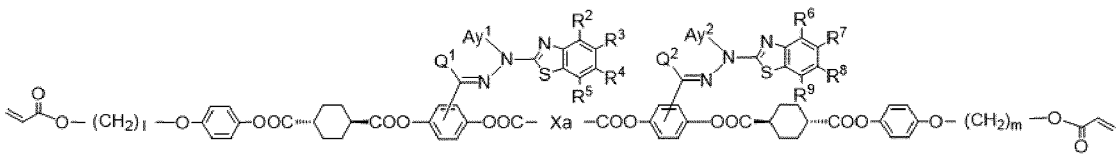

(VI-1)

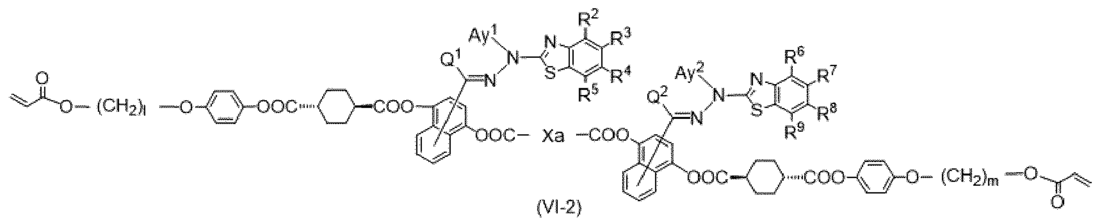

(VI-2)

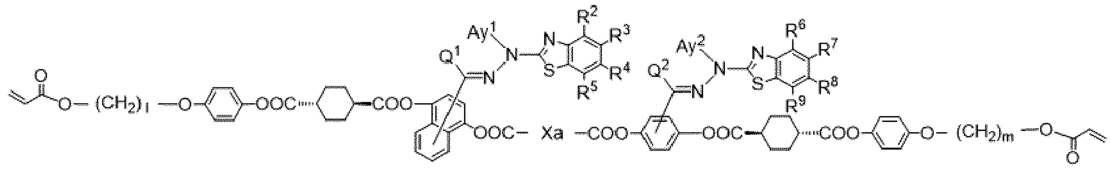

(VI-3)

where, in formulae (VI-1) to (VI-3),

Xa has the same meaning as previously described,
$R^2$ to $R^9$ each represent, independently of one another, a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, $-OCF_3$, $-O-C(=O)-R^b$, or $-C(=O)-O-R^b$,
$R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18,
one or more of ring constituents $C-R^2$ to $C-R^9$ may be replaced by a nitrogen atom,
$Ay^1$ and $Ay^2$ each represent, independently of one another, a hydrogen atom or an optionally substituted organic group having a carbon number of 1 to 30,
$Q^1$ and $Q^2$ each represent, independently of one another, a hydrogen atom or an alkyl group having a carbon number of 1 to 6, and
1 and m each represent, independently of one another, an integer of 1 to 18.

**8.** The polymerizable compound according to any one of claims 1 to 7, wherein Xa is represented by any one of formulae (VII-1) to (VII-29), shown below.

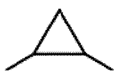 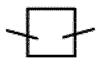 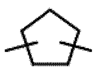 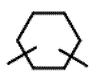 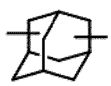 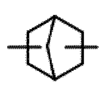

(VII-1) (VII-2) (VII-3) (VII-4) (VII-5) (VII-6)

$-CH_2-$ $-(CH_2)_2-$(VII-7) (VII-8) $-(CH_2)_3-$ $-(CH_2)_4-$ $-(CH_2)_5-$ $-(CH_2)_6-$ $-(CH_2)_7-$ $-(CH_2)_8-$ $-(CH_2)_9-$(VII-9) (VII-10) (VII-11) (VII-12) (VII-13) (VII-14) (VII-15) $-(CH_2)_{10}-$ $-(CH_2)_{11}-$ $-(CH_2)_{12}-$ $-(CH_2)_{13}-$ $-(CH_2)_{14}-$ $-(CH_2)_{15}-$(VII-16) (VII-17) (VII-18) (VII-19) (VII-20) (VII-21)

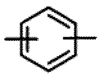 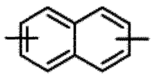 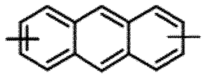 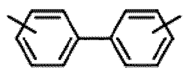 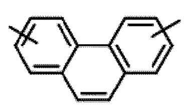

(VII-22) (VII-23) (VII-24) (VII-25) (VII-26)

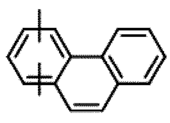 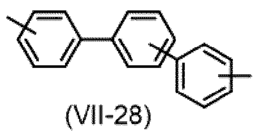 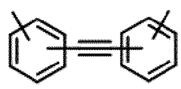

(VII-27) (VII-28) (VII-29)

**9.** A polymerizable liquid crystal mixture comprising the polymerizable compound according to any one of claims 1 to 8 as a main component.

**10.** The polymerizable liquid crystal mixture according to claim 9, comprising:

the polymerizable compound according to any one of claims 1 to 8; and
a polymerizable compound having a chemical structure differing from formula (1-1), shown below,

$$P^1-L^1-Y^3\left[B^1-Y^1\right]_pA^1-Z^1-Ar^0-Z^2-Xa-Z^3-Ar^1-Z^4-A^2\left[Y^2-B^2\right]_qY^4-L^2-P^2$$

(I－1)

where, in formula (1-1),
$Ar^0$ represents an aromatic hydrocarbon cyclic group having at least $D^0$ as a substituent or an aromatic heterocyclic group having at least $D^0$ as a substituent,
$Ar^1$ represents an aromatic hydrocarbon cyclic group having at least $D^1$ as a substituent or an aromatic heterocyclic group having at least $D^1$ as a substituent,
$D^0$ and $D^1$ each represent, independently of one another, an organic group having a carbon number of 1 to 67 and including at least one aromatic ring selected from the group consisting of an aromatic hydrocarbon ring and an aromatic heterocyclic ring,
Xa represents an optionally substituted organic group having a carbon number of 1 to 20,
$Z^1$ to $Z^4$ each represent, independently of one another, a single bond, -O-, $-O-CH_2-$, $-CH_2-O-$, -C(=O)-O-, -O-C(=O)-, -C(=O)-S-, -S-C(=O)-, $-NR^{20}C(=O)-$, $-C(=O)-NR^{20}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, $-CH_2-C(=O)-O-$, $-O-C(=O)-CH_2-$, $-CH_2-O-C(=O)-$, $-C(=O)-O-CH_2-$, $-O-CH_2-CH_2-$, $-CH_2-CH_2-O-$, or -C≡C-, where $R^{20}$ represents a hydrogen atom or an alkyl group having a carbon number of 1 to 6,
$A^1$, $A^2$, $B^1$, and $B^2$ each represent, independently of one another, an optionally substituted alicyclic group or an optionally substituted aromatic group,
$Y^1$ to $Y^4$ each represent, independently of one another, a single bond, -O-, -C(=O)-, -C(=O)-O-, -O-C(=O)-, $-NR^{21}-C(=O)-$, $-C(=O)-NR^{21}-$, -O-C(=O)-O-, $-NR^{21}-C(=O)-O-$, $-O-C(=O)-NR^{21}-$, or $-NR^{21}-C(=O)-NR^{22}-$, where $R^{21}$ and $R^{22}$ each represent, independently of one another, a hydrogen atom or an alkyl group having a carbon number of 1 to 6,
$L^1$ and $L^2$ are each, independently of one another, an organic group that is either an alkylene group having a carbon number of 1 to 20 or a group in which at least one methylene group ($-CH_2-$) included in an alkylene group having a carbon number of 1 to 20 is replaced by -O- or -C(=O)-, where hydrogen atoms included in the organic groups of $L^1$ and $L^2$ may each be replaced by an alkyl group having a carbon number of 1 to 5, an alkoxy group having a carbon number of 1 to 5, or a halogen atom, and with a proviso that methylene groups ($-CH_2-$) at both ends of $L^1$ and $L^2$ are not replaced by -O- or -C(=O)-,
one of $P^1$ and $P^2$ represents a hydrogen atom or a polymerizable group and the other of $P^1$ and $P^2$ represents a polymerizable group,
p and q are each, independently of one another, an integer of 0 to 2, and
in a case in which more than one $B^1$, $B^2$, $Y^1$, or $Y^2$ is present, each $B^1$, $B^2$, $Y^1$, or $Y^2$ may be the same or different,
wherein
an area value for the polymerizable compound according to any one of claims 1 to 8 relative to a sum total of area values for the polymerizable compound according to any one of claims 1 to 8 and the polymerizable compound having a chemical structure differing from formula (1-1), as measured by high-performance liquid chromatography (HPLC), is more than 50%.

**11.** The polymerizable liquid crystal mixture according to claim 9 or 10, comprising:

the polymerizable compound according to any one of claims 1 to 8; and
a polymerizable compound indicated by formula (I-2), shown below,

$$P^3-L^3-Y^7\left[B^3-Y^5\right]_{p1}A^3-Z^5-Ar^2-Z^6-A^4\left[Y^6-B^4\right]_{q1}Y^8-L^4-P^4$$

(I－2).

where, in formula (I-2),
$Ar^2$ represents an aromatic hydrocarbon cyclic group having at least $D^2$ as a substituent or an aromatic heterocyclic group having at least $D^2$ as a substituent,

$D^2$ represents an organic group having a carbon number of 1 to 67 and including at least one aromatic ring selected from the group consisting of an aromatic hydrocarbon ring and an aromatic heterocyclic ring,
$Z^5$ and $Z^6$ each represent, independently of one another, a single bond, -O-, -O-$CH_2$-, -$CH_2$-O-, -C(=O)-O-, -O-C(=O)-, -C(=O)-S-, -S-C(=O)-, -$NR^{20}$-C(=O)-, -C(=O)-$NR^{20}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, -$CH_2$-C(=O)-O-, -O-C(=O)-$CH_2$-, -$CH_2$-O-C(=O)-, -C(=O)-O-$CH_2$-, -O-$CH_2$-$CH_2$-, -$CH_2$-$CH_2$-O-, or -C≡C-, where $R^{20}$ represents a hydrogen atom or an alkyl group having a carbon number of 1 to 6,
$A^3$, $A^4$, $B^3$, and $B^4$ each represent, independently of one another, an optionally substituted alicyclic group or an optionally substituted aromatic group,
$Y^5$ to $Y^8$ each represent, independently of one another, a single bond, -O-, -C(=O)-, -C(=O)-O-, -O-C(=O)-, -$NR^{21}$-C(=O)-, -C(=O)-$NR^{21}$-, -O-C(=O)-O-, -$NR^{21}$-C(=O)-O-, -O-C(=O)-$NR^{21}$-, or -$NR^{21}$-C(=O)-$NR^{22}$-, where $R^{21}$ and $R^{22}$ each represent, independently of one another, a hydrogen atom or an alkyl group having a carbon number of 1 to 6,
$L^3$ and $L^4$ are each, independently of one another, an organic group that is either an alkylene group having a carbon number of 1 to 20 or a group in which at least one methylene group (-$CH_2$-) included in an alkylene group having a carbon number of 1 to 20 is replaced by -O- or -C(=O)-, where hydrogen atoms included in the organic groups of $L^3$ and $L^4$ may each be replaced by an alkyl group having a carbon number of 1 to 5, an alkoxy group having a carbon number of 1 to 5, or a halogen atom, and with a proviso that methylene groups (-$CH_2$-) at both ends of $L^3$ and $L^4$ are not replaced by -O- or -C(=O)-,
one of $P^3$ and $P^4$ represents a hydrogen atom or a polymerizable group and the other of $P^3$ and $P^4$ represents a polymerizable group,
p1 and q1 are each, independently of one another, an integer of 0 to 2, and
in a case in which more than one $B^3$, $B^4$, $Y^5$, or $Y^6$ is present, each $B^3$, $B^4$, $Y^5$, or $Y^6$ may be the same or different, wherein
an area value for the polymerizable compound according to any one of claims 1 to 8 relative to a sum total of area values for the polymerizable compound according to any one of claims 1 to 8 and the polymerizable compound indicated by formula (I-2), as measured by high-performance liquid chromatography (HPLC), is more than 50%.

**12.** The polymerizable liquid crystal mixture according to claim 11, wherein $Ar^2$ is indicated by any one of formulae (II-1) to (II-7), shown below,

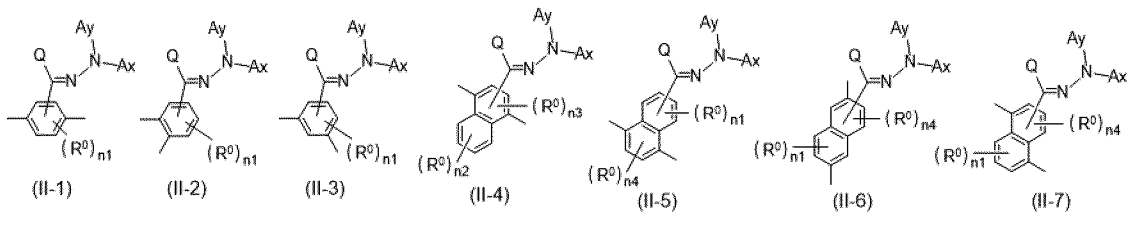
(II-1) (II-2) (II-3) (II-4) (II-5) (II-6) (II-7)

where, in formulae (II-1) to (II-7),

Ax represents an organic group including at least one aromatic ring selected from the group consisting of an aromatic hydrocarbon ring having a carbon number of 6 to 30 and an aromatic heterocyclic ring having a carbon number of 2 to 30, where the aromatic ring of Ax is optionally substituted,
Ay represents a hydrogen atom or an optionally substituted organic group having a carbon number of 1 to 30,
Q represents a hydrogen atom or an alkyl group having a carbon number of 1 to 6,
$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, -C(=O)-$R^a$, -O-C(=O)-$R^a$, -C(=O)-O-$R^a$, or -$SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, n1 is an integer of 0 to 3, n2 is an integer of 0 to 4, n3 is 0 or 1, and n4 is an integer of 0 to 2, and
in a case in which more than one $R^0$ is present, each $R^0$ may be the same or different.

**13.** The polymerizable liquid crystal mixture according to claim 11 or 12, wherein $P^3$ and $P^4$ are each, independently of

one another, indicated by formula (IV), shown below,

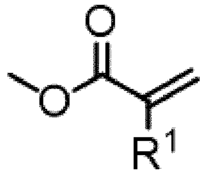

(IV)

where, in formula (IV), $R^1$ represents a hydrogen atom, a methyl group, or a chlorine atom.

**14.** A polymer obtained by polymerizing the polymerizable liquid crystal mixture according to any one of claims 9 to 13.

**15.** An optical film comprising the polymer according to claim 14 as a constituent material.

**16.** An optically anisotropic body comprising a layer having the polymer according to claim 14 as a constituent material.

**17.** A polarizer comprising:

the optically anisotropic body according to claim 16; and
a polarizing film.

**18.** A display comprising the polarizer according to claim 17.

**19.** An antireflection film comprising the polarizer according to claim 17.

**20.** A compound indicated by formula (VIII-1), shown below,

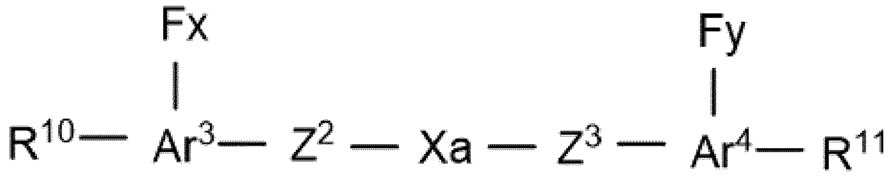

(VIII-1)

where, in formula (VIII-1),

$Ar^3$ and $Ar^4$ each represent, independently of one another, an aromatic hydrocarbon cyclic group or an aromatic heterocyclic group,
Xa represents an optionally substituted organic group having a carbon number of 1 to 20,
$Z^2$ and $Z^3$ each represent, independently of one another, a single bond, -O-, $-O-CH_2-$, $-CH_2-O-$, -C(=O)-O-, -O-C(=O)-, -C(=O)-S-, -S-C(=O)-, $-NR^{20}C(=O)-$, $-C(=O)-NR^{20}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, $-CH_2-C(=O)-O-$, $-O-C(=O)-CH_2-$, $-CH_2-O-C(=O)-$, $-C(=O)-O-CH_2-$, $-O-CH_2-CH_2-$, $-CH_2-CH_2-O-$, or -C≡C-, where $R^{20}$ represents a hydrogen atom or an alkyl group having a carbon number of 1 to 6,
Fx and Fy each represent, independently of one another, $-C(R^f)=N-N(R^g)R^h$, $-C(R^f)=N-N=C(R^{g1})R^h$, or -CHO, where $R^f$ represents a hydrogen atom or an alkyl group having a carbon number of 1 to 6, $R^g$ and $R^{g1}$ each represent, independently of one another, a hydrogen atom or an optionally substituted organic group having a carbon number of 1 to 30, and $R^h$ represents an organic group including at least one aromatic ring selected from the group consisting of an aromatic hydrocarbon ring having a carbon number of 6 to 30 and an aromatic heterocyclic ring having a carbon number of 2 to 30, and
$R^{10}$ and $R^{11}$ each represent, independently of one another, $-OR^p$, $-CH_2OR^p$, $-CH_2CH_2OR^p$, $-C(=O)-OR^p$, $-CH_2-C(=O)-OR^p$, $-CH_2CH_2-C(=O)-OR^p$, a hydroxy group, a carboxyl group, $-CH_2-C(=O)-OH$, $-CH_2CH_2-C(=O)-OH$, $-CH_2OH$, $-CH_2CH_2OH$, or an amino group, where $R^p$ represents a protecting group.

**21.** The compound according to claim 20, wherein $Ar^3$-Fx and $Ar^4$-Fy are each, independently of one another, indicated

by any one of formulae (IX-1) to (IX-14), shown below,

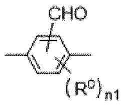
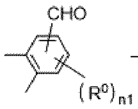
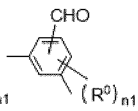
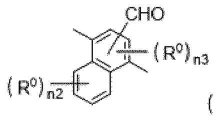
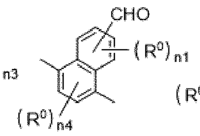
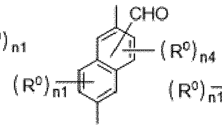
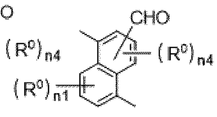

(IX-1) (IX-2) (IX-3) (IX-4) (IX-5) (IX-6) (IX-7)

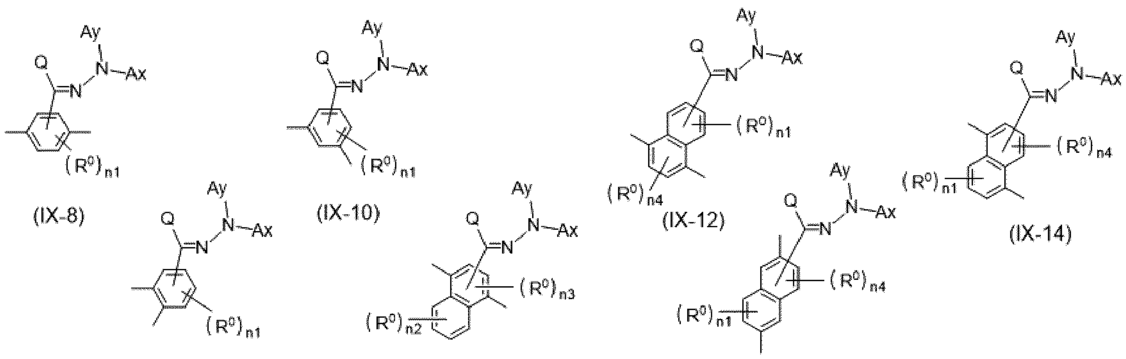

(IX-8) (IX-9) (IX-10) (IX-11) (IX-12) (IX-13) (IX-14)

where, in formulae (IX-1) to (IX-14),

Ax represents an organic group including at least one aromatic ring selected from the group consisting of an aromatic hydrocarbon ring having a carbon number of 6 to 30 and an aromatic heterocyclic ring having a carbon number of 2 to 30, where the aromatic ring of Ax is optionally substituted,
Ay represents a hydrogen atom or an optionally substituted organic group having a carbon number of 1 to 30,
Q represents a hydrogen atom or an alkyl group having a carbon number of 1 to 6,
$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, $-C(=O)-R^a$, $-O-C(=O)-R^a$, $-C(=O)-O-R^a$, or $-SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, n1 is an integer of 0 to 3, n2 is an integer of 0 to 4, n3 is 0 or 1, and n4 is an integer of 0 to 2, and
in a case in which more than one $R^0$ is present, each $R^0$ may be the same or different.

**22.** The compound according to claim 21, wherein the compound is indicated by any one of formulae (X-1) to (X-12), shown below,

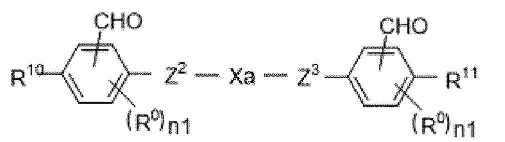
(X-1)

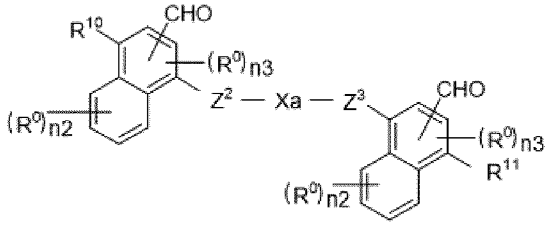
(X-2)

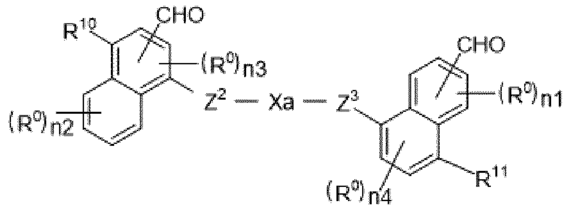

(X-3)

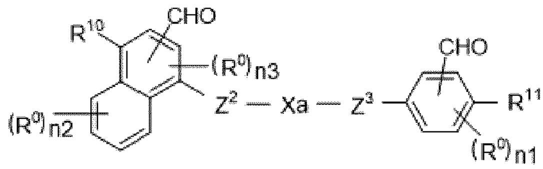

(X-4)

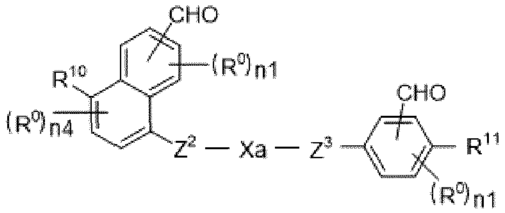

(X-5)

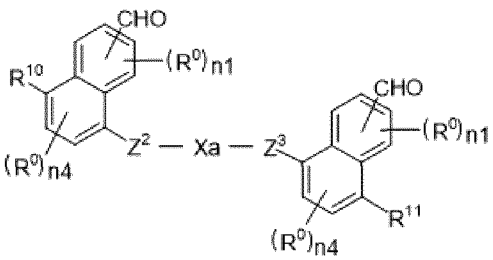

(X-6)

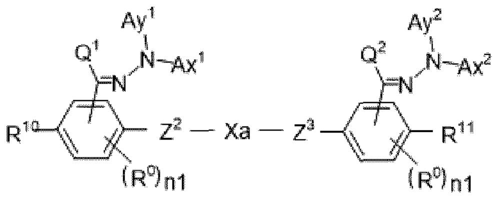

(X-7)

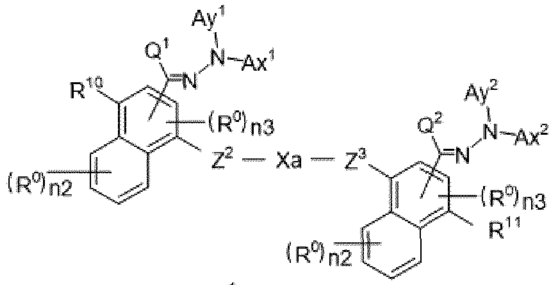

(X-8)

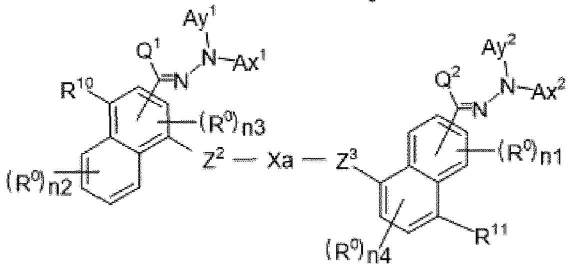

(X-9)

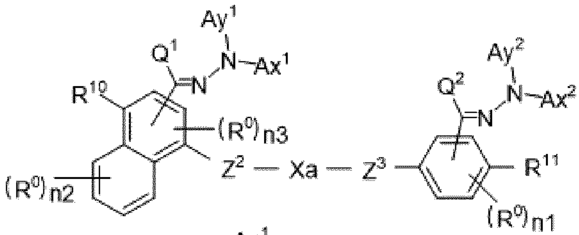

(X-10)

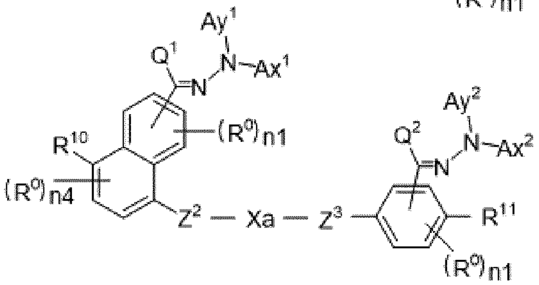

(X-11)

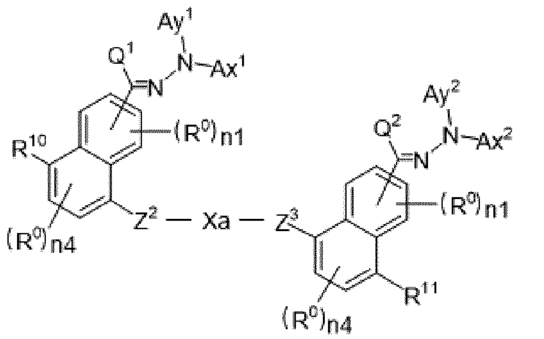

(X-12)

where, in formulae (X-1) to (X-12),

Xa, $Z^2$, $Z^3$, $R^{10}$, $R^{11}$, $R^0$, n1, n2, n3, and n4 have the same meaning as previously described,

$Ax^1$ and $Ax^2$ each represent, independently of one another, an organic group including at least one aromatic ring selected from the group consisting of an aromatic hydrocarbon ring having a carbon number of 6 to 30 and an aromatic heterocyclic ring having a carbon number of 2 to 30, where the aromatic ring of each of $Ax^1$ and $Ax^2$ is optionally substituted,

$Ay^1$ and $Ay^2$ each represent, independently of one another, a hydrogen atom or an optionally substituted organic group having a carbon number of 1 to 30,

$Q^1$ and $Q^2$ each represent, independently of one another, a hydrogen atom or an alkyl group having a carbon number of 1 to 6, and

in a case in which more than one $R^0$ is present, each $R^0$ may be the same or different.

**23.** A compound indicated by any one of formulae (XI-1) to (XI-6), shown below,

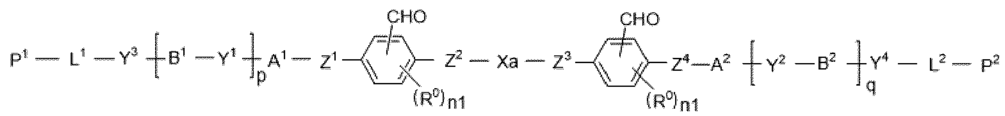

(XI-1)

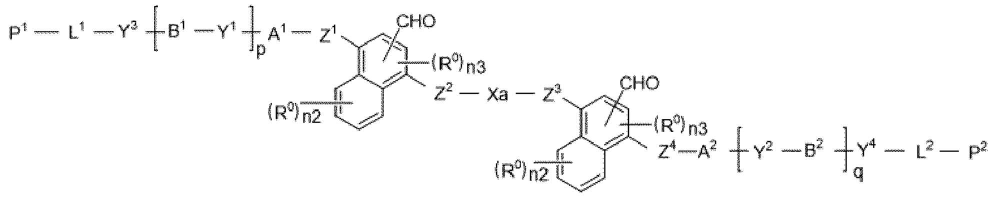

(XI-2)

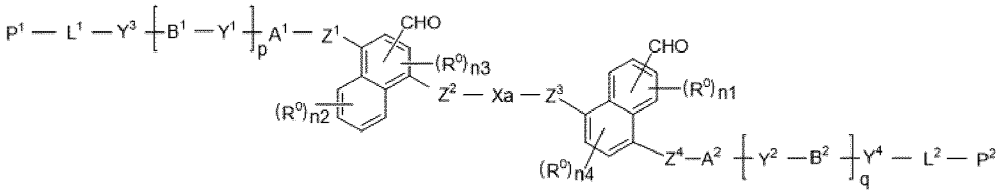

(XI-3)

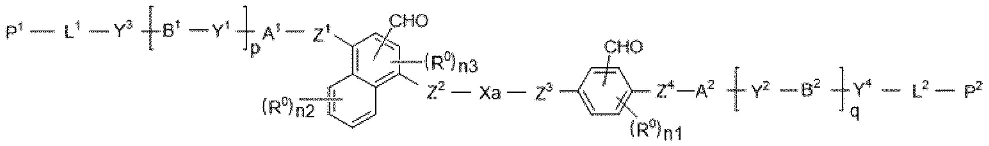

(XI-4)

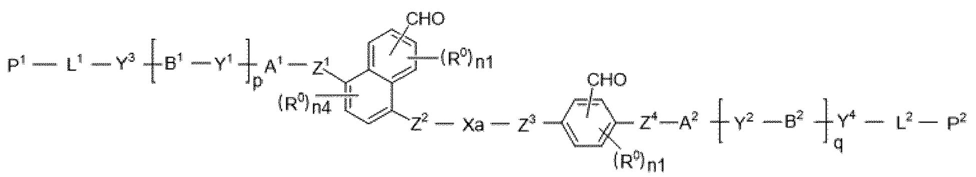

(XI-5)

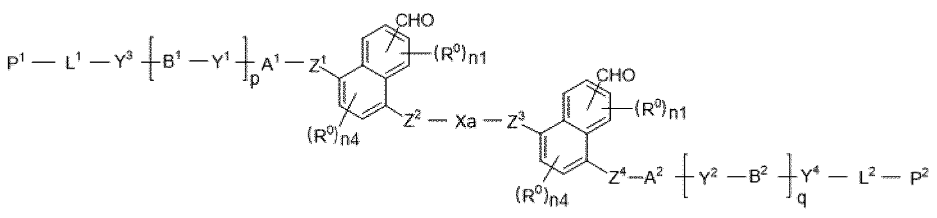

(XI-6)

where, in formulae (XI-1) to (XI-6),

Xa represents an optionally substituted organic group having a carbon number of 1 to 20,

$Z^1$ to $Z^4$ each represent, independently of one another, a single bond, -O-, -O-$CH_2$-, -$CH_2$-O-, -C(=O)-O-, -O-C(=O)-, -C(=O)-S-, -S-C(=O)-, -$NR^{20}$-C(=O)-, -C(=O)-$NR^{20}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH- -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, -$CH_2$-C(=O)-O-, -O-C(=O)-$CH_2$-, -$CH_2$-O-C(=O)-, -C(=O)-O-$CH_2$-, -O-$CH_2$-$CH_2$-, -$CH_2$-$CH_2$-O-, or -C≡C-, where $R^{20}$ represents a hydrogen atom or an alkyl group having a carbon number of 1 to 6,

$A^1$, $A^2$, $B^1$, and $B^2$ each represent, independently of one another, an optionally substituted alicyclic group or an optionally substituted aromatic group,

$Y^1$ to $Y^4$ each represent, independently of one another, a single bond, -O-, -C(=O)-, -C(=O)-O-, -O-C(=O)-, -$NR^{21}$-C(=O)-, -C(=O)-$NR^{21}$-, -O-C(=O)-O-, -$NR^{21}$-C(=O)-O-, -O-C(=O)-$NR^{21}$-, or -$NR^{21}$-C(=O)-$NR^{22}$-, where $R^{21}$ and $R^{22}$ each represent, independently of one another, a hydrogen atom or an alkyl group having a carbon number of 1 to 6,

$L^1$ and $L^2$ are each, independently of one another, an organic group that is either an alkylene group having a carbon number of 1 to 20 or a group in which at least one methylene group (-$CH_2$-) included in an alkylene group having a carbon number of 1 to 20 is replaced by -O- or -C(=O)-, where hydrogen atoms included in the organic groups of $L^1$ and $L^2$ may each be replaced by an alkyl group having a carbon number of 1 to 5, an alkoxy group having a carbon number of 1 to 5, or a halogen atom, and with a proviso that methylene groups (-$CH_2$-) at both ends of $L^1$ and $L^2$ are not replaced by -O- or -C(=O)-,

one of $P^1$ and $P^2$ represents a hydrogen atom or a polymerizable group and the other of $P^1$ and $P^2$ represents a polymerizable group,

p and q are each, independently of one another, an integer of 0 to 2,

$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, -C(=O)-$R^a$, -O-C(=O)-$R^a$, -C(=O)-O-$R^a$, or -$SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, n1 is an integer of 0 to 3, n2 is an integer of 0 to 4, n3 is 0 or 1, and n4 is an integer of 0 to 2, and in a case in which more than one $R^0$, $B^1$, $B^2$, $Y^1$, or $Y^2$ is present, each $R^0$, $B^1$, $B^2$, $Y^1$, or $Y^2$ may be the same or different.

**24.** The compound according to claim 23, wherein the compound is indicated by any one of formulae (XII-1) to (XII-3), shown below,

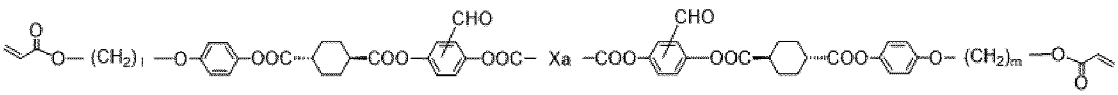

(XII-1)

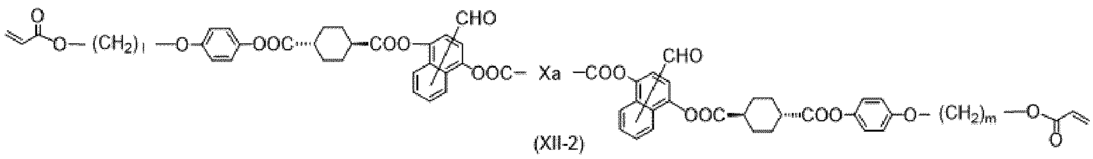

(XII-2)

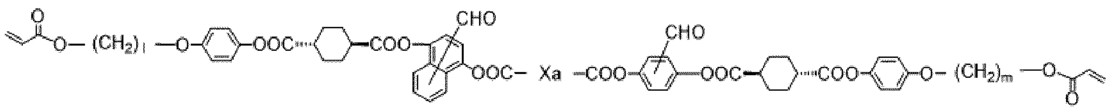

(XII-3)

where, in formulae (XII-1) to (XII-3),

Xa has the same meaning as previously described, and

1 and m each represent, independently of one another, an integer of 1 to 18.

## INTERNATIONAL SEARCH REPORT

International application No. PCT/JP2017/044699

**A. CLASSIFICATION OF SUBJECT MATTER**

Int. Cl. C07D277/82(2006.01)i, C08F20/38(2006.01)i, G02B1/111(2015.01)i, G02B5/30(2006.01)i, G02F1/1335(2006.01)i, G02F1/13363(2006.01)i

According to International Patent Classification (IPC) or to both national classification and IPC

**B. FIELDS SEARCHED**

Minimum documentation searched (classification system followed by classification symbols)

Int. Cl. C07D277/82, C08F20/38, G02B1/111, G02B5/30, G02F1/1335, G02F1/13363

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2016/104317 A1 (DIC CORP.) 30 June 2016, p. 197, 199-206, 299, embodiments 74-76, 78, tables 16-19, claims & CN 107108458 A & KR 10-2017-0101194 A | 1-24 |
| X<br>A | CERRADA, P. et al., Journal of Polymer Science: Part A: Polymer Chemistry, 1996, vol. 34, pp. 2603-2611 | 20-22<br>1-19, 23, 24 |

☒ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| | |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

INTERNATIONAL SEARCH REPORT

International application No.
PCT/JP2017/044699

C (Continuation). DOCUMENTS CONSIDERED TO BE RELEVANT

| Category* | Citation of document, with indication, where appropriate, of the relevant passages | Relevant to claim No. |
|---|---|---|
| X<br>A | CN 102234291 A (SHANGHAI INSTITUTE OF ORGANIC CHEMISTRY, CHINA ACADEMY OF SCIENCES) 09 November 2011, paragraphs [0042], [0048], [0052], [0054], [0060] (Family: none) | 20-22<br>1-19, 23, 24 |
| X<br>A | JP 2012-519225 A (DOW GLOBAL TECHNOLOGIES LLC) 23 August 2012, p. 9, formula & US 2011/0301366 A1, p. 5, formula & WO 2010/099295 A1 & CN 102325820 A & KR 10-2011-0132337 A | 20-22<br>1-19, 23, 24 |
| X<br>A | SURESH, P. et al., Chemistry Letters, 2007, vol. 36, no. 11, pp. 1332-1333 | 20-22<br>1-19, 23, 24 |

Form PCT/ISA/210 (continuation of second sheet) (January 2015)

INTERNATIONAL SEARCH REPORT

International application No.
PCT/JP2017/044699

Scope of search

Claim 1 sets forth a polymerizable compound, represented by formula (I-1), that can be used to prepare an optical film/optically anisotropic body. Claim 1 also sets forth that the substituents $D^0$, $D^1$ of $Ar^0$ and $Ar^1$, which pertains to the main backbone structure of formula (I-1), are "$C_{1-67}$ organic group having at least one aromatic ring selected from the group consisting of an aromatic hydrocarbon ring and a heteroaromatic ring," and $X_a$ is an "optionally substituted $C_{1-20}$ organic group." These wordings encompass very broad ranges of structures.

Claims 20 and 23 set forth compounds represented by formula (VIII-1) and formulas (XI-1) to (XI-6) as compounds that become intermediates in the manufacturing process of the polymerizable compound represented by formula (I-1), and that $X_a$ in these formulas is an "optionally substituted $C_{1-20}$ organic group." Claims 20 and 23 also set forth substituents $F_x$, $F_y$ of $Ar^3$, $Ar^4$, which pertain to the main backbone structure of formula (VIII-1), using language that encompasses very broad range of structures.

Meanwhile, the only compounds disclosed in the embodiments in the specification, are six compounds, where $D^0$ and $D^1$ in formula (I-1) are groups having a structure of "-C=N-N-benzothiazole" and $X_a$ is butylene, cyclohexylene, or phenylene, and the manufacturing intermediates of the compounds.

However, it could be said to have been common general technical knowledge at the time of filing of the present application that chemical properties vary significantly if there are major differences in main backbone structure between compounds. With the above-mentioned embodiments, therefore, no basis can be found for extending the definitions of $D^0$, $D^1$, and $X_a$, which pertain to the main backbone structure of formula (I-1), to the scope of the definitions set forth in claim 1. No basis can be found also for extending the manufacturing intermediates of the polymerizable compound represented by formula (I-1) to the scope of the compounds represented by formula (VIII-1) and formulas (XI-1) to (XI-6) set forth in claims 20 and 23.

The invention in claims 1, 20, and 23 of the present international application cannot accordingly be said to be disclosed in the description of the present international application in a manner sufficiently clear and complete for the invention to be carried out by a person skilled in the art (PCT Article 5), and also cannot be said to be disclosed with sufficient support (PCT Article 6).

As such, it is not possible to conduct a meaningful search—and therefore no search was conducted—other than for compounds represented by formula (I-1) in which $Ar^0$ and $Ar^1$, which include $D^0$ and $D^1$, are represented by any of formulas (II-1) to (II-7) set forth in claim 2 and $X_a$ is either an alkylene or a carbocycle, and for the manufacturing intermediates the compounds.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014010325 A1
- JP 2007002208 A
- JP 2009173893 A
- JP 2009274984 A
- JP 2010030979 A
- JP 2010031223 A
- JP 2011006360 A
- JP 2010024438 A
- WO 2012141245 A1
- WO 2012147904 A1
- WO 2012169424 A1
- WO 201276679 A1
- WO 2013180217 A1
- WO 2014061709 A1
- WO 2014065176 A1
- WO 2014126113 A1
- WO 2015025793 A1
- WO 2015064698 A1
- WO 2015122384 A1
- WO 2015122385 A1
- JP H05310845 A
- US 5179171 A
- JP H0597978 A
- US 5202388 A
- JP H11124429 A
- WO 9920676 A1


### Non-patent literature cited in the description


- March's Advanced Organic Chemistry. Wiley
- **SANDLER ; KARO.** Syntheses of Organic Compounds Classified by Functional Group. Hirokawa Publishing Company
- Greene's Protective Groups in Organic Synthesis. Wiley
- March's Advanced Organic Chemistr. Wiley